(19) Europäisches Patentamt
European Patent Office
Office européen des brevets

(11) EP 1 646 663 B1

(12) EUROPÄISCHE PATENTSCHRIFT

(45) Veröffentlichungstag und Bekanntmachung des Hinweises auf die Patenterteilung:
**24.01.2007 Patentblatt 2007/04**

(21) Anmeldenummer: **04740968.5**

(22) Anmeldetag: **13.07.2004**

(51) Int Cl.:
***C08F 26/00*** *(2006.01)* ***C08F 2/10*** *(2006.01)*
***C08F 20/04*** *(2006.01)*

(86) Internationale Anmeldenummer:
**PCT/EP2004/007741**

(87) Internationale Veröffentlichungsnummer:
**WO 2005/005497 (20.01.2005 Gazette 2005/03)**

# (54) WÄSSRIGE POLYMERDISPERSION UND DEREN VERWENDUNG IN DER KOSMETIK

AQUEOUS POLYMER DISPERSION AND USE THEREOF IN COSMETICS

DISPERSION POLYMERE AQUEUSE ET SON UTILISATION EN COSMETIQUE

(84) Benannte Vertragsstaaten:
**AT BE BG CH CY CZ DE DK EE ES FI FR GB GR HU IE IT LI LU MC NL PL PT RO SE SI SK TR**

(30) Priorität: **14.07.2003 DE 10331865**

(43) Veröffentlichungstag der Anmeldung:
**19.04.2006 Patentblatt 2006/16**

(73) Patentinhaber: **BASF Aktiengesellschaft
67056 Ludwigshafen (DE)**

(72) Erfinder:
- **CHRISSTOFFELS, Lysander
67117 Limburgerhof (DE)**
- **HÖSSEL, Peter
67105 Schifferstadt (DE)**
- **GARCIA CASTRO, Ivette
67067 Lugwigshafen (DE)**
- **WOOD, Claudia
69469 Weinheim (DE)**
- **ANGEL, Maximilian
67105 Schifferstadt (DE)**
- **MATHAUER, Klemens
69115 Heidelberg (DE)**

(74) Vertreter: **Wortmann, Jens
Reitstötter, Kinzebach & Partner
Ludwigsplatz 4
D-67059 Ludwigshafen (DE)**

(56) Entgegenhaltungen:
**US-A1- 2003 091 602** **US-B1- 6 362 245**
**US-B1- 6 426 383**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft eine wässrige Polymerdispersion, die durch radikalische Polymerisation eines Monomergemischs erhältlich ist, welches wenigstens eine amidgruppenhaltige Verbindung, wenigstens einen Vernetzer und wenigstens ein Monomer mit mindestens einer kationogenen und/oder kationischen Gruppe enthält. Die Erfindung betrifft weiterhin die durch Trocknen einer solchen Polymerdispersion erhältlichen Polymere sowie kosmetische oder pharmazeutische Mittel, die eine solche Polymerdispersion oder ein solches Polymer enthalten.

Kosmetisch und pharmazeutisch akzeptable wasserlösliche Polymere finden in Kosmetik und Medizin vielfache Anwendung. In Seifen, Cremes und Lotionen beispielsweise dienen sie in der Regel als Formulierungsmittel, z. B. als Verdicker, Schaumstabilisator oder Wasserabsorbens oder auch dazu, die reizende Wirkung anderer Inhaltsstoffe abzumildern oder die dermale Applikation von Wirkstoffen zu verbessern. Ihre Aufgabe in der Haarkosmetik besteht darin, die Eigenschaften des Haares zu beeinflussen. In der Pharmazie dienen sie beispielsweise als Beschichtungsmittel oder Bindemittel für feste Arzneiformen.

Für die Haarkosmetik werden filmbildende Polymere beispielsweise als Conditioner dazu eingesetzt, um die Trocken- und Nasskämmbarkeit, Anfassgefühl, Glanz und Erscheinungsform zu verbessern sowie dem Haar antistatische Eigenschaften zu verleihen. Es ist bekannt, wasserlösliche Polymere mit kationischen Funktionalitäten in Haarkonditioniermitteln einzusetzen, die eine größere Affinität zur strukturell bedingt negativ geladenen Oberfläche des Haares aufweisen und eine elektrostatische Aufladung des Haares verhindern. Struktur und Wirkungsweise verschiedener Haarbehandlungspolymere sind in Cosmetic & Toiletries 103 (1988) 23 beschrieben. Handelsübliche kationische Conditionerpolymere sind z. B. kationische Hydroxyethylcellulose, kationische Polymere auf der Basis von N-Vinylpyrrolidon, z. B. Copolymere aus N-Vinylpyrrolidon und quarterniertem N-Vinylimidazol oder Copolymere aus Acrylamid und Diallyldimethylammoniumchlorid. Letztere haben dabei den Nachteil eines hohen Restmonomerengehalts, da die Copolymerisationsparameter dieser Monomere ungünstig sind.

Zur Festigung von Haarfrisuren werden beispielsweise Vinyllactam-Homo- und Copolymere und Carboxylatgruppen-haltige Polymere eingesetzt. Anforderungen an Haarfestigerharze sind zum Beispiel eine starke Festigung bei hoher Luftfeuchtigkeit, Elastizität, Auswaschbarkeit vom Haar, Verträglichkeit in der Formulierung und ein angenehmer Griff des damit behandelten Haares.

Schwierigkeiten bereitet oft die Bereitstellung von Produkten mit einem komplexen Eigenschaftsprofil. So besteht ein Bedarf an Polymeren für kosmetische Mittel, die zur Bildung im Wesentlichen glatter, klebfreier Filme befähigt sind, die dem Haar und der Haut gute sensorisch erfassbare Eigenschaften, wie einen angenehmen Griff, verleihen und gleichzeitig eine gute Konditionierwirkung bzw. Festigungswirkung aufweisen. Zusätzlich zu guten kosmetischen Wirkeigenschaften sollen sich Produkte mit möglichst hohen Feststoffgehalten bei gleichzeitig guten rheologischen Eigenschaften formulieren lassen. Zudem werden an kosmetische und pharmazeutische Produkte vom Verbraucher zunehmend ästhetische Anforderungen gestellt. So wird bei derartigen Produkten derzeit eine Bevorzugung von klaren, opaquen Formulierungen beobachtet. Dazu müssen die eingesetzten Polymere eine gute Verträglichkeit mit möglichst vielen weiteren kosmetischen Formulierungsbestandteilen aufweisen.

Die EP-A-183 466 beschreibt ein Verfahren zur Herstellung einer Polymerdispersion durch Polymerisation eines wasserlöslichen Monomers in einem wässrigen salzhaltigen Medium in Gegenwart eines Dispergiermittels. Bei dem wasserlöslichen Monomer kann es sich u. a. um eine ethylenisch ungesättigte Verbindung mit einer kationischen Gruppe handeln. Als Dispergiermittel können Polyelektrolyte eingesetzt werden, deren ionogene/ionische Gruppen der Ladung der eingesetzten Monomere entsprechen müssen. Eine Mitverwendung von Vernetzern bei der Polymerisation wird nicht beschrieben.

Die EP-A-670 333 beschreibt vernetzte wasserlösliche Polymerdispersionen, die durch Polymerisation eines Monomergemischs, enthaltend wenigstens ein wasserlösliches Monomer, wenigstens einen Vernetzer sowie gegebenenfalls hydrophobe und/oder amphiphile Monomere in Gegenwart eines polymeren Dispergiermittels erhältlich sind. Als wasserlösliche Monomere können neben einer Vielzahl weiterer auch N-Vinylpyrrolidon sowie Monomere mit kationischen/kationisierbaren Gruppen, wie N-Vinylimidazol, eingesetzt werden. Bei den polymeren Dispergiermitteln kann es sich um Polyelektrolyte handeln, die beispielweise Salze der (Meth)acrylsäure als anionische Monomerbausteine oder quaternierte Derivate von N,N-Dimethylaminoethyl(meth)acrylat als kationische Bausteine einpolymerisiert enthalten. In diesem Dokument wird weder konkret der Einsatz von Monomergemischen, die ein amidgruppenhaltiges Monomer, einen Vernetzer und ein Monomer mit kationogenen/kationischen Gruppen enthalten noch der Einsatz von Monomergemischen und Dispergiermitteln mit entgegengesetzt geladenen/ladbaren Gruppen beschrieben.

Die EP-A-855 407 beschreibt Zusammensetzungen auf Basis wasserlöslicher Polymere, die durch Polymerisation wenigstens eines wasserlöslichen Monomers in Gegenwart eines Styrol-Maleinsäureanhydrid-Copolymers erhältlich sind. Bei den eingesetzten wasserlöslichen Monomeren kann es sich u. a. um ethylenisch ungesättigte Verbindungen mit kationogenen/kationischen Gruppen handeln. Die Anmeldung betrifft weiterhin wasserlösliche Zusammensetzungen, die wenigstens ein wasserlösliches Polymer und ein polymeres Dispergiermittel enthalten und wobei das wasserlösliche Polymer ein kationisches Acrylmonomer mit einer Benzylammoniumgruppe einpolymerisiert enthält. Bei

den Dispergiermitteln der letztgenannten Zusammensetzungen kann es sich neben Styrol-Maleinsäureanhydrid-Copolymeren auch um Poly(diallyldimethylammoniumchlorid) handeln. Polymere, die wenigstens eine N-Vinylamidgruppenhaltige Verbindung und wenigstens einen Vernetzer einpolymerisiert enthalten, sind nicht beschrieben.

Die WO 98/54234 beschreibt wässrige Dispersionen von Polymeren, die wenigstens ein N-Vinylamidmonomer sowie gegebenenfalls weitere Comonomere einpolymerisiert enthalten. Bei diesen Comonomeren kann es sich beispielsweise um Vinylpyrrolidon oder Acrylatmonomere mit kationogenen/kationischen Gruppen handeln. Die Herstellung erfolgt durch Polymerisation in Gegenwart eines wasserlöslichen Stabilisatorpolymers. Als geeignete Stabilisatorpolymere werden mit Methylchlorid quaternisiertes Poly(dimethylaminoethylacrylat) sowie Polyvinylalkohol genannt. Wässrige Polymerdispersionen, die durch radikalische Polymerisation eines Monomergemischs, das wenigstens ein kationisches Monomer und wenigstens einen Vernetzer enthält, in Gegenwart eines anionischen Dispergiermittels erhältlich sind, sind nicht beschrieben.

Ein Einsatz der in den zuvor genannten Dokumenten beschriebenen Polymere und Polymerdispersionen in der Kosmetik wird nicht beschrieben.

Die EP-A-929 285 lehrt die Verwendung von wasserlöslichen Copolymeren, die Vinylcarbonsäureamid-Einheiten und Vinylimidazol-Einheiten einpolymerisiert enthalten als Bestandteil kosmetischer Mittel. Auch der Einsatz von Vernetzern zur Modifizierung dieser Polymere ist beschrieben. Die Polymerisation in Gegenwart wenigstens eines polymeren anionischen Dispergiermittels ist dagegen in diesem Dokument nicht offenbart.

Die WO 00/27893 beschreibt wässrige Polymerdispersionen auf der Basis von N-Vinylcarbonsäureamiden und gegebenenfalls weiteren Comonomeren, wobei die Polymerisation in Gegenwart wenigstens eines polymeren Dispergiermittels erfolgt. Ein Einsatz in der Kosmetik wird nur ganz allgemein und ohne Beleg durch ein Ausführungsbeispiel beschrieben.

Die WO 02/34796 beschreibt ein Polymerisationsverfahren, bei dem wenigstens ein Monomer in Gegenwart wenigstens eines wasserlöslichen polymeren Dispergiermittels und eines Salzgemischs fraktioniert polymerisiert wird. Bei den Monomeren kann es sich um wasserlösliche nichtionische, anionische oder kationische Monomere handeln. Als polymere Dispergiermittel können Polyelektrolyte eingesetzt werden, wobei die Dispergiermittel die gleiche Ladung wie die zur Polymerisation eingesetzten Monomere aufweisen. Ein Einsatz dieser Polymere in der Kosmetik wird nur ganz allgemein ohne Angabe eines möglichen Einsatzgebiets beschrieben.

Die WO 02/41856 beschreibt die Verwendung von Polymerdispersionen, die durch Polymerisation wenigstens eines wasserlöslichen Monomers in einer wässrigen Salzlösung, die wenigstens einen Polyelektrolyten als Dispergiermittel enthält, erhältlich sind zur kosmetischen Behandlung von keratinischen Materialien. Zusätzlich enthalten die Dispersionen wenigstens ein Mittel zur Einstellung der Viskosität, beispielsweise eine Polycarbonsäure oder ein Salz davon. Als wasserlösliche Monomere können kationische, anionische und nichtionische Monomere eingesetzt werden, bevorzugt sind Monomergemische, die wenigstens ein kationisches Monomer sowie gegebenenfalls zusätzlich Acrylsäure und/oder Acrylamid enthalten. Als geeignete Dispergiermittel werden konkret nur kationische Polyelektrolyte offenbart. Die Herstellung der Polymerdispersionen in Gegenwart wenigstens eines Vernetzers ist nicht beschrieben.

Die unveröffentlichte internationale Anmeldung PCT/EP03/04647 beschreibt kosmetische oder pharmazeutische Mittel, die wenigstens ein wasserlösliches oder wasserdispergierbares Copolymer enthalten, das durch radikalische Copolymerisation eines Monomergemischs, enthaltend (Meth)acrylsäureamid, wenigstens ein amidgruppenhaltiges Monomer und gegebenenfalls weiterer damit copolymerisierbarer Verbindungen, erhältlich ist. Als weitere Monomere können auch Vernetzer eingesetzt werden.

Die unveröffentlichte deutsche Patentanmeldung P 102 61 750.3 beschreibt ein ampholytisches Copolymer, das durch radikalische Copolymerisation von

a) wenigstens einer ethylenisch ungesättigten Verbindung mit mindestens einer anionogenen und/oder anionischen Gruppe,

b) wenigstens einer ethylenisch ungesättigten Verbindung mit mindestens einer kationogenen und/oder kationischen Gruppe,

c) wenigstens einer ungesättigten amidgruppenhaltigen Verbindung

sowie gegebenenfalls weiterer Comonomere erhältlich ist. Zur Herstellung dieser ampholytischen Copolymere können auch Vernetzer eingesetzt werden. Beschrieben sind weiterhin Polyelektrolyt-Komplexe, die ein solches ampholytisches Copolymer enthalten sowie kosmetische oder pharmazeutische Mittel auf Basis dieser ampholytischen Copolymere und Polyelektrolyt-Komplexe.

Die unveröffentlichte deutsche Patentanmeldung 102 37 378.7 beschreibt die Verwendung von Polymeren, die erhältlich sind durch

(i) radikalisch initiierte Copolymerisation von Monomergemischen aus

(a) mindestens einem kationischen Monomeren oder quaternisierbaren Monomeren,
(b) gegebenenfalls einem wasserlöslichen Monomeren,
(c) gegebenenfalls einem weiteren radikalisch copolymerisierbaren Monomeren,
(d) mindestens einem als Vernetzer wirkenden Monomeren mit mindestens zwei ethylenisch ungesättigten, nichtkonjugierten Doppelbindungen, und
(e) mindestens einem Regler,

(ii) anschließende Quaternisierung oder Protonierung des Polymeren, sofern als Monomeres (a) ein nicht oder nur partiell quaternisiertes Monomer eingesetzt wird,

in haarkosmetischen Zubereitungen.

Die unveröffentlichte deutsche Patentanmeldung 102 61 197.1 beschreibt eine wässrige Dispersion erhältlich durch radikalische Polymerisation von

a) mindestens einem N-Vinyl-haltigen Monomer,
b) mindestens einem polymeren Dispergiermitteln,
c) mindestens einem polymeren Fällungsagens,
d) mindestens einem Vernetzer,
e) gegebenenfalls weiteren Monomeren,
f) gegebenenfalls mindestens einem Regler,
g) gegebenenfalls einer Puffersubstanz,

wobei das Gewichtsverhältnis von b) zu c) im Bereich von 1:50 bis 1:0,02 liegt und deren Verwendung in kosmetischen Zubereitungen.

Trotz der umfangreichen Bemühungen besteht nach wie vor Verbesserungsbedarf bei den aus dem Stand der Technik bekannten Polymeren zur Erzeugung elastischer Frisuren bei gleichzeitig starker Festigung auch bei hoher Luftfeuchtigkeit, guter Auswaschbarkeit und gutem Griff des Haares. Der Verbesserungsbedarf besteht ebenso bei Polymeren zur Erzeugung von gut kämmbarem, entwirrbarem Haar und zur Konditionierung von Haut und Haar in ihren sensorisch erfassbaren Eigenschaften wie Griff, Volumen, Handhabbarkeit usw. Ferner sind klare wässrige Zubereitungen dieser Polymere wünschenswert, die sich demnach durch eine gute Verträglichkeit mit anderen Formulierungsbestandteilen auszeichnen.

Weiterhin besteht Bedarf nach Polymeren, die als Konditioniermittel für kosmetische Zubereitungen geeignet sind und die mit einem hohen Feststoffgehalt hergestellt werden können. Von besonderem Interesse sind Polymere, die einen hohen Feststoffgehalt haben, eine geringe Viskosität aufweisen unter gleichzeitigen guten anwendungstechnischen Eigenschaften (wie beispielsweise Kämmbarkeit).

Aufgabe der vorliegenden Erfindung war es, ein Konditioniermittel für kosmetische Zubereitungen, insbesondere Shampoos zu finden, welche die genannten Nachteile nicht aufweist.

Überraschenderweise wurde nun gefunden, dass diese Aufgabe durch wässrige Polymerdispersionen und die daraus durch Trocknen erhältlichen Polymerisate gelöst wird, die durch radikalische Polymerisation eines Monomergemischs, enthaltend wenigstens ein N-Vinyl-haltiges Monomer, wenigstens einen Vernetzer und wenigstens ein Monomer mit mindestens einer kationogenen und/oder kationischen Gruppe in einem wässrigen Medium in Gegenwart wenigstens eines polymeren anionischen Dispergiermittels erhältlich sind.

Gegenstand der Erfindung ist daher eine wässrige Polymerdispersion Pd), die erhältlich ist durch radikalische Polymerisation eines Monomergemischs M), enthaltend

a) wenigstens eine $\alpha,\beta$-ethylenisch ungesättigte amidgruppenhaltige Verbindung der allgemeinen Formel I

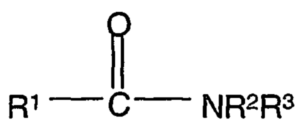

(I)

wobei

$R^2$ für eine Gruppe der Formel $CH_2=CR^4$- steht und $R^1$ und $R^3$ unabhängig voneinander für H, Alkyl, Cycloalkyl, Heterocycloalkyl, Aryl oder Hetaryl stehen, oder

$R^1$ und $R^3$ gemeinsam mit der Amidgruppe, an die sie gebunden sind, für ein Lactam mit 5 bis 8 Ringatomen stehen,

b) wenigstens eine radikalisch polymerisierbare vernetzende Verbindung mit wenigstens zwei $\alpha,\beta$-ethylenisch ungesättigten Doppelbindungen pro Molekül,

c) wenigstens eine Verbindung mit einer radikalisch polymerisierbaren $\alpha,\beta$-ethylenisch ungesättigten Doppelbindung und mindestens einer kationogenen und/oder kationischen Gruppe pro Molekül,

in einem wässrigen Medium in Gegenwart wenigstens eines polymeren anionischen Dispergiermittels D).

Gegenstand der Erfindung ist weiterhin ein durch Trocknen einer Polymerdispersion Pd) erhältliches Polymer P), kosmetische oder pharmazeutische Mittel, die eine solche Polymerdispersion oder ein solches Polymer enthalten sowie die Verwendung dieser Polymerdispersionen und Polymere.

Im Rahmen der vorliegenden Erfindung umfasst der Ausdruck Alkyl geradkettige und verzweigte Alkylgruppen. Geeignete kurzkettige Alkylgruppen sind z. B. geradkettige oder verzweigte $C_1$-$C_7$-Alkyl-, bevorzugt $C_1$-$C_6$-Alkyl- und besonders bevorzugt $C_1$-$C_4$-Alkylgruppen. Dazu zählen insbesondere Methyl, Ethyl, Propyl, Isopropyl, n-Butyl, 2-Butyl, sec.-Butyl, tert.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl- 2-methylpropyl, n-Heptyl, 2-Heptyl, 3-Heptyl, 2-Ethylpentyl, 1-Propylbutyl, Octyl etc.

Geeignete längerkettige $C_8$-$C_{30}$-Alkyl- bzw. $C_8$-$C_{30}$-Alkenylgruppen sind geradkettige und verzweigte Alkyl- bzw. Alkenylgruppen. Bevorzugt handelt es sich dabei um überwiegend lineare Alkylreste, wie sie auch in natürlichen oder synthetischen Fettsäuren und Fettalkoholen sowie Oxoalkoholen vorkommen, die gegebenenfalls zusätzlich einfach, zweifach oder mehrfach ungesättigt sein können. Dazu zählen z. B. n-Hexyl(en), n-Heptyl(en), n-Octyl(en), n-Nonyl(en), n-Decyl(en), n-Undecyl(en), n-Dodecyl(en), n-Tridecyl(en), n-Tetradecyl(en), n-Pentadecyl(en), n-Hexadecyl (en), n-Heptadecyl(en), n-Octadecyl(en), n-Nonadecyl(en) etc.

Cycloalkyl steht vorzugsweise für $C_5$-$C_8$-Cycloalkyl, wie Cyclopentyl, Cyclohexyl, Cycloheptyl oder Cyclooctyl.

Aryl umfasst unsubstituierte und substituierte Arylgruppen und steht vorzugsweise für Phenyl, Tolyl, Xylyl, Mesityl, Naphthyl, Fluorenyl, Anthracenyl, Phenanthrenyl, Naphthacenyl und insbesondere für Phenyl, Tolyl, Xylyl oder Mesityl.

Im Folgenden werden Verbindungen, die sich von Acrylsäure und Methacrylsäure ableiten können teilweise verkürzt durch Einfügen der Silbe "(meth)" in die von der Acrylsäure abgeleitete Verbindung bezeichnet.

Die erfindungsgemäßen Polymerdispersionen Pd) und Polymere P) eignen sich u. a. zur Formulierung von Gelen. "Gelförmige Konsistenz" zeigen Formulierungen, die eine höhere Viskosität als eine Flüssigkeit aufweisen und die selbsttragend sind, d. h. die eine ihnen verliehene Form ohne formstabilisierende Umhüllung behalten. Im Gegensatz zu festen Formulierungen lassen sich gelförmige Formulierungen jedoch leicht unter Anwendung von Scherkräften deformieren. Die Viskosität der gelförmigen Mittel liegen vorzugsweise in einem Bereich von größer als 600 bis etwa 60000 mPas, besonders bevorzugt von 6000 bis 30000 mPas. Vorzugsweise handelt es sich bei den Gelen um Haargele.

Unter wasserlöslichen Monomeren und Polymeren werden im Rahmen der vorliegenden Erfindung Monomere und Polymere verstanden, die sich zu mindestens 1 g/l bei 20 °C in Wasser lösen. Unter wasserdispergierbaren Monomeren und Polymeren werden Monomere und Polymere verstanden, die unter Anwendung von Scherkräften beispielsweise durch Rühren in dispergierbare Partikel zerfallen. Hydrophile Monomere sind vorzugsweise wasserlöslich oder zumindest wasserdispergierbar. Die erfindungsgemäßen Polymere P) sind im Allgemeinen in wässrigen Medien redispergierbar bzw. löslich.

Das zur Herstellung der erfindungsgemäßen Polymerdispersionen Pd) eingesetzte Monomergemisch M) weist Monomere mit kationogenen und/oder kationischen Gruppen auf. In einer geeigneten Ausführungsform können zur Polymerisation zusätzlich auch Monomere mit anionogenen und/oder anionischen Gruppen eingesetzt werden. Die Menge an zur Polymerisation eingesetzten Monomeren mit anionogenen und/oder anionischen Gruppen wird dabei so bemessen, dass bezogen auf die insgesamt zur Polymerisation eingesetzten Monomere der Molanteil an anionogenen und anionischen Gruppen geringer ist als der Molanteil an kationogenen und kationischen Gruppen. Die in den wässrigen Polymerdispersionen Pd) enthaltenen Polymere P) weisen daher im Mittel einen molaren Überschuss an kationogenen/kationischen Gruppen gegenüber anionogenen/anionischen Gruppen auf.

Zur Herstellung der erfindungsgemäßen wässrigen Polymerdispersionen werden vorzugsweise Monomere mit geladenen, d. h. mit kationischen Gruppen eingesetzt. Es ist jedoch auch möglich, diese Monomere in teilweise oder vollständig nicht protonierter und quaternisierter Form einzusetzen. Werden zur Herstellung der Dispersionen Pd) sowohl

Monomere mit kationogenen/kationischen Gruppen als auch Monomere mit anionogenen/anionischen Gruppen verwendet, so können diese sowohl in nicht geladener als auch in geladener Form eingesetzt werden. In einer geeigneten Ausführungsform erfolgt der Einsatz dieser Monomere gemeinsam, d. h. in Form so genannter "Salzpaare". Werden zur Herstellung bereits geladene Monomere eingesetzt, so leiten sich deren Gegenionen vorzugsweise von Säuren oder Basen ab, wie sie im Folgenden zur Einstellung des pH-Werts bei der Polymerisation oder der erhaltenen Polymerisate beschrieben werden.

Bei den polymeren anionischen Dispergiermitteln D) handelt es sich um Polyelektrolyte mit einer größeren Anzahl anionisch dissoziierbarer Gruppen in der Hauptkette und/oder einer Seitenkette. Zur Polymerisation können die Dispergiermittel D) in im Wesentlichen nicht geladener oder in teilweise oder vollständig geladener Form eingesetzt werden. Die Gegenionen, die die geladenen Gruppen der anionischen Dispergiermittel tragen, leiten sich vorzugsweise von Basen ab, wie sie im Folgenden zur Einstellung des pH-Werts bei der Polymerisation beschrieben werden. Die erfindungsgemäß eingesetzten polymeren anionischen Dispergiermittel D) sind befähigt, mit den in den Dispersionen Pd) enthaltenen Polymeren P) Polyelektrolyt-Komplexe (Symplexe) zu bilden.

Zur Polymerisation wird der pH-Wert des wässrigen Mediums im Allgemeinen auf einen Wert von 5 bis 10, bevorzugt 6 bis 8, besonders bevorzugt 6,5 bis 7,5 und insbesondere 6,8 bis 7 eingestellt. Es ist vorteilhaft, den pH-Wert während der Polymerisation in den zuvor genannten Bereichen zu halten. In vielen Fällen ist die Zugabe von pHregelnden Substanzen, wie Säuren, Basen oder Puffern, zum Polymerisationsgemisch nicht erforderlich, da die erfindungsgemäß eingesetzte Kombination aus Monomeren mit kationogenen/kationischen Gruppen und Dispergiermitteln mit anionogenen/anionischen Gruppen bereits als Puffer wirken und die pH-Werte des wässrigen Polymerisationsmediums in einem weiten Bereich gegen Verdünnung und Säure- oder Basenzusatz stabil sind. In einer geeigneten Ausführungsform ist es jedoch auch möglich, zur Polymerisation einen Puffer zuzusetzen. Geeignete Puffergemische sind beispielsweise in Römpp, Chemielexikon, 9. Auflage, Paperback-Ausgabe, Band 5, S. 3677 - 3678, Verlag Thieme (1995) beschrieben, worauf hier Bezug genommen wird. Des Weiteren ist es auch möglich, den pH-Wert des Polymerisationsgemischs während der Polymerisation beispielsweise durch eine Einstabmesskette zu bestimmen und durch Zugabe von Säure oder Base im bevorzugten pH-Bereich zu halten.

Zur Einstellung des pH-Werts bei der Polymerisation oder im Anschluss daran eignen sich prinzipiell alle anorganischen oder organischen Säuren und Basen, insbesondere solche, die wasserlöslich sind. Geeignete Säuren sind z. B. Carbonsäuren, wie Milchsäure, Zitronensäure oder Weinsäure oder Mineralsäuren, wie Phosphorsäure, Schwefelsäure oder Salzsäure. Geeignete Basen sind z. B. Alkali- und Erdalkalihydroxide, Ammoniak sowie primäre, sekundäre und tertiäre Amine, wie Triethylamin, sowie Aminoalkohole, wie Triethanolamin, Methyldiethanolamin, Dimethylethanolamin oder 2-Amino-2-methylpropanol. Als Puffer eignen sich vorzugsweise Salze der oben genannten schwachen Säuren, bevorzugt Alkali- und Erdalkalimetallsalze, wie Natrium-, Kalium-, Ammonium- oder Magnesiumsalze. Bevorzugte Puffersubstanzen sind Natriumacetat, Natriumcitrat, Natriumpyrophosphat, Kaliumpyrophosphat, Natriumdihydrogenphosphat, Dinatriumhydrogenphosphat, Natriumhydrogencarbonat und/oder Natriumborat. Die genannten pH-einstellenden Substanzen können sowohl einzeln als auch in Mischungen eingesetzt werden. Die Puffersubstanzen können zur Einstellung des pH-Werts gemeinsam oder auch jeweils einzeln zugegeben werden.

Die Herstellung von vernetzten kationischen Polymeren nach herkömmlichen Polymerisationsverfahren, z. B. durch Lösungs- oder Substanzpolymerisation, führt in der Regel zu hochmolekularen polymeren Netzwerken, deren anwendungstechnische Eigenschaften im Hinblick auf einen Einsatz in kosmetischen und pharmazeutischen Mitteln noch verbesserungswürdig sind. So resultieren dabei im Allgemeinen Polymerzusammensetzungen, in denen die Polymere gelöst oder gequollen vorliegen und die sehr hohe Viskositäten aufweisen. Nach dem erfindungsgemäß eingesetzten Herstellverfahren werden Polymerdispersionen erhalten, d. h. Polymerzusammensetzungen, die sich durch einen größeren Anteil darin enthaltener dispergierter diskreter Teilchen auszeichnen. Es wird angenommen, dass hierbei auch die Vernetzung zum Teil erst in den Dispersionsteilchen erfolgt, so dass die resultierenden Dispersionen höhere Feststoffgehalte und niedrigere Viskositäten als die aus dem Stand der Technik bekannten vernetzten kationischen Polymerzusammensetzungen aufweisen. Anwendungstechnisch zeichnen sie sich beispielsweise durch eine verbesserte Wirksamkeit als Conditioner aus. Sie haben zudem in der Regel gute rheologische Eigenschaften, die in weiten Bereichen vom Vernetzungsgrad (Anteil Komponente b)) unabhängig sind.

Eine Bestimmung, inwieweit in Polymerzusammensetzungen die Polymere in Form dispergierter diskreter Teilchen bzw. gelöst oder gequollen vorliegen, kann mittels Trübungsmessung (Turbidimetrie) erfolgen. So streuen dispergierte diskrete Teilchen eingestrahltes Licht stärker als gelöste oder gequollene Polymerteilchen. Zur Messung kann der abgestrahlte Streulicht- oder Fluoreszenzstrahlungsanteil oder im einfachsten Falle die Extinktion des Durchlichts herangezogen werden.

Die erfindungsgemäß erhältlichen Dispersionen weisen in einer bevorzugten Ausführungsform einen LD-Wert kleiner gleich 30 %, insbesondere kleiner gleich 20 %, bevorzugt kleiner gleich 10 %, insbesondere kleiner gleich 5 %.

Die Bestimmung des LD-Wertes (Lichtdurchlässigkeit) bei wässrigen Polymerdispersionen wird z. B. im Vergleich zu reinem Wasser als Referenz bei einer Küvettenlänge von 2,5 cm bei 600 nm gemessen. Das Spektrophotometer (z. B. Hach: Spektrophotometer DR/2000, Messart "Transmission") wird zuerst mit reinem Wasser auf 100 % eingestellt.

Danach spült man die Küvette mehrmals mit der Dispersion, füllt die Dispersion in die Küvette und liest die Lichtdurchlässigkeit in % ab.

Das Polymerisationsmedium kann sowohl nur aus Wasser als auch aus Mischungen aus Wasser und wassermischbaren Flüssigkeiten, z. B. Alkoholen, wie z. B. Methanol, Ethanol, n-Propanol, Isopropanol etc. bestehen. Vorzugsweise wird nur Wasser verwendet.

Monomer a)

Als Monomere a) eignen sich N-Vinyllactame und deren Derivate, die z. B. einen oder mehrere $C_1$-$C_6$-Alkylsubstituenten, wie Methyl, Ethyl, n-Propyl, Isopropyl, n-Butyl, sec.-Butyl, tert.-Butyl etc., aufweisen können. Dazu zählen z. B. N-Vinylpyrrolidon, N-Vinylpiperidon, N-Vinylcaprolactam, N-Vinyl-5-methyl-2-pyrrolidon, N-Vinyl-5-ethyl-2-pyrrolidon, N-Vinyl-6-methyl-2-piperidon, N-Vinyl-6-ethyl-2-piperidon, N-Vinyl-7-methyl-2-caprolactam, N-Vinyl-7-ethyl-2-caprolactam etc. Bevorzugt werden N-Vinylpyrrolidon und N-Vinylcaprolactam eingesetzt.

Als Monomere a) geeignete offenkettige N-Vinylamidverbindungen sind beispielsweise N-Vinylformamid, N-Vinyl-N-methylformamid, N-Vinylacetamid, N-Vinyl-N-methylacetamid, N-Vinyl-N-ethylacetamid, N-Vinylpropionamid, N-Vinyl-N-methylpropionamid und N-Vinylbutyramid.

In einer bevorzugten Ausführungsform der Erfindung wird als Monomer a) ein N-Vinyllactam, insbesondere N-Vinylpyrrolidon, eingesetzt.

Vernetzer b)

Monomere b), die eine vernetzende Funktion besitzen, sind Verbindungen mit mindestens zwei ethylenisch ungesättigten, nichtkonjugierten Doppelbindungen im Molekül.

Geeignete Vernetzer b) sind zum Beispiel Acrylester, Methacrylester, Allylether oder Vinylether von mindestens zweiwertigen Alkoholen. Die OH-Gruppen der zugrundeliegenden Alkohole können dabei ganz oder teilweise verethert oder verestert sein; die Vernetzer enthalten aber mindestens zwei ethylenisch ungesättigte Gruppen.

Beispiele für die zugrundeliegenden Alkohole sind zweiwertige Alkohole wie 1,2-Ethandiol, 1,2-Propandiol, 1,3-Propandiol, 1,2-Butandiol, 1,3-Butandiol, 2,3-Butandiol, 1,4-Butandiol, But-2-en-1,4-diol, 1,2-Pentandiol, 1,5-Pentandiol, 1,2-Hexandiol, 1,6-Hexandiol, 1,10-Decandiol, 1,2-Dodecandiol, 1,12-Dodecandiol, Neopentylglykol, 3-Methylpentan-1,5-diol, 2,5-Dimethyl-1,3-hexandiol, 2,2,4-Trimethyl-1,3-pentandiol, 1,2-Cyclohexandiol, 1,4-Cyclohexandiol, 1,4-Bis(hydroxymethyl)cyclohexan, Hydroxypivalinsäure-neopentylglykolmonoester, 2,2-Bis(4-hydroxyphenyl)-propan, 2,2-Bis[4-(2-hydroxypropyl)phenyl]propan, Diethylenglykol, Triethylenglykol, Tetraethylenglykol, Dipropylenglykol, Tripropylenglykol, Tetrapropylenglykol, 3-Thio-pentan-1,5-diol, sowie Polyethylenglykole, Polypropylenglykole und Polytetrahydrofurane mit Molekulargewichten von jeweils 200 bis 10000. Außer den Homopolymerisaten des Ethylenoxids bzw. Propylenoxids können auch Blockcopolymerisate aus Ethylenoxid oder Propylenoxid oder Copolymerisate, die Ethylenoxid- und Propylenoxid-Gruppen eingebaut enthalten, eingesetzt werden. Beispiele für zugrundeliegende Alkohole mit mehr als zwei OH-Gruppen sind Trimethylolpropan, Glycerin, Pentaerythrit, 1,2,5-Pentantriol, 1,2,6-Hexantriol, Triethoxycyanursäure, Sorbitan, Zucker wie Saccharose, Glucose, Mannose. Selbstverständlich können die mehrwertigen Alkohole auch nach Umsetzung mit Ethylenoxid oder Propylenoxid als die entsprechenden Ethoxylate bzw. Propoxylate eingesetzt werden. Die mehrwertigen Alkohole können auch zunächst durch Umsetzung mit Epichlorhydrin in die entsprechenden Glycidylether überführt werden.

Weitere geeignete Vernetzer b) sind die Vinylester oder die Ester einwertiger, ungesättigter Alkohole mit ethylenisch ungesättigten $C_3$-$C_6$-Carbonsäuren, beispielsweise Acrylsäure, Methacrylsäure, Itaconsäure, Maleinsäure oder Fumarsäure. Beispiele für solche Alkohole sind Allylalkohol, 1 -Buten-3-ol, 5-Hexen-1-ol, 1-Octen-3-ol, 9-Decen-1-ol, Dicyclopentenylalkohol, 10-Undecen-1-ol, Zimtalkohol, Citronellol, Crotylalkohol oder cis-9-Octadecen-1-ol. Man kann aber auch die einwertigen, ungesättigten Alkohole mit mehrwertigen Carbonsäuren verestern, beispielsweise Malonsäure, Weinsäure, Trimellithsäure, Phthalsäure, Terephthalsäure, Zitronensäure oder Bernsteinsäure.

Weitere geeignete Vernetzer b) sind Ester ungesättigter Carbonsäuren mit den oben beschriebenen mehrwertigen Alkoholen, beispielsweise der Ölsäure, Crotonsäure, Zimtsäure oder 10-Undecensäure.

Geeignet als Monomere b) sind außerdem geradkettige oder verzweigte, lineare oder cyclische, aliphatische oder aromatische Kohlenwasserstoffe, die über mindestens zwei Doppelbindungen verfügen, die bei aliphatischen Kohlenwasserstoffen nicht konjugiert sein dürfen, z. B. Divinylbenzol, Divinyltoluol, 1,7-Octadien, 1,9-Decadien, 4-Vinyl-1-cyclohexen, Trivinylcyclohexan oder Polybutadiene mit Molekulargewichten von 200 bis 20000.

Als Vernetzer sind ferner geeignet die Acrylsäureamide, Methacrylsäureamide und N-Allylamine von mindestens zweiwertigen Aminen. Solche Amine sind zum Beispiel 1,2-Diaminomethan, 1,2-Diaminoethan, 1,3-Diaminopropan, 1,4-Diaminobutan, 1,6-Diaminohexan, 1,12-Dodecandiamin, Piperazin, Diethylentriamin oder Isophorondiamin. Ebenfalls geeignet sind die Amide aus Allylamin und ungesättigten Carbonsäuren, wie Acrylsäure, Methacrylsäure, Itaconsäure, Maleinsäure, oder mindestens zweiwertigen Carbonsäuren, wie sie oben beschrieben wurden.

Ferner sind Triallylamin und Triallylmonoalkylammoniumsalze, z. B. Triallylmethylammoniumchlorid oder -methylsulfat, als Vernetzer b) geeignet.

Geeignet sind auch N-Vinyl-Verbindungen von Harnstoffderivaten, mindestens zweiwertigen Amiden, Cyanuraten oder Urethanen, beispielsweise von Harnstoff, Ethylenharnstoff, Propylenharnstoff oder Weinsäurediamid, z. B. N,N'-Divinylethylenharnstoff oder N,N'-Divinylpropylenharnstoff.

Weitere geeignete Vernetzer sind Divinyldioxan, Tetraallylsilan oder Tetravinylsilan.

Selbstverständlich können auch Mischungen der vorgenannten Verbindungen eingesetzt werden. Vorzugsweise werden wasserlösliche Vernetzer eingesetzt.

Besonders bevorzugt eingesetzte Vernetzer b) sind beispielsweise Methylenbisacrylamid, Triallylamin und Triallylalkylammoniumsalze, Divinylimidazol, Pentaerythrittriallylether, N,N'-Divinylethylenharnstoff, Umsetzungsprodukte mehrwertiger Alkohole mit Acrylsäure oder Methacrylsäure, Methacrylsäureester und Acrylsäureester von Polyalkylenoxiden oder mehrwertigen Alkoholen, die mit Ethylenoxid und/oder Propylenoxid und/oder Epichlorhydrin umgesetzt worden sind.

Ganz besonders bevorzugt als Vernetzer b) sind Pentaerythrittriallylether, Methylenbisacrylamid, N,N'-Divinylethylenharnstoff, Triallylamin und Triallylmonoalkylammoniumsalze und Acrylsäureester von Glykol, Butandiol, Trimethylolpropan oder Glycerin oder Acrylsäureester von mit Ethylenoxid und/oder Epichlorhydrin umgesetztem Glykol, Butandiol, Trimethylolpropan oder Glycerin.

Monomer c)

Bevorzugt handelt es sich bei den kationogenen bzw. kationischen Gruppen der Komponente c) um stickstoffhaltige Gruppen, wie primäre, sekundäre und tertiäre Aminogruppen sowie quaternäre Ammoniumgruppen. Vorzugsweise handelt es sich bei den stickstoffhaltigen Gruppen um tertiäre Aminogruppen oder quaternäre Ammoniumgruppen. Geladene kationische Gruppen lassen sich aus den Aminstickstoffen entweder durch Protonierung, z. B. mit einwertigen oder mehrwertigen Carbonsäuren, wie Milchsäure oder Weinsäure, oder Mineralsäuren, wie Phosphorsäure, Schwefelsäure und Salzsäure, oder durch Quaternisierung, z. B. mit Alkylierungsmitteln, wie $C_1$-$C_4$-Alkylhalogeniden oder -sulfaten, erzeugen. Beispiele solcher Alkylierungsmittel sind Ethylchlorid, Ethylbromid, Methylchlorid, Methylbromid, Dimethylsulfat und Diethylsulfat. In einer bevorzugten Ausführungsform werden die Monomere c) in geladener Form zur Polymerisation eingesetzt.

Geeignete Verbindungen c) sind z. B. die Ester von $\alpha,\beta$-ethylenisch ungesättigten Mono- und Dicarbonsäuren mit Aminoalkoholen. Bevorzugte Aminoalkohole sind $C_2$-$C_{12}$-Aminoalkoholen, welche am Aminstickstoff $C_1$-$C_8$-dialkyliert sind. Als Säurekomponente dieser Ester eignen sich z. B. Acrylsäure, Methacrylsäure, Fumarsäure, Maleinsäure, Itaconsäure, Crotonsäure, Maleinsäureanhydrid, Monobutylmaleat und Gemische davon. Bevorzugt werden Acrylsäure, Methacrylsäure und deren Gemische eingesetzt. Bevorzugt sind N,N-Dimethylaminomethyl(meth)acrylat, N,N-Dimethylaminoethyl(meth)acrylat, N,N-Diethylaminoethyl(meth)acrylat, N,N-Dimethylaminopropyl(meth)acrylat, N,N-Diethylaminopropyl(meth)acrylat und N,N-Dimethylaminocyclohexyl(meth)acrylat.

Geeignete Monomere c) sind weiterhin die Amide der zuvor genannten $\alpha,\beta$-ethylenisch ungesättigten Mono- und Dicarbonsäuren mit Diaminen, welche mindestens eine primäre oder sekundäre Aminogruppe aufweisen. Bevorzugt sind Diamine, die eine tertiäre und eine primäre oder sekundäre Aminogruppe aufweisen. Bevorzugt werden als Monomere c) N-[2-(dimethylamino)ethyl]acrylamid, N-[2-(dimethylamino)ethyl]methacrylamid, N-[3-(dimethylamino)propyl]acrylamid, N-[3-(dimethylamino)propyl]methacrylamid, N-[4-(dimethylamino)butyl]acrylamid, N-[4-(dimethylamino)- butyl]methacrylamid, N-[2-(diethylamino)ethyl]acrylamid, N-[4-(dimethylamino)cyclohexyl]acrylamid, N-[4-(dimethylamino)cyclohexyl]methacrylamid etc eingesetzt. Besonders bevorzugt werden N-[3-(dimethyl-amino)propyl]acrylamid und/oder N-[3-(dimethylamino)propyl]methacrylamid eingesetzt.

Geeignete Monomere c) sind weiterhin N,N-Diallylamine und N,N-Diallyl-N-alkylamine und deren Säureadditionssalze und Quaternisierungsprodukte. Alkyl steht dabei vorzugsweise für $C_1$-$C_{24}$-Alkyl. Bevorzugt sind N,N-Diallyl-N-methylamin und N,N-Diallyl-N,N-dimethylammonium-Verbindungen, wie z. B. die Chloride und Bromide.

Geeignete Monomere c) sind weiterhin vinyl- und allylsubstituierte Stickstoffheterocyclen, wie N-Vinylimidazol, N-Vinyl-2-methylimidazol, vinyl- und allylsubstituierte heteroaromatische Verbindungen, wie 2- und 4-Vinylpyridin, 2- und 4-Allylpyridin, und die Salze davon.

Bevorzugte Monomere c) sind die N-Vinylimidazol-Derivate der allgemeinen Formel (II), worin $R^1$ bis $R^3$ für Wasserstoff, $C_1$-$C_4$-Alkyl oder Phenyl steht

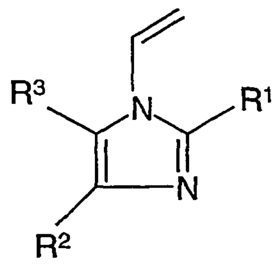

(II)

Beispiele für Verbindungen der allgemeinen Formel (II) sind folgender Tabelle 1 zu entnehmen:

Tabelle 1

| $R^1$ | $R^2$ | $R^3$ |
|---|---|---|
| H | H | H |
| Me | H | H |
| H | Me | H |
| H | H | Me |
| Me | Me | H |
| H | Me | Me |
| Me | H | Me |
| Ph | H | H |
| H | Ph | H |
| H | H | Ph |
| Ph | Me | H |
| Ph | H | Me |
| Me | Ph | H |
| H | Ph | Me |
| H | Me | Ph |
| Me | H | Ph |
| Me = Methyl<br>Ph = Phenyl | | |

Bevorzugte Beispiele für Monomere c) sind 3-Methyl-1-vinylimidazoliumchlorid und -methosulfat, Dimethyldiallylammoniumchlorid sowie N,N-Dimethylaminoethylmethacrylat und N-[3-(dimethylamino)propyl]methacrylamid, die durch Methylchlorid, Dimethylsulfat oder Diethylsulfat quaternisiert wurden.

Besonders bevorzugte Monomere c) sind 3-Methyl-1-vinylimidazoliumchlorid und -methosulfat und Dimethyldiallylammoniumchlorid (DADMAC), ganz besonders bevorzugt sind 3-Methyl-1-vinylimidazoliumchlorid und -methosulfat.

Eine bevorzugte Kombination der Komponenten a) und c) umfasst wenigstens ein N-Vinyllactam, insbesondere N-Vinylpyrrolidon, und wenigstens ein N-Vinylimidazol-Derivat, insbesondere ein quaternisiertes Vinylimidazol, und/oder Diallyldimethylammoniumchlorid.

Monomer d)

Die zur Herstellung der Polymerdispersionen Pd) eingesetzten Monomergemische M) können zusätzlich wenigstens ein weiteres Monomer d) enthalten. Die zusätzlichen Monomere d) sind vorzugsweise ausgewählt unter Estern α,β-ethylenisch ungesättigter Mono- und Dicarbonsäuren mit $C_1$-$C_{30}$-Alkanolen und $C_1$-$C_{30}$-Alkandiolen, Amiden α,β-ethylenisch ungesättigter Mono- und Dicarbonsäuren mit $C_2$-$C_{30}$-Aminoalkoholen, die eine primäre oder sekundäre Aminogruppe aufweisen, primären Amiden α,β-ethylenisch ungesättigter Monocarbonsäuren und deren N-Alkyl- und N,N-Dialkylderivaten, Estern von Vinylalkohol und Allylalkohol mit $C_1$-$C_{30}$-Monocarbonsäuren, Vinylethern, Vinylaromaten, Vinylhalogeniden, Vinylidenhalogeniden, $C_1$-$C_8$-Monoolefinen, nicht aromatischen Kohlenwasserstoffen mit mindestens zwei konjugierten Doppelbindungen und Mischungen davon.

Geeignete zusätzliche Monomere d) sind Methyl(meth)acrylat, Methylethacrylat, Ethyl(meth)acrylat, Ethylethacrylat, tert.-Butyl(meth)acrylat, tert.-Butylethacrylat, n-Octyl(meth)acrylat, 1,1,3,3-Tetramethylbutyl(meth)acrylat, Ethylhexyl(meth)acrylat, n-Nonyl(meth)acrylat, n-Decyl(meth)acrylat, n-Undecyl(meth)acrylat, Tridecyl(meth)acrylat, Myristyl(meth)acrylat, Pentadecyl(meth)acrylat, Palmityl(meth)acrylat, Heptadecyl(meth)acrylat, Nonadecyl(meth)acrylat, Arachinyl(meth)acrylat, Behenyl(meth)acrylat, Lignoceryl(meth)acrylat, Cerotinyl(meth)acrylat, Melissinyl(meth) acrylat, Palmitoleinyl(meth)acrylat, Oleyl(meth)acrylat, Linolyl(meth)acrylat, Linolenyl(meth)acrylat, Stearyl(meth)acrylat, Lauryl(meth)acrylat und Mischungen davon.

Geeignete zusätzliche Monomere d) sind weiterhin 2-Hydroxyethylacrylat, 2-Hydroxyethylmethacrylat, 2-Hydroxyethylethacrylat, 2-Hydroxypropylacrylat, 2-Hydroxypropylmethacrylat, 3-Hydroxypropylacrylat, 3-Hydroxypropylmethacrylat, 3-Hydroxybutylacrylat, 3-Hydroxybutylmethacrylat, 4-Hydroxybutylacrylat, 4-Hydroxybutylmethacrylat, 6-Hydroxyhexylacrylat, 6-Hydroxyhexylmethacrylat, 3-Hydroxy-2-ethylhexylacrylat, 3-Hydroxy-2-ethylhexylmethacrylat etc.

Geeignete zusätzliche Monomere d) sind weiterhin Acrylsäureamid, Methacrylsäureamid, N-Methyl(meth)acrylamid, N-Ethyl(meth)acrylamid, N-Propyl(meth)acrylamid, N-(n-Butyl)(meth)acrylamid, N-(tert.-Butyl)(meth)acrylamid, N-(n-Octyl)(meth)acrylamid, N-(1,1,3,3-Tetramethylbutyl)(meth)acrylamid, N-Ethylhexyl(meth)acrylamid, N-(n-Nonyl) (meth)acrylamid, N-(n-Decyl)(meth)acrylamid, N-(n-Undecyl)(meth)acrylamid, N-Tridecyl(meth)acrylamid, N-Myristyl (meth)acrylamid, N-Pentadecyl(meth)acrylamid, N-Palmityl(meth)acrylamid, N-Heptadecyl(meth)acrylamid, N-Nonadecyl(meth)acrylamid, N-Arachinyl(meth)acrylamid, N-Behenyl(meth)acrylamid, N-Lignoceryl(meth)acrylamid, N-Cerotinyl (meth)acrylamid, N-Melissinyl(meth)acrylamid, N-Palmitoleinyl(meth)acrylamid, N-Oleyl(meth)acrylamid, N-Linolyl(meth)acrylamid, N-Linolenyl(meth)acrylamid, N-Stearyl(meth)acrylamid, N-Lauryl(meth)acrylamid, N,N-Dimethyl(meth) acrylamid, N,N-Diethyl(meth)acrylamid, Morpholinyl(meth)acrylamid.

Geeignete zusätzliche Monomere d) sind weiterhin Vinylacetat, Vinylpropionat, Vinylbutyrat und Mischungen davon.

Geeignete zusätzliche Monomere d) sind weiterhin Ethylen, Propylen, Isobutylen, Butadien, Styrol, α-Methylstyrol, Acrylnitril, Methacrylnitril, Vinylchlorid, Vinylidenchlorid, Vinylfluorid, Vinylidenfluorid und Mischungen davon.

Die zuvor genannten zusätzlichen Monomere d) können einzeln oder in Form von beliebigen Mischungen eingesetzt werden.

Monomer e)

Das Monomergemisch M) kann zusätzlich wenigstens eine Verbindung e) mit einer radikalisch polymerisierbaren α,β-ethylenisch ungesättigten Doppelbindung und mit einer anionogenen und/oder anionischen Gruppe pro Molekül enthalten, mit der Maßgabe, dass der Molanteil an anionogenen und anionischen Gruppen der Komponente e) geringer ist als der Molanteil an kationogenen und kationischen Gruppen der Komponente c).

Vorzugsweise sind die Verbindungen e) ausgewählt unter monoethylenisch ungesättigten Carbonsäuren, Sulfonsäuren, Phosphonsäuren und Mischungen davon.

Zu den Monomeren e) zählen monoethylenisch ungesättigte Mono- und Dicarbonsäuren mit 3 bis 25 vorzugsweise 3 bis 6 C-Atomen, die auch in Form ihrer Salze oder Anhydride eingesetzt werden können. Beispiele hierfür sind Acrylsäure, Methacrylsäure, Ethacrylsäure, α-Chloracrylsäure, Crotonsäure, Maleinsäure, Maleinsäureanhydrid, Itaconsäure, Citraconsäure, Mesaconsäure, Glutaconsäure, Aconitsäure und Fumarsäure. Zu den Monomeren e) zählen weiterhin die Halbester von monoethylenisch ungesättigten Dicarbonsäuren mit 4 bis 10 vorzugsweise 4 bis 6 C-Atomen, z. B. von Maleinsäure wie Maleinsäuremonomethylester. Zu den Monomeren e) zählen auch monoethylenisch ungesättigte Sulfonsäuren und Phosphonsäuren, beispielsweise Vinylsulfonsäure, Allylsulfonsäure, Sulfoethylacrylat, Sulfoethylmethacrylat, Sulfopropylacrylat, Sulfopropylmethacrylat, 2-Hydroxy-3-acryloxypropylsulfonsäure, 2-Hydroxy-3-methacryloxypropylsulfonsäure, Styrolsulfonsäure, 2-Acrylamido-2-methylpropansulfonsäure, Vinylphosphonsäure und Allylphosphonsäure. Zu den Monomeren e) zählen auch die Salze der zuvor genannten Säuren, insbesondere die Natrium-, Kalium- und Ammoniumsalze sowie die Salze mit den zuvor genannten Aminen. Die Monomere e) können als solche oder als Mischungen untereinander eingesetzt werden. Die angegebenen Gewichtsanteile beziehen sich sämtlich auf die Säureform.

Vorzugsweise ist die Komponente e) ausgewählt unter Acrylsäure, Methacrylsäure, Ethacrylsäure, α-Chloracrylsäure, Crotonsäure, Maleinsäure, Maleinsäureanhydrid, Fumarsäure, Itaconsäure, Citraconsäure, Mesaconsäure, Glutaconsäure, Aconitsäure und Mischungen davon.

Besonders bevorzugt ist die Komponente e) ausgewählt unter Acrylsäure, Methacrylsäure, Itaconsäure und

Mischungen davon.

Dispergiermittel D)

Zur Herstellung der erfindungsgemäßen wässrigen Polymerdispersionen Pd) wird ein polymeres anionisches Dispergiermittel D) eingesetzt, das zur Dispergierung der bei der radikalischen Polymerisation des Monomergemischs M) erhaltenen Polymerisate dient.

Das zahlenmittlere Molekulargewicht der Dispergiermittel D) liegt vorzugsweise in einem Bereich von 500 bis 2000000, besonders bevorzugt 1000 bis 100000, insbesondere von 5000 bis 90000 und speziell von 10000 bis 700000.

Geeignete Dispergiermittel D) sind z. B. durch radikalische Polymerisation $\alpha,\beta$-ethylenisch ungesättigter Monomere erhältlich. Dabei werden Monomere eingesetzt, die wenigstens eine radikalisch polymerisierbare, $\alpha,\beta$-ethylenisch ungesättigte Doppelbindung und wenigstens eine anionogene und/oder anionische Gruppe pro Molekül enthalten. Zur Herstellung der Dispergiermittel D) können die zuvor genannten Monomere e) eingesetzt werden, worauf hier in vollem Umfang Bezug genommen wird.

Bevorzugte polymere Dispergiermittel D) sind Polymerisate, die mindestens 5 Gew.-%, besonders bevorzugt mindestens 10 Gew.-% und insbesondere mindestens 30 Gew.-% wenigstens eines Monomers mit einer radikalisch polymerisierbaren $\alpha,\beta$-ethylenisch ungesättigten Doppelbindung und einer anionogenen und/oder anionischen Gruppe pro Molekül, bezogen auf das Gesamtgewicht der zur Herstellung der Dispergiermittel D) eingesetzten Monomere, einpolymerisiert enthalten.

Prinzipiell eignen sich als Comonomere zur Herstellung der Dispergiermittel D) die zuvor als Komponenten des Monomergemischs M) genannten Komponenten a) bis d) mit der Maßgabe, dass der Molanteil an anionogenen und anionischen Gruppen, die das Dispergiermittel D) einpolymerisiert enthält, größer ist als der Molanteil an kationogenen und kationischen Gruppen. Die polymeren anionischen Dispergiermittel D) unterscheiden sich insofern immer von den durch radikalische Polymerisation des Monomergemischs M) erhaltenen Polymeren.

Vorzugsweise ist das anionische Dispergiermittel D) ausgewählt unter Polymeren, die wenigstens ein Monomer, das ausgewählt ist unter Acrylsäure, Methacrylsäure, Maleinsäure und Mischungen davon, einpolymerisiert enthalten.

Die Säuregruppen der Dispergiermittel D) können teilweise oder vollständig neutralisiert sein. Dann liegt wenigstens ein Teil der Säuregruppen in deprotonierter Form vor, wobei die Gegenionen vorzugsweise ausgewählt sind unter Alkalimetallionen, wie $Na^+$, $K^+$, Ammoniumionen und deren organischen Derivaten etc.

Bevorzugte Dispergiermittel D) sind z. B. Maleinsäure-Acrylsäure-Copolymerisate und Salze davon (z. B. Sokalan® CP 5 der BASF Aktienges.), Maleinsäure-Alkylvinylether-Copolymerisate, wie beispielsweise Maleinsäure/Methylvinylether-Copolymerisate und Salze davon (z. B. Sokalan® CP 2), Maleinsäure-Olefin-Copolymerisate und Salze davon (z. B. Sokalan® CP 9), Polyacrylsäure und Salze davon (z. B. Sokalan® CP 10), Maleinsäureanhydrid-Styrol-Copolymere, etc.

Die Herstellung dieser polymeren Dispergiermittel erfolgt nach bekannten Verfahren, zum Beispiel der Lösungs-, Fällungs-, Suspensions- oder Emulsionspolymerisation unter Verwendung von Verbindungen, die unter den Polymerisationsbedingungen Radikale bilden. Die Polymerisationstemperaturen liegen üblicherweise in dem Bereich von 30 bis 200 °C, vorzugsweise 40 bis 110 °C. Geeignete Initiatoren sind beispielsweise Azo- und Peroxyverbindungen sowie die üblichen Redoxinitiatorsysteme, wie Kombinationen aus Wasserstoffperoxid und reduzierend wirkenden Verbindungen, z. B. Natriumsulfit, Natriumbisulfit, Natriumformaldehydsulfoxilat und Hydrazin. Diese Systeme können gegebenenfalls zusätzlich noch geringe Mengen eines Schwermetallsalzes enthalten.

Einsatzmengen der Komponenten

Zur Herstellung der erfindungsgemäßen wässrigen Polymerdispersionen Pd) wird die Komponente a) vorzugsweise in einer Menge von 10 bis 90 Gew.-%, bevorzugt 20 bis 70 Gew.-%, insbesondere 30 bis 60 Gew.-%, bezogen auf das Gesamtgewicht der Komponente a) und des Dispergiermittels D), eingesetzt.

Das Dispergiermittel D) wird vorzugsweise in einer Menge von 10 bis 90 Gew.-%, bevorzugt 20 bis 70 Gew.-%, insbesondere 30 bis 60 Gew.-%, bezogen auf das Gesamtgewicht der Komponente a) und des Dispergiermittels D), eingesetzt.

Die Komponente b) wird vorzugsweise in einer Menge von 0,0005 bis 5 Gew.%, bevorzugt 0,001 bis 2,5 Gew.-%, insbesondere 0,01 bis 1,5 Gew.-%, bezogen auf das Gewicht der Komponente a), eingesetzt.

Zur Herstellung der erfindungsgemäßen wässrigen Polymerdispersion Pd) wird die Komponente c) vorzugsweise in einer Menge von 1 bis 40 Gew.-%, bevorzugt 5 bis 30 Gew.-%, bezogen auf das Gesamtgewicht der Komponente a) und des Dispergiermittels D), eingesetzt.

Regler

Die radikalische Polymerisation des Monomergemischs M) kann in Gegenwart mindestens eines Reglers erfolgen. Regler werden vorzugsweise in einer Einsatzmenge von 0,0005 bis 5 Gew.-%, besonders bevorzugt von 0,001 bis 2,5 Gew.-% und insbesondere von 0,01 bis 1,5 Gew.-%, bezogen auf das Gesamtgewicht der Komponente a) und des Dispergiermittels D), eingesetzt.

Als Regler (Polymerisationsregler) werden allgemein Verbindungen mit hohen Übertragungskonstanten bezeichnet. Regler beschleunigen Kettenübertragungsreaktionen und bewirken damit eine Herabsetzung des Polymerisationsgrades der resultierenden Polymeren, ohne die Bruttoreaktions-Geschwindigkeit zu beeinflussen.

Bei den Reglern kann man zwischen mono-, bi- oder polyfunktionalen Regler unterscheiden je nach Anzahl der funktionellen Gruppen im Molekül, die zu einen oder mehreren Kettenübertragungsreaktionen führen können. Geeignete Regler werden beispielsweise ausführlich beschrieben von K.C. Berger und G. Brandrup in J. Brandrup, E.H. Immergut, Polymer Handbook, 3. Aufl., John Wiley & Sons, New York, 1989, S. II/81 - II/141.

Als Regler eignen sich beispielsweise Aldehyde wie Formaldehyd, Acetaldehyd, Propionaldehyd, n-Butyraldehyd, Isobutyraldehyd.

Ferner können auch als Regler eingesetzt werden: Ameisensäure, ihre Salze oder Ester, wie Ammoniumformiat, 2,5-Diphenyl-1-hexen, Hydroxylammoniumsulfat, und Hydroxylammoniumphosphat.

Weitere geeignete Regler sind Halogenverbindungen, z. B. Alkylhalogenide, wie Tetrachlormethan, Chloroform, Bromtrichlormethan, Bromoform, Allylbromid, und Benzylverbindungen, wie Benzylchlorid oder Benzylbromid.

Weitere geeignete Regler sind Allylverbindungen, wie z. B. Allylalkohol, funktionalisierte Allylether, wie Allylethoxylate, Alkylallylether, oder Glycerinmonoallylether.

Bevorzugt werden als Regler Verbindungen eingesetzt, die Schwefel in gebundener Form enthalten.

Verbindungen dieser Art sind beispielsweise anorganische Hydrogensulfite, Disulfite und Dithionite oder organische Sulfide, Disulfide, Polysulfide, Sulfoxide und Sulfone. Dazu zählen Di-n-butylsulfid, Di-n-octylsulfid, Diphenylsulfid, Thiodiglykol, Ethylthioethanol, Diisopropyldisulfid, Di-n-butyldisulfid, Di-n-hexyldisulfid, Diacetyldisulfid, Diethanolsulfid, Di-t-butyltrisulfid, Dimethylsulfoxid, Dialkylsulfid, Dialkyldisulfid und/oder Diarylsulfid.

Besonders bevorzugt sind organische Verbindungen, die Schwefel in gebundener Form enthalten.

Bevorzugt als Polymerisationsregler eingesetzte Verbindungen sind Thiole (Verbindungen, die Schwefel in Form von SH-Gruppen erhalten, auch als Mercaptane bezeichnet). Bevorzugt sind als Regler mono-, bi- und polyfunktionale Mercaptane, Mercaptoalkohole und/oder Mercaptocarbonsäuren.

Beispiele für diese Verbindungen sind Allylthioglykolate, Ethylthioglykolat, Cystein, 2-Mercaptoethanol, 1,3-Mercaptopropanol, 3-Mercaptopropan-1,2-diol, 1,4-Mercaptobutanol, Mercaptoessigsäure, 3-Mercaptopropionsäure, Mercaptobernsteinsäure, Thioglycerin, Thioessigsäure, Thioharnstoff und Alkylmercaptane wie n-Butylmercaptan, n-Hexylmercaptan oder n-Dodecylmercaptan.

Besonders bevorzugte Thiole sind Cystein, 2-Mercaptoethanol, 1,3-Mercaptopropanol, 3-Mercaptopropan-1,2-diol, Thioglycerin, Thioharnstoff.

Beispiele für bifunktionale Regler, die zwei Schwefel in gebundener Form enthalten sind bifunktionale Thiole wie z. B. Dimercaptopropansulfonsäure (Natrium Salz), Dimercaptobernsteinsäure, Dimercapto-1-propanol, Dimercaptoethan, Dimercaptopropan, Dimercaptobutan, Dimercaptopentan, Dimercaptohexan, Ethylenglykol-bisthioglykolate und Butandiol-bis-thioglykolat.

Beispiele für polyfunktionale Regler sind Verbindungen, die mehr als zwei Schwefel in gebundener Form enthalten. Beispiele hierfür sind trifunktionale und/oder tetrafunktionale Mercaptane.

Bevorzugte trifunktionale Regler sind trifunktionale Mercaptane, wie z. B. Trimethylolpropan-tris(2-mercaptoethanat, Trimethylolpropan-tris(3-mercaptopropionat), Trimethylolpropan-tris(4-mercaptobutanat), Trimethylolpropan-tris(5-mercaptopentanat), Trimethylolpropan-tris(6-mercaptohexanat), Trimethylolpropan-tris(2-mercaptoacetat), Glycerylthioglycolat, Glycerylthiopropionat, Glycerylthioethylat, Glycerylthiobutanat, 1,1,1-Propanetriyl-tris-(mercaptoacetat), 1,1,1-Propanetriyl-tris-(mercaptoethanat), 1,1,1-Propanetriyl-tris-(mercaptoproprionat), 1,1,1-Propanetriyl-tris-(mercaptobutanat), 2-hydroxmethyl-2-methyl-1,3-propandiol-tris-(mercaptoacetat), 2-hydroxmethyl-2-methyl-1,3-propandiol-tris-(mercaptoethanat), 2-hydroxmethyl-2-methyl-1,3-propandiol-tris-(mercaptopropionat), 2-hydroxmethyl-2-methyl-1,3-propandiol-tris-(mercaptobutanat).

Besonders bevorzugte trifunktionale Regler sind Glycerylthioglycolat, Trimethylolpropan-tris(2-mercaptoacetat), 2-hydroxmethyl-2-methyl-1,3-propandiol tris-(mercaptoacetat).

Bevorzugte tetrafunktionale Mercaptane sind Pentaerythrit-tetrakis-(2-mercaptoacetat), Pentaerythrit-tetrakis-(2-mercaptoethanat), Pentaerythrit-tetrakis(3-mercaptopropionat), Pentaerythrit-tetrakis-(4-mercaptobutanat), Pentaerythrit-tetrakis(5-mercaptopentanat), Pentaerythrit-tetrakis-(6-mercaptohexanat).

Als weitere polyfunktionale Regler eignen sich Si-Verbindungen, die durch Umsetzung von Verbindungen der Formel (IVa) entstehen. Weiterhin eignen sich als polyfunktionale Regler Si-Verbindungen der Formel (IVb).

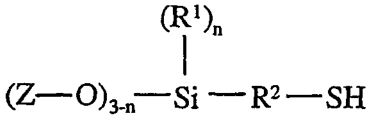 (IVa)

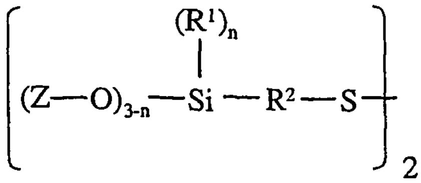 (IVb)

in der

n ein Wert von 0 bis 2 ist,

$R^1$ eine $C_1$-$C_{16}$-Alkylgruppe oder Phenylgruppe bedeutet,

$R^2$ eine $C_1$-$C_{18}$-Alkylgruppe, die Cyclohexyl- oder Phenylgruppe bezeichnet,

Z für eine $C_1$-$C_{18}$ Alkylgruppe, $C_2$-$C_{18}$-Alkylengruppe oder $C_2$-$C_{18}$-Alkinylgruppe steht, deren Kohlenstoffatome durch nicht benachbarte Sauerstoff- oder Halogenatome ersetzt sein können, oder für eine der Gruppen

$N{=}C(R_3)_2$ oder 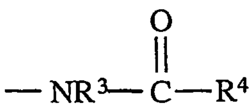

in denen

$R_3$ eine $C_1$-$C_{12}$-Alkylgruppe bedeutet und

$R_4$ eine $C_1$-$C_{18}$-Alkylgruppe bezeichnet.

Besonders bevorzugt sind die Verbindungen IVa, darunter vor allem Mercaptopropyltrimethoxysilan und Mercaptopropyltriethoxysilan.

Alle genannten Regler können einzeln oder in Kombination miteinander eingesetzt werden.

In einer bevorzugten Ausführungsform des Verfahrens werden multifunktionelle Regler eingesetzt.

Zur Herstellung der wässrigen Polymerdispersion Pd) wird das Monomergemisch M) in wässrigem Medium in Anwesenheit der Dispergiermittel D) in üblicher Weise polymerisiert, wie sie z. B. für Verfahren der radikalischen wässrigen Emulsionspolymerisation üblich sind. Das Polymerisationsmedium kann dabei sowohl nur aus Wasser, als auch aus Wasser und wassermischbaren Flüssigkeiten, wie Alkoholen, z. B. Methanol, Ethanol, n-Propanol, Isopropanol, n-Butanol, tert.-Butanol etc. bestehen. Vorzugsweise wird als Polymerisationsmedium nur Wasser verwendet.

Die Polymerisation erfolgt im Allgemeinen bei Temperaturen in einem Bereich von 0 bis 150 °C, bevorzugt 20 bis 100 °C, besonders bevorzugt 30 bis 95 °C. Die Polymerisation erfolgt vorzugsweise bei Normaldruck, möglich ist jedoch auch eine Polymerisation unter erhöhtem Druck, beispielsweise dem Eigendruck der zur Polymerisation eingesetzten Komponenten. Gewünschtenfalls kann die Polymerisation in Gegenwart wenigstens eines Inertgases, wie z. B. Stickstoff, erfolgen.

Die Polymerisation kann sowohl als Batch-Prozess als auch in Form eines Zulaufverfahrens, einschließlich Stufen- und Gradientenfahrweise, durchgeführt werden. Bevorzugt erfolgt die Polymerisation als Zulaufverfahren, bei dem man einen Teil des Polymerisationsansatzes vorlegt und die übrigen Komponenten ganz oder teilweise, absatzweise oder kontinuierlich, gemeinsam oder in getrennten Zuläufen zu der Vorlage gibt. Bevorzugt wird dabei ein Teil der eingesetzten Monomere und wenigstens ein Teil des polymeren anionischen Dispergiermittels D) und wenigstens ein

Teil des wässrigen Mediums in einer Polymerisationszone vorgelegt, auf die Polymerisationstemperatur erwärmt, gegebenenfalls diese Vorlage anpolymerisiert, und anschließend der Rest des Polymerisationsansatzes über einen oder mehrere räumlich getrennte Zuläufe unter Aufrechterhaltung der Polymerisation der Polymerisationszone zugeführt. Üblicherweise werden dabei Polymerisationsinitiator und Monomere in getrennten Zuläufen zugegeben. Die Zuführung der Monomere kann einzeln oder in Form von Gemischen, in reiner oder in wässrigem Medium gelöster oder in emulgierter Form erfolgen.

Vorteilhafterweise ermöglicht der Einsatz der polymeren anionischen Dispergiermittel D) die Herstellung von wässrigen Polymerdispersionen ohne den Einsatz weiterer grenzflächenaktiver Substanzen. Die Dispergiermittel D) können jedoch auch im Gemisch mit anderen grenzflächenaktiven Substanzen als Zusatzstoffe angewendet werden.

Geeignete weitere grenzflächenaktive Zusatzstoffe sind die üblicherweise bei der Emulsionspolymerisation als Dispergiermittel eingesetzten Schutzkolloide und Emulgatoren, wie sie z. B. in Houben-Weyl, Methoden der organischen Chemie, Band XIV/1, Makromolekulare Stoffe, Georg-Thieme-Verlag, Stuttgart, 1961, S. 411 bis 420 beschrieben sind. Geeignete zusätzliche Schutzkolloide sind z. B. Polyvinylalkohole und teilverseifte Polyvinylacetate, Polyacrylate, Polyvinylpyrrolidon, Cellulose und Cellulosederivate, wie z. B. Methylcellulose, Hydroxyethylcellulose, Carboxymethylcellulose, Stärke und Stärkederivate, wie z. B. Cyanalkyletherstärke, Hydroxyalkyletherstärke, Carboxymethylstärke etc. Als Emulgatoren sind sowohl anionische, kationische als auch nichtionische Emulgatoren geeignet.

Vorzugsweise werden als grenzflächenaktive Substanzen Emulgatoren eingesetzt, deren relative Molekulargewichte im Unterschied zu den Schutzkolloiden üblicherweise unter 3500 Dalton liegen.

Brauchbare nichtionische Emulgatoren sind araliphatische oder aliphatische nichtionische Emulgatoren, beispielsweise ethoxylierte Mono-, Di- und Trialkylphenole (EO-Grad: 3 bis 50, Alkylrest: $C_4$-$C_{10}$), Ethoxylate langkettiger Alkohole (EO-Grad: 3 bis 50, Alkylrest: $C_8$-$C_{36}$) sowie Polyethylenoxid/Polypropylenoxid-Blockcopolymere. Bevorzugt werden Ethoxylate langkettiger Alkanole (Alkylrest $C_{10}$-$C_{22}$, mittlerer Ethoxylierungsgrad 10 bis 50) und darunter besonders bevorzugt solche mit einem linearen $C_{12}$-$C_{18}$-Alkylrest und einem mittleren Ethoxylierungsgrad von 10 bis 50 sowie ethoxylierte Monoalkylphenole.

Geeignete anionische Emulgatoren sind beispielsweise Alkali- und Ammoniumsalze von Alkylsulfaten (Alkylrest: $C_8$-$C_{22}$), von Schwefelsäurehalbestern ethoxylierter Alkanole (EO-Grad: 2 bis 50, Alkylrest: $C_{12}$-$C_{18}$) und ethoxylierter Alkylphenole (EO-Grad: 3 bis 50, Alkylrest: $C_4$-$C_9$), von Alkylsulfonsäuren (Alkylrest: $C_{12}$-$C_{18}$) und von Alkylarylsulfonsäuren (Alkylrest: $C_9$-$C_{18}$). Weitere geeignete Emulgatoren finden sich in Houben-Weyl, Methoden der organischen Chemie, Band XIV/1, Makromolekulare Stoffe, Georg-Thieme-Verlag, Stuttgart, 1961, S. 192-208). Als anionische Emulgatoren sind ebenfalls Bis(phenoylsulfonsäure)ether bzw. deren Alkali- oder Ammoniumsalze, die an einem oder beiden aromatischen Ringen eine $C_4$-$C_{24}$-Alkylgruppe tragen, geeignet. Diese Verbindungen sind allgemein bekannt, z. B. aus der US-A-4,269,749, und im Handel erhältlich, beispielsweise als Dowfax® 2A1 (Dow Chemical Company).

Geeignete kationische Emulgatoren sind vorzugsweise quartäre Ammoniumhalogenide, z. B. Trimethylcetylammoniumchlorid, Methyltrioctylammoniumchlorid, Benzyltriethylammoniumchlorid oder quartäre Verbindungen von N-$C_6$-$C_{20}$-Alkylpyridinen, -morpholinen oder -imidazolen, z. B. N-Laurylpyridiniumchlorid.

Wird einer der vor genannten Emulgatoren verwendet, so ist dieser nach Maßgabe seiner Verträglichkeit mit dem jeweiligen anionischen polymeren Dispergiermittel D) auszuwählen. Die Menge an Emulgator beträgt im Allgemeinen etwa 0 bis 10 Gew.-%, bevorzugt 0,01 bis 5 Gew.-%, bezogen auf die Menge an zu polymerisierenden Monomeren.

Zur Herstellung der Polymerdispersionen Pd) können weiterhin von den polymeren anionischen Dispergiermitteln D) verschiedene polymere Dispergiermittel eingesetzt werden. Diese zusätzlichen polymeren Dispergiermittel werden im Allgemeinen in Mengen von 0 bis 10 Gew.-%, bevorzugt 0,01 bis 5 Gew.-%, bezogen auf die Menge an zu polymerisierenden Monomeren eingesetzt.

Die zusätzlichen polymeren Dispergiermittel enthalten im Allgemeinen wenigstens eine funktionelle Gruppe, ausgewählt aus Ether-, Hydroxyl-, Sulfatester-, Amino-, Imino-, tert.-Amino-, und/oder quaternären Ammoniumgruppen. Beispiele für solche Verbindungen sind: Polyvinylacetat, Polyalkylenglykole, insbesonderen Polyethylenglykole, Polyvinylalkohol, Polyvinylpyridin, Polyethylenimin, Polyvinylimidazol, Polyvinylsuccinimid und Polydiallyldimethylammoniumchlorid, Polyvinylpyrrolidon, Polymerisaten, die mindestens 5 Gew.-% an Vinylpyrrolidon-Einheiten enthalten, Polymerisaten, die mindestens 50 Gew.-% an Vinylalkohol-Einheiten enthalten, Oligosacchariden, Polysacchariden, oxidativ, hydrolytisch oder enzymatisch abgebauten Polysacchariden, chemisch modifizierten Oligo- oder Polysacchariden wie beispielsweise Carboxymethylcellulose, wasserlösliche Stärke und Stärkederivate, Stärkeester, Stärkexanthanogenate, Stärkeacetate, Dextran, und deren Mischungen.

Den Polymerdispersionen können weiterhin übliche Hilfs- und Zusatzstoffe zugesetzt werden. Dazu zählen beispielsweise den pH-Wert einstellende Substanzen, Reduktions- und Bleichmittel, wie z. B. die Alkalimetallsalze der Hydroxymethansulfinsäure (z. B. Rongallit® C der BASF Aktienges.), Komplexbildner, Desodorantien, Geruchsstoffe und Viskositätsveränderer, wie Alkohole, z. B. Glycerin, Methanol, Ethanol, tert.-Butanol, Glykol etc. Diese Hilfs- und Zusatzstoffe können den Polymerdispersionen in der Vorlage, einem der Zuläufe oder nach Abschluss der Polymerisation zugesetzt werden.

Die Dispersionen Pd) haben in der Regel eine Viskosität von 100 bis 50000 mPas, bevorzugt von 200 bis

20000 mPas, besonders bevorzugt von 300 bis 15000 mPas.

Die bei der Polymerisation entstandenen Dispersionen können im Anschluss an den Polymerisationsprozess einer physikalischen oder chemischen Nachbehandlung unterworfen werden. Solche Verfahren sind beispielsweise die bekannten Verfahren zur Restmonomerenreduzierung wie z. B. die Nachbehandlung durch Zusatz von Polymerisationsinitiatoren oder Mischungen mehrerer Polymerisationsinitiatoren bei geeigneten Temperaturen oder Erhitzen der Polymerisationslösung auf Temperaturen oberhalb der Polymerisationstemperatur, eine Nachbehandlung der Polymerlösung mittels Wasserdampf oder Strippen mit Stickstoff oder Behandeln der Reaktionsmischung mit oxidierenden oder reduzierenden Reagenzien, Adsorptionsverfahren wie die Adsorption von Verunreinigung an ausgewählten Medien wie z. B. Aktivkohle oder eine Ultrafiltration. Es können sich auch die bekannten Aufarbeitungsschritte anschließen, beispielsweise geeignete Trockenverfahren oder Walzentrocknung oder an die Trocknung anschließende Agglomerationsverfahren. Die nach dem erfindungsgemäßen Verfahren erhaltenen restmonomerenarmen Dispersionen können direkt in den Handel gebracht werden.

Die Polymerdispersionen Pd) können durch verschiedene Trocknungsverfahren, wie z. B. Sprühtrocknung, Fluidized Spray Drying, Walzentrockung oder Gefriertrocknung in Pulverform überführt werden. Bevorzugt wird die Sprühtrocknung eingesetzt. Die so erhaltenen Polymer-Trockenpulver lassen sich vorteilhafterweise durch Lösen bzw. Redispergieren in Wasser erneut in eine wässrige Lösung bzw. Dispersion überführen. Pulverförmige Copolymere haben den Vorteil einer besseren Lagerfähigkeit, einer einfacheren Transportmöglichkeit und zeigen in der Regel eine geringere Neigung für Keimbefall. Gegenstand der Erfindung sind auch die durch Trocknen einer Polymerdispersion Pd) erhältlichen Polymere P).

Die zuvor beschriebenen Polymerdispersionen Pd) und die daraus erhältlichen Polymere P) eignen sich hervorragend zur Herstellung kosmetischer und pharmazeutischer Mittel. Sie dienen dabei z. B. als polymere Filmbildner in Zubereitungen für die Körperpflege, was die Anwendung kosmetischer Zubereitungen auf keratinösen Oberflächen wie Haut, Haar, Nägel sowie auch Mundpflegepräparate beinhaltet. Sie sind universell in den verschiedensten kosmetischen Zubereitungen einsetzbar und einformulierbar und mit den üblichen Komponenten verträglich. Die erfindungsgemäßen Dispersionen zeichnen sich durch einen hohen Anteil an dispergierten Polymerteilchen und einen entsprechend niedrigen Anteil an gelösten oder gequollenen Polymeren aus. Bei gleichem Feststoffgehalt zeigen sie daher im Allgemeinen deutlich geringere Viskositäten als wässrige Zubereitungen auf Basis von aus dem Stand der Technik bekannten Polymeren. Sie erlauben somit die Formulierung von flüssigen bis gelförmigen Produkten mit höheren Feststoffgehalten und zeichnen sich durch verbesserte Conditioner-Eigenschaften aus.

Ein weiterer Gegenstand der Erfindung ist ein kosmetisches oder pharmazeutisches Mittel, enthaltend

A) wenigstens eine Polymerdispersion Pd), wie zuvor definiert, oder ein Polymer P), wie zuvor definiert, und

B) wenigstens einen kosmetisch akzeptablen Träger.

Die erfindungsgemäßen Mittel weisen einen kosmetisch oder pharmazeutisch akzeptablen Träger B) auf, der ausgewählt ist unter

i) Wasser,

ii) wassermischbaren organischen Lösungsmitteln, vorzugsweise $C_1$-$C_4$-Alkanolen,

iii) Ölen, Fetten, Wachsen,

iv) von iii) verschiedenen Estern von $C_6$-$C_{30}$-Monocarbonsäuren mit ein-, zwei- oder dreiwertigen Alkoholen,

v) gesättigten acyclischen und cyclischen Kohlenwasserstoffen,

vi) Fettsäuren,

vii) Fettalkoholen

und Mischungen davon.

Die erfindungsgemäßen Mittel weisen z. B. eine Öl- bzw. Fettkomponente B) auf, die ausgewählt ist unter: Kohlenwasserstoffen geringer Polarität, wie Mineralölen; linearen gesättigten Kohlenwasserstoffen, vorzugsweise mit mehr als 8 C-Atomen, wie Tetradecan, Hexadecan, Octadecan etc.; cyclischen Kohlenwasserstoffen, wie Decahydronaphthalin; verzweigten Kohlenwasserstoffen; tierischen und pflanzlichen Ölen; Wachsen; Wachsestern; Vaselin; Estern, bevorzugt Estern von Fettsäuren, wie z. B. die Ester von $C_1$-$C_{24}$-Monoalkoholen mit $C_1$-$C_{22}$-Monocarbonsäuren,

wie Isopropylisostearat, n-Propylmyristat, iso-Propylmyristat, n-Propylpalmitat, iso-Propylpalmitat, Hexacosanylpalmitat, Octacosanylpalmitat, Triacontanylpalmitat, Dotriacontanylpalmitat, Tetratriacontanylpalmitat, Hexacosanylstearat, Octacosanylstearat, Triacontanylstearat, Dotriacontanylstearat, Tetratriacontanylstearat; Salicylaten, wie $C_1$-$C_{10}$-Salicylaten, z. B. Octylsalicylat; Benzoatestern, wie $C_{10}$-$C_{15}$-Alkylbenzoaten, Benzylbenzoat; anderen kosmetischen Estern, wie Fettsäuretriglyceriden, Propylenglykolmonolaurat, Polyethylenglykolmonolaurat, $C_{10}$-$C_{15}$-Alkyllactaten, etc. und Mischungen davon.

Geeignete Siliconöle B) sind z. B. lineare Polydimethylsiloxane, Poly(methylphenylsiloxane), cyclische Siloxane und Mischungen davon. Das zahlenmittlere Molekulargewicht der Polydimethylsiloxane und Poly(methylphenylsiloxane) liegt vorzugsweise in einem Bereich von etwa 1000 bis 150000 g/mol. Bevorzugte cyclische Siloxane weisen 4- bis 8-gliedrige Ringe auf. Geeignete cyclische Siloxane sind z. B. unter der Bezeichnung Cyclomethicon kommerziell erhältlich.

Bevorzugte Öl- bzw. Fettkomponenten B) sind ausgewählt unter Paraffin und Paraffinölen; Vaselin; natürlichen Fetten und Ölen, wie Castoröl, Sojaöl, Erdnussöl, Olivenöl, Sonnenblumenöl, Sesamöl, Avocadoöl, Kakaobutter, Mandelöl, Pfirsichkernöl, Ricinusöl, Lebertran, Schweineschmalz, Walrat, Spermacetöl, Spermöl, Weizenkeimöl, Macadamianussöl, Nachtkerzenöl, Jojobaöl; Fettalkoholen, wie Laurylalkohol, Myristylalkohol, Cetylalkohol, Stearylalkohol, Oleylalkohol; Fettsäuren, wie Myristinsäure, Stearinsäure, Palmitinsäure, Ölsäure, Linolsäure, Linolensäure und davon verschiedenen gesättigten, ungesättigten und substituierten Fettsäuren; Wachsen, wie Bienenwachs, Carnaubawachs, Candilillawachs, Walrat sowie Mischungen der zuvor genannten Öl- bzw. Fettkomponenten.

Geeignete kosmetisch und pharmazeutisch verträgliche Öl- bzw. Fettkomponenten B) sind in Karl-Heinz Schrader, Grundlagen und Rezepturen der Kosmetika, 2. Auflage, Verlag Hüthig, Heidelberg, S. 319 - 355 beschrieben, worauf hier Bezug genommen wird.

Geeignete hydrophile Träger B) sind ausgewählt unter Wasser, 1-, 2- oder mehrwertigen Alkoholen mit vorzugsweise 1 bis 8 Kohlenstoffatomen, wie Ethanol, n-Propanol, iso-Propanol, Propylenglycol, Glycerin, Sorbit, etc.

Bei den erfindungsgemäßen kosmetischen Mitteln kann es sich um hautkosmetische, haarkosmetische, dermatologische, hygienische oder pharmazeutische Mittel handeln. Aufgrund ihrer filmbildenden Eigenschaften eignen sich die zuvor beschriebenen Polymere P) und Polymerdispersionen Pd) insbesondere als Zusatzstoffe für Haar- und Hautkosmetika.

Vorzugsweise liegen die erfindungsgemäßen Mittel in Form eines Gels, Schaums, Sprays, einer Salbe, Creme, Emulsion, Suspension, Lotion, Milch oder Paste vor. Gewünschtenfalls können auch Liposomen oder Mikrosphären eingesetzt werden.

Die erfindungsgemäßen kosmetisch oder pharmazeutisch aktiven Mittel können zusätzlich kosmetisch und/ oder dermatologisch aktive Wirkstoffe sowie Hilfsstoffe enthalten.

Vorzugsweise enthalten die erfindungsgemäßen kosmetischen Mittel wenigstens ein wie vorstehend definiertes Polymer P) oder eine Polymerdispersion Pd), wenigstens einen wie vorstehend definierten Träger B) und wenigstens einen, von P) oder Pd) verschiedenen Bestandteil, der ausgewählt ist unter kosmetisch aktiven Wirkstoffen, Emulgatoren, Tensiden, Konservierungsmitteln, Parfümölen, Verdickern, Haarpolymeren, Haar- und Hautconditionern, Pfropfpolymeren, wasserlöslichen oder dispergierbaren silikonhaltigen Polymeren, Lichtschutzmitteln, Bleichmitteln, Gelbildnern, Pflegemitteln, Färbemitteln, Tönungsmitteln, Bräunungsmitteln, Farbstoffen, Pigmenten, Konsistenzgebern, Feuchthaltemitteln, Rückfettern, Collagen, Eiweisshydrolysaten, Lipiden, Antioxidantien, Entschäumern, Antistatika, Emollienzien und Weichmachern.

Übliche Verdickungsmittel in derartigen Formulierungen sind vernetzte Polyacrylsäuren und deren Derivate, Polysaccharide wie Xanthan-gum, Agar-Agar, Alginate oder Tylosen, Cellulosederivate, z. B. Carboxymethylcellulose oder Hydroxycarboxymethylcellulose, Fettalkohole, Monoglyceride und Fettsäuren, Polyvinylalkohol und Polyvinylpyrrolidon. Geeignete Verdicker sind auch die Aculyn ®-Marken der Fa. Rohm und Haas, wie Aculyn® 22 (Copolymerisat aus Acrylaten und Methacrylsäureethoxilaten mit Stearylrest (20 EO-Einheiten)) und Aculyn® 28 (Copolymerisat aus Acrylaten und Methacrylsäureethoxilaten mit Behenylrest (25 EO-Einheiten)).

Geeignete kosmetisch und/oder dermatologisch aktive Wirkstoffe sind z. B. färbende Wirkstoffe, Haut- und Haarpigmentierungsmittel, Tönungsmittel, Bräunungsmittel, Bleichmittel, Keratin-härtende Stoffe, antimikrobielle Wirkstoffe, Lichtfilterwirkstoffe, Repellentwirkstoffe, hyperemisierend wirkende Stoffe, keratolytisch und keratoplastisch wirkende Stoffe, Antischuppenwirkstoffe, Antiphlogistika, keratinisierend wirkende Stoffe, antioxidativ bzw. als Radikalfänger aktive Wirkstoffe, hautbefeuchtende oder -feuchthaltende Stoffe, rückfettende Wirkstoffe, antierythimatös oder antiallergisch aktive Wirkstoffe und Mischungen davon.

Künstlich hautbräunende Wirkstoffe, die geeignet sind, die Haut ohne natürliche oder künstliche Bestrahlung mit UV-Strahlen zu bräunen, sind z. B. Dihydroxyaceton, Alloxan und Walnussschalenextrakt. Geeignete Keratin-härtende Stoffe sind in der Regel Wirkstoffe, wie sie auch in Antitranspirantien eingesetzt werden, wie z. B. Kaliumaluminiumsulfat, Aluminiumhydroxychlorid, Aluminiumlactat, etc. Antimikrobielle Wirkstoffe werden eingesetzt, um Mikroorganismen zu zerstören bzw. ihr Wachstum zu hemmen und dienen somit sowohl als Konservierungsmittel als auch als desodorierend wirkender Stoff, welcher die Entstehung oder die Intensität von Körpergeruch vermindert. Dazu zählen z. B. übliche, dem Fachmann bekannte Konservierungsmittel, wie p-Hydroxybenzoesäureester, Imidazolidinyl-Harnstoff,

Formaldehyd, Sorbinsäure, Benzoesäure, Salicylsäure, etc. Derartige desodorierend wirkende Stoffe sind z. B.

Zinkricinoleat, Triclosan, Undecylensäurealkylolamide, Citronensäuretriethylester, Chlorhexidin etc. Geeignete Lichtfilterwirkstoffe sind Stoffe, die UV-Strahlen im UV-B-und/oder UV-A-Bereich absorbieren. Geeignete UV-Filter sind z. B. 2,4,6-Triaryl-1,3,5-triazine, bei denen die Arylgruppen jeweils wenigstens einen Substituenten tragen können, der vorzugsweise ausgewählt ist unter Hydroxy, Alkoxy, speziell Methoxy, Alkoxycarbonyl, speziell Methoxycarbonyl und Ethoxycarbonyl und Mischungen davon. Geeignet sind weiterhin p-Aminobenzoesäureester, Zimtsäureester, Benzophenone, Campherderivate sowie UV-Strahlen abhaltende Pigmente, wie Titandioxid, Talkum und Zinkoxid. Geeignete Repellentwirkstoffe sind Verbindungen, die in der Lage sind, bestimmte Tiere, insbesondere Insekten, vom Menschen abzuhalten oder zu vertreiben. Dazu gehört z. B. 2-Ethyl-1,3-hexandiol, N,N-Diethyl-m-toluamid etc. Geeignete hyperemisierend wirkende Stoffe, welche die Durchblutung der Haut anregen, sind z. B. ätherische Öle, wie Latschenkiefer, Lavendel, Rosmarin, Wacholderbeer, Rosskastanienextrakt, Birkenblätterextrakt, Heublumenextrakt, Ethylacetat, Campher, Menthol, Pfefferminzöl, Rosmarinextrakt, Eukalyptusöl, etc. Geeignete keratolytisch und keratoplastisch wirkende Stoffe sind z. B. Salicylsäure, Kalziumthioglykolat, Thioglykolsäure und ihre Salze, Schwefel, etc. Geeignete Antischuppen-Wirkstoffe sind z. B. Schwefel, Schwefelpolyethylenglykolsorbitanmonooleat, Schwefelricinolpolyethoxylat, Zinkpyrithion, Aluminiumpyrithion, etc. Geeignete Antiphlogistika, die Hautreizungen entgegenwirken, sind z. B. Allantoin, Bisabolol, Dragosantol, Kamillenextrakt, Panthenol, etc.

Die erfindungsgemäßen kosmetischen Mittel können als kosmetischen und/oder pharmazeutischen Wirkstoff (wie auch gegebenenfalls als Hilfsstoff) wenigstens ein kosmetisch oder pharmazeutisch akzeptables von P) oder Pd) verschiedenes Polymer enthalten. Dazu zählen ganz allgemein anionische, kationische, amphotere und neutrale Polymere.

Beispiele für anionische Polymere sind Homo- und Copolymerisate von Acrylsäure und Methacrylsäure oder deren Salze, Copolymere von Acrylsäure und Acrylamid und deren Salze; Natriumsalze von Polyhydroxycarbonsäuren, wasserlösliche oder wasserdispergierbare Polyester, Polyurethane, z. B. Luviset PUR® der Fa. BASF, und Polyharnstoffe. Besonders geeignete Polymere sind Copolymere aus t-Butylacrylat, Ethylacrylat, Methacrylsäure (z. B. Luvimer® 100P), Copolymere aus Ethylacrylat und Methacrylsäure (z. B. Luviflex® Soft und Luvimer® MAE), Copolymere aus N-tert.-Butylacrylamid, Ethylacrylat, Acrylsäure (Ultrahold® 8, strong), Copolymere aus Vinylacetat, Crotonsäure und gegebenenfalls weitere Vinylester (z. B. Luviset® Marken), Maleinsäureanhydridcopolymere, gegebenenfalls mit Alkohol umgesetzt, anionische Polysiloxane, z. B. carboxyfunktionelle, t-Butylacrylat, Methacrylsäure (z. B. Luviskol® VBM), Copolymere von Acrylsäure und Methacrylsäure mit hydrophoben Monomeren, wie z. B. $C_4$-$C_{30}$-Alkylester der Meth (acrylsäure), $C_4$-$C_{30}$-Alkylvinylester, $C_4$-$C_{30}$-Alkylvinylether und Hyaluronsäure. Beispiele für anionische Polymere sind weiterhin Vinylacetat/Crotonsäure-Copolymere, wie sie beispielsweise unter den Bezeichnungen Resyn® (National Starch) und Gafset® (GAF) im Handel sind und Vinylpyrrolidon/Vinylacrylat-Copolymere, erhältlich beispielsweise unter dem Warenzeichen Luviflex® (BASF). Weitere geeignete Polymere sind das unter der Bezeichnung Luviflex® VBM-35 (BASF) erhältliche Vinylpyrrolidon/Acrylat-Terpolymer und Natriumsulfonat-haltige Polyamide oder Natriumsulfonat-haltige Polyester.

Weiterhin umfasst die Gruppe der zur Kombination mit den erfindungsgemäßen Polymerisaten geeigneten Polymere beispielhaft Balance® CR (National Starch; Acrylate Copolymer), Balance® 0/55 (National Starch; Acrylate Copolymer), Balance® 47 (National Starch; Octylacrylamid/Acrylate/Butylaminoethylmethacrylate-Copolymer), Aquaflex® FX 64 (ISP; Isobutylen/Ethylmaleimid/Hydroxyethylmaleimid-Copolymer), Aquaflex® SF-40 (ISP / National Starch; VPNinyl Caprolactam/DMAPA Acrylate Copolymer), Allianz® LT-120 (ISP / Rohm & Haas; Acrylat/C1-2 Succinat/Hydroxyacrylat Copolymer), Aquarez® HS (Eastman; Polyester-1), Diaformer® Z-400 (Clariant; Methacryloytethylbetain/Methacrylat-Copolymer), Diaformer® Z-711 (Clariant; Methacryloylethyl N-oxid/Methacrylat-Copolymer), Diaformer® Z-712 (Clariant; Methacryloylethyl N-oxide/Methacrylat-Copolymer), Omnirez® 2000 (ISP; Monoethylester von Poly(Methylvinylether/Maleinsäure in Ethanol), Amphomer® HC (National Starch; Acrylat/Octylacrylamid-Copolymer), Amphomer® 28-4910 (National Starch; Octylacrylamid/Acrylat/Butylaminoethylmethacrylat-Copolymer), Advantage® HC 37 (ISP; Terpolymer aus Vinylcaprolactam/Vinylpyrrolidon/Dimethylaminoethylmethacrylat), Advantage® LC55 und LC80 oder LC A und LC E, Advantage® Plus (ISP; VA/Butyl Maleate/Isobornyl Acrylate Copolymer), Aculyne® 258 (Rohm & Haas; Acrylat/ Hydroxyesteracrylat-Copolymer), Luviset® P.U.R. (BASF, Polyurethane-1), Luviflex® Silk (BASF), Eastman® AQ 48 (Eastman), Styleze® CC-10 (ISP; VP/DMAPA Acrylates Copolymer), Styleze® 2000 (ISP; VP/Acrylates/Lauryl Methacrylate Copolymer), DynamX (National Starch; Polyurethane-14 AMP-Acrylates Copolymer), Resyn XP (National Starch; Acrylates/Octylacrylamide Copolymer), Fixomer A-30 (Ondeo Nalco; polymethacrylic acid (and) acrylamidomethyl propane sulfonic acid), Fixate G-100 (Noveon; AMP-Acrylates/Allyl Methacrylate Copolymer).

Weitere geeignete Polymere sind kationische Polymere mit der Bezeichnung Polyquaternium nach INCI, z. B. Copolymere aus Vinylpyrrolidon/N-Vinylimidazoliumsalzen (Luviquat® FC, Luviquat® HM, Luviquat® MS, Luviquat® Care), Copolymere aus N-Vinylpyrrolidon/Dimethylaminoethylmethacrylat, quaternisiert mit Diethylsulfat (Luviquat® PQ 11), Copolymere aus N-Vinylcaprolactam/N-Vinylpyrrolidon/N-Vinylimidazoliumsalzen (Luviquat® Hold); kationische Cellulosederivate (Polyquaternium-4 und -10), Acrylamidocopolymere (Polyquaternium-7) und Chitosan. Geeignete kationische (quaternisierte) Polymere sind auch Merquat® (Polymer auf Basis von Dimethyldiallylammoniumchlorid), Gafquat® (quaternäre Polymere, die durch Reaktion von Polyvinylpyrrolidon mit quaternären Ammoniumverbindungen entstehen), Polymer JR (Hydroxyethylcellulose mit kationischen Gruppen) und kationische Polymere auf pflanzlicher Basis, z. B. Guarpolymere, wie die Jaguar®-Marken der Fa. Rhodia.

Weitere geeignete Polymere sind auch neutrale Polymere, wie Polyvinylpyrrolidone, Copolymere aus N-Vinylpyrrolidon und Vinylacetat und/oder Vinylpropionat, Polysiloxane, Polyvinylcaprolactam und andere Copolymere mit N-Vinylpyrrolidon, Polyethylenimine und deren Salze, Polyvinylamine und deren Salze, Cellulosederivate, Polyasparaginsäuresalze und Derivate. Dazu zählt beispielsweise Luviflex® Swing (teilverseiftes Copolymerisat von Polyvinylacetat und Polyethylenglykol, Fa. BASF).

Geeignete Polymere sind auch nichtionische, wasserlösliche bzw. wasserdispergierbare Polymere oder Oligomere, wie Polyvinylcaprolactam, z. B. Luviskol® Plus (BASF), oder Polyvinylpyrrolidon und deren Copolymere, insbesondere mit Vinylestern, wie Vinylacetat, z. B. Luviskol® VA 37 (BASF); Polyamide, z. B. auf Basis von Itaconsäure und aliphatischen Diaminen, wie sie z. B. in der DE-A-43 33 238 beschrieben sind.

Geeignete Polymere sind auch amphotere oder zwitterionische Polymere, wie die unter den Bezeichnungen Amphomer® (National Starch) erhältlichen Octylacrylamid/Methylmethacrylat/tert.-Butylaminoethylmethacrylat/2-Hydroxypropylmethacrylat-Copolymere sowie zwitterionische Polymere, wie sie beispielsweise in den deutschen Patentanmeldungen DE 39 29 973, DE 21 50 557, DE 28 17 369 und DE 37 08 451 offenbart sind. Acrylamidopropyltrimethylammoniumchlorid/Acrylsäure- bzw. -Methacrylsäure-Copolymerisate und deren Alkali- und Ammoniumsalze sind bevorzugte zwitterionische Polymere. Weiterhin geeignete zwitterionische Polymere sind Methacroylethylbetain/Methacrylat-Copolymere, die unter der Bezeichnung Amersette® (AMERCHOL) im Handel erhältlich sind, und Copolymere aus Hydroxyethylmethacrylat, Methylmethacrylat, N,N-Dimethylaminoethylmethacrylat und Acrylsäure (Jordapon®).

Geeignete Polymere sind auch nichtionische, siloxanhaltige, wasserlösliche oder -dispergierbare Polymere, z. B. Polyethersiloxane, wie Tegopren® (Fa. Goldschmidt) oder Belsil® (Fa. Wacker).

Die Formulierungsgrundlage erfindungsgemäßer pharmazeutischer Mittel enthält bevorzugt pharmazeutisch akzeptable Hilfsstoffe. Pharmazeutisch akzeptabel sind die im Bereich der Pharmazie, der Lebensmitteltechnologie und angrenzenden Gebieten bekanntermaßen verwendbaren Hilfsstoffe, insbesondere die in einschlägigen Arzneibüchern (z. B. DAB Ph. Eur. BP NF) gelisteten sowie andere Hilfsstoffe, deren Eigenschaften einer physiologischen Anwendung nicht entgegenstehen.

Geeignete Hilfsstoffe können sein: Gleitmittel, Netzmittel, emulgierende und suspendierende Mittel, konservierende Mittel, Antioxidantien, Antireizstoffe, Chelatbildner, Emulsionsstabilisatoren, Filmbildner, Gelbildner, Geruchsmaskierungsmittel, Harze, Hydrokolloide, Lösemittel, Lösungsvermittler, Neutralisierungsmittel, Permeationsbeschleuniger, Pigmente, quaternäre Ammoniumverbindungen, Rückfettungs- und Überfettungsmittel, Salben-, Creme- oder Öl-Grundstoffe, Siliconderivate, Stabilisatoren, Sterilantien, Treibmittel, Trocknungsmittel, Trübungsmittel, Verdickungsmittel, Wachse, Weichmacher, Weißöle. Eine diesbezügliche Ausgestaltung beruht auf fachmännischem Wissen, wie sie beispielsweise in Fiedler, H. P. Lexikon der Hilfsstoffe für Pharmazie, Kosmetik und angrenzende Gebiete, 4. Aufl., Aulendorf: ECV-Editio-Kantor-Verlag, 1996, dargestellt sind.

Zur Herstellung der erfindungsgemäßen dermatologischen Mittel können die Wirkstoffe mit einem geeigneten Hilfsstoff (Exzipient) vermischt oder verdünnt werden. Exzipienten können feste, halb feste oder flüssige Materialien sein, die als Vehikel, Träger oder Medium für den Wirkstoff dienen können. Die Zumischung weiterer Hilfsstoffe erfolgt gewünschtenfalls in der dem Fachmann bekannten Weise. Weiterhin sind die Polymere P) und Dispersionen Pd) geeignet als Hilfsmittel in der Pharmazie, bevorzugt als oder in Beschichtungsmittel(n) oder Bindemittel(n) für feste Arzneiformen. Sie können auch in Cremes und als Tablettenüberzugsmittel und Tablettenbindemittel verwendet werden.

Nach einer bevorzugten Ausführungsform handelt es sich bei den erfindungsgemäßen Mitteln um ein Hautreinigungsmittel.

Bevorzugte Hautreinigungsmittel sind Seifen von flüssiger bis gelförmiger Konsistenz, wie Transparentseifen, Luxusseifen, Deoseifen, Cremeseifen, Babyseifen, Hautschutzseifen, Abrasiveseifen und Syndets, pasteuse Seifen, Schmierseifen und Waschpasten, flüssige Wasch-, Dusch- und Badepräparate, wie Waschlotionen, Duschbäder und -gele, Schaumbäder, Ölbäder und Scrub-Präparate, Rasierschäume, -lotionen und - cremes.

Nach einer weiteren bevorzugten Ausführungsform handelt es sich bei den erfindungsgemäßen Mitteln um kosmetische Mittel zur Pflege und zum Schutz der Haut, Nagelpflegemittel oder Zubereitungen für die dekorative Kosmetik.

Geeignete hautkosmetische Mittel sind z. B. Gesichtswässer, Gesichtsmasken, Deodorantien und andere kosmetische Lotionen. Mittel für die Verwendung in der dekorativen Kosmetik umfassen beispielsweise Abdeckstifte, Theaterfarben, Mascara und Lidschatten, Lippenstifte, Kajalstifte, Eyelinern, Rouges, Puder und Augenbrauenstifte.

Außerdem können die Polymere P) und Dispersionen Pd) verwendet werden in Nose-Strips zur Porenreinigung, in Antiaknemitteln, Repellents, Rasiermitteln, Haarentfernungsmitteln, Intimpflegemitteln, Fußpflegemitteln sowie in der Babypflege.

Bei den erfindungsgemäßen Hautpflegemitteln handelt es sich insbesondere um W/O- oder O/W-Hautcremes, Tag- und Nachtcremes, Augencremes, Gesichtscremes, Antifaltencremes, Feuchthaltecremes, Bleichcremes, Vitamincremes, Hautlotionen, Pflegelotionen und Feuchthaltelotionen.

Hautkosmetische und dermatologische Mittel auf Basis der zuvor beschriebenen Polymere P) und Dispersionen Pd) zeigen vorteilhafte Wirkungen. Die Polymere können unter anderem zur Feuchthaltung und Konditionierung der Haut und zur Verbesserung des Hautgefühls beitragen. Die Polymere können auch als Verdicker in den Formulierungen wirken. Durch Zusatz der erfindungsgemäßen Polymere kann in bestimmten Formulierungen eine erhebliche Verbesserung der Hautverträglichkeit erreicht werden.

Hautkosmetische und dermatologische Mittel enthalten vorzugsweise wenigstens ein Polymer P), gegebenenfalls in Form einer Dispersion Pd) in einem Anteil von etwa 0,001 bis 30 Gew.-%, vorzugsweise 0,01 bis 20 Gew.-%, ganz besonders bevorzugt 0,1 bis 12 Gew.-%, bezogen auf das Gesamtgewicht des Mittels.

Besonders Lichtschutzmittel auf Basis der Polymere P) und Dispersionen Pd) besitzen die Eigenschaft, die Verweilzeit der UV-absorbierenden Inhaltsstoffe im Vergleich zu gängigen Hilfsmitteln wie Polyvinylpyrrolidon zu erhöhen.

Je nach Anwendungsgebiet können die erfindungsgemäßen Mittel in einer zur Hautpflege geeigneten Form, wie z. B. als Creme, Schaum, Gel, Stift, Mousse, Milch, Spray (Pumpspray oder treibmittelhaltiger Spray) oder Lotion appliziert werden.

Die hautkosmetischen Zubereitungen können neben den Polymeren P) und Dispersionen Pd) und geeigneten Trägern noch weitere in der Hautkosmetik übliche Wirkstoffe und Hilfsstoffe, wie zuvor beschrieben, enthalten. Dazu zählen vorzugsweise Emulgatoren, Konservierungsmittel, Parfümöle, kosmetische Wirkstoffe wie Phytantriol, Vitamin A, E und C, Retinol, Bisabolol, Panthenol, Lichtschutzmittel, Bleichmittel, Färbemittel, Tönungsmittel, Bräunungsmittel, Collagen, Eiweißhydrolysate, Stabilisatoren, pH-Wert-Regulatoren, Farbstoffe, Salze, Verdicker, Gelbildner, Konsistenzgeber, Silicone, Feuchthaltemittel, Rückfetter und weitere übliche Additive.

Bevorzugte Öl- und Fettkomponenten der hautkosmetischen und dermatologischen Mittel sind die zuvor genannten mineralischen und synthetischen Öle, wie z. B. Paraffine, Siliconöle und aliphatische Kohlenwasserstoffe mit mehr als 8 Kohlenstoffatomen, tierische und pflanzliche Öle, wie z. B. Sonnenblumenöl, Kokosöl, Avocadoöl, Olivenöl, Lanolin, oder Wachse, Fettsäuren, Fettsäureester, wie z. B. Triglyceride von $C_6$-$C_{30}$-Fettsäuren, Wachsester, wie z. B. Jojobaöl, Fettalkohole, Vaseline, hydriertes Lanolin und acetyliertes Lanolin sowie Mischungen davon.

Man kann die erfindungsgemäßen Polymere auch mit herkömmlichen Polymeren abmischen, falls spezielle Eigenschaften eingestellt werden sollen.

Zur Einstellung bestimmter Eigenschaften wie z. B. Verbesserung des Anfassgefühls, des Spreitverhaltens, der Wasserresistenz und/oder der Bindung von Wirk- und Hilfsstoffen, wie Pigmenten, können die hautkosmetischen und dermatologischen Zubereitungen zusätzlich auch konditionierende Substanzen auf Basis von Siliconverbindungen enthalten. Geeignete Siliconverbindungen sind beispielsweise Polyalkylsiloxane, Polyarylsiloxane, Polyarylalkylsiloxane, Polyethersiloxane oder Siliconharze.

Die Herstellung der kosmetischen oder dermatologischen Zubereitungen erfolgt nach üblichen, dem Fachmann bekannten Verfahren.

Bevorzugt liegen die kosmetischen und dermatologischen Mittel in Form von Emulsionen insbesondere als Wasser-in-Öl-(W/O)- oder Öl-in-Wasser(O/W)-Emulsionen vor. Es ist aber auch möglich, andere Formulierungsarten zu wählen, beispielsweise Hydrodispersionen, Gele, Öle, Oleogele, multiple Emulsionen, beispielsweise in Form von W/O/W- oder O/W/O-Emulsionen, wasserfreie Salben bzw. Salbengrundlagen, usw.

Die Herstellung von Emulsionen erfolgt nach bekannten Methoden. Die Emulsionen enthalten neben wenigstens einem Polymer P) in der Regel übliche Bestandteile, wie Fettalkohole, Fettsäureester und insbesondere Fettsäuretriglyceride, Fettsäuren, Lanolin und Derivate davon, natürliche oder synthetische Öle oder Wachse und Emulgatoren in Anwesenheit von Wasser. Die Auswahl der Emulsionstyp-spezifischen Zusätze und die Herstellung geeigneter Emulsionen ist beispielsweise beschrieben in Schrader, Grundlagen und Rezepturen der Kosmetika, Hüthig Buch Verlag, Heidelberg, 2. Auflage, 1989, dritter Teil, worauf hiermit ausdrücklich Bezug genommen wird.

Eine geeignete Emulsion, z. B. für eine Hautcreme etc., enthält im Allgemeinen eine wässrige Phase, die mittels eines geeigneten Emulgatorsystems in einer Öl- oder Fettphase emulgiert ist. Zur Bereitstellung der wässrigen Phase kann eine Polymerdispersion Pd) eingesetzt werden.

Der Anteil des Emulgatorsystems beträgt in diesem Emulsionstyp bevorzugt etwa 4 und 35 Gew.-%, bezogen auf das Gesamtgewicht der Emulsion. Vorzugsweise beträgt der Anteil der Fettphase etwa 20 bis 60 Gew.-%. Vorzugsweise beträgt der Anteil der wässrigen Phase etwa 20 bis 70 %, jeweils bezogen auf das Gesamtgewicht der Emulsion. Bei den Emulgatoren handelt es sich um solche, die in diesem Emulsionstyp üblicherweise verwendet werden. Sie werden z. B. ausgewählt unter: $C_{12}$-$C_{18}$-SorbitanFettsäureestern; Estern von Hydroxystearinsäure und $C_{12}$-$C_{30}$-Fettalkoholen; Mono- und Diestern von $C_{12}$-$C_{18}$-Fettsäuren und Glycerin oder Polyglycerin; Kondensaten von Ethylenoxid und Propylenglykolen; oxypropylenierten/oxyethylierten $C_{12}$-$C_{18}$-Fettalkoholen; polycyclischen Alkoholen, wie Sterolen; aliphatischen Alkoholen mit einem hohen Molekulargewicht, wie Lanolin; Mischungen von oxypropylenierten/polyglycerinierten Alkoholen und Magnesiumisostearat; Succinestern von polyoxyethylenierten oder polyoxypropylenierten Fettalkoholen; und Mischungen von Magnesium-, Calcium-, Lithium-, Zink- oder Aluminiumlanolat und hydriertem Lanolin

oder Lanolinalkohol.

Bevorzugte Fettkomponenten, welche in der Fettphase der Emulsionen enthalten sein können, sind: Kohlenwasserstofföle, wie Paraffinöl, Purcellinöl, Perhydrosqualen und Lösungen mikrokristalliner Wachse in diesen Ölen; tierische oder pflanzliche Öle, wie Süßmandelöl, Avocadoöl, Calophylumöl, Lanolin und Derivate davon, Ricinusöl, Sesamöl, Olivenöl, Jojobaöl, Karité-Öl, Hoplostethus-Öl; mineralische Öle, deren Destillationsbeginn unter Atmosphärendruck bei ca. 250 °C und deren Destillationsendpunkt bei 410 °C liegt, wie z. B. Vaselinöl; Ester gesättigter oder ungesättigter Fettsäuren, wie Alkylmyristate, z. B. i-Propyl-, Butyl- oder Cetylmyristat, Hexadecylstearat, Ethyl- oder i-Propylpalmitat, Octan- oder Decansäuretriglyceride und Cetylricinoleat.

Die Fettphase kann auch in anderen Ölen lösliche Siliconöle, wie Dimethylpolysiloxan, Methylphenylpolysiloxan und das Siliconglykol-Copolymer, Fettsäuren und Fettalkohole enthalten.

Um die Retention von Ölen zu begünstigen, können neben den Polymeren P) auch Wachse verwendet werden, wie z. B. Carnaubawachs, Candilillawachs, Bienenwachs, mikrokristallines Wachs, Ozokeritwachs und Ca-, Mg- und Al-Oleate, -Myristate, -Linoleate und -Stearate.

Im Allgemeinen werden die Wasser-in-Öl-Emulsionen so hergestellt, dass die Fettphase und der Emulgator in einen Ansatzbehälter gegeben werden. Man erwärmt diesen bei einer Temperatur von etwa 50 bis 75 °C, gibt dann die in Öl löslichen Wirkstoffe und/oder Hilfsstoffe zu und fügt unter Rühren Wasser hinzu, welches vorher etwa auf die gleiche Temperatur erwärmt wurde und worin man gegebenenfalls die wasserlöslichen Ingredienzien vorher gelöst hat. Man rührt, bis man eine Emulsion der gewünschten Feinheit erhält und lässt dann auf Raumtemperatur abkühlen, wobei gegebenenfalls weniger gerührt wird.

Weiterhin kann eine erfindungsgemäße Pflegeemulsion als O/W-Emulsion vorliegen. Eine derartige Emulsion enthält üblicherweise eine Ölphase, Emulgatoren, die die Ölphase in der Wasserphase stabilisieren, und eine wässrige Phase, die üblicherweise verdickt vorliegt.

Die wässrige Phase der O/W-Emulsion der erfindungsgemäßen Zubereitungen enthält gegebenenfalls:

- Alkohole, Diole oder Polyole sowie deren Ether, vorzugsweise Ethanol, Isopropanol, Propylenglycol, Glycerin, Ethylenglycolmonoethylether;
- übliche Verdickungsmittel bzw. Gelbildner, wie z. B. vernetzte Polyacrylsäuren und deren Derivate, Polysaccharide wie Xanthan Gum oder Alginate, Carboxymethylcellulose oder Hydroxycarboxymethylcellulose, Fettalkohole, Polyvinylalkohol und Polyvinylpyrrolidon.

Die Ölphase enthält in der Kosmetik übliche Ölkomponenten, wie beispielsweise:

- Ester aus gesättigten und/oder ungesättigten, verzweigten und/oder unverzweigten $C_3$-$C_{30}$-Alkancarbonsäuren und gesättigten und/oder ungesättigten, verzweigten und/oder unverzweigten $C_3$-$C_{30}$-Alkoholen, aus aromatischen Carbonsäuren und gesättigten und/oder ungesättigten, verzweigten und/oder unverzweigten $C_3$-$C_{30}$-Alkoholen, beispielhaft isopropylmyristat, Isopropylstearat, Hexyldecylstearat, Oleyloleat; außerdem synthetische, halbsynthetische und natürliche Gemische solcher Ester, wie Jojobaöl;
- verzweigte und/oder unverzweigte Kohlenwasserstoffe und -wachse;
- Silikonöle wie Cyclomethicon, Dimethylpolysiloxan, Diethylpolysiloxan, Octamethylcyclotetrasiloxan sowie Mischungen daraus;
- Dialkylether;
- Mineralöle und Mineralwachse;
- Triglyceride gesättigter und/oder ungesättigter, verzweigter und/oder unverzweigter $C_8$-$C_{24}$-Alkancarbonsäuren; sie können ausgewählt werden aus synthetischen, halbsynthetischen oder natürlichen Ölen, wie Olivenöl, Palmöl, Mandelöl oder Mischungen.

Als Emulgatoren kommen vorzugsweise O/W-Emulgatoren, wie Polyglycerinester, Sorbitanester oder teilveresterte Glyceride, in Betracht.

Die Herstellung kann durch Aufschmelzen der Ölphase bei ca. 80 °C erfolgen; die wasserlöslichen Bestandteile werden in heißem Wasser gelöst, langsam und unter Rühren zur Ölphase zugegeben; homogenisiert und kaltgerührt.

Nach einer weiteren bevorzugten Ausführungsform handelt es sich bei den erfindungsgemäßen Mitteln um ein Duschgel, eine Shampoo-Formulierung oder ein Badepräparat.

Solche Formulierungen enthalten wenigstens ein Polymer P) oder eine Dispersion Pd) sowie üblicherweise anionische Tenside als Basistenside und amphotere und/oder nichtionische Tenside als Cotenside. Weitere geeignete Wirkstoffe und/oder Hilfsstoffe sind im Allgemeinen ausgewählt unter Lipiden, Parfümölen, Farbstoffen, organischen Säuren, Konservierungsstoffen und Antioxidantien sowie Verdickern/Gelbildnern, Hautkonditioniermitteln und Feuchthaltemitteln.

Diese Formulierungen enthalten vorzugsweise 2 bis 50 Gew.-%, bevorzugt 5 bis 40 Gew.-%, besonders bevorzugt 8 bis 30 Gew.-% Tenside, bezogen auf das Gesamtgewicht der Formulierung.

In den Wasch-, Dusch- und Badepräparaten können alle in Körperreinigungsmitteln üblicherweise eingesetzten anionische, neutrale, amphotere oder kationische Tenside verwendet werden.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z. B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid- oder Propylenoxideinheiten, bevorzugt 1 bis 3 Ethylenoxideinheiten im Molekül aufweisen.

Dazu zählen z. B. Natriumlaurylsulfat, Ammoniumlaurylsulfat, Natriumlaurylethersulfat, Ammoniumlaurylethersulfat, Natriumlaurylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind z. B. Alkylbetaine, Alkylamidopropylbetaine, Alkylsulfobetaine, Alkylglycinate, Alkylcarboxyglycinate, Alkylamphoacetate oder -propionate, Alkylamphodiacetate oder -dipropionate.

Beispielsweise können Cocodimethylsulfopropylbetain, Laurylbetain, Cocamidopropylbetain oder Natriumcocamphopropionat eingesetzt werden.

Als nichtionische Tenside sind beispielsweise geeignet die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid. Die Menge Alkylenoxid beträgt ca. 6 bis 60 Mole auf ein Mol Alkohol. Ferner sind Alkylaminoxide, Mono- oder Dialkylalkanolamide, Fettsäureester von Polyethylenglykolen, ethoxylierte Fettsäureamide, Alkylpolyglycoside oder Sorbitanetherester geeignet.

Außerdem können die Wasch-, Dusch- und Badepräparate übliche kationische Tenside enthalten, wie z. B. quaternäre Ammoniumverbindungen, beispielsweise Cetyltrimethylammoniumchlorid.

Zusätzlich können auch weitere übliche kationische Polymere eingesetzt werden, so z. B. Copolymere aus Acrylamid und Dimethyldiallylammoniumchlorid (Polyquaternium-7), kationische Cellulosederivate (Polyquaternium-4, -10), Guarhydroxypropyltrimethylammoniumchlorid (INCI: Hydroxylpropyl Guar Hydroxypropyltrimonium Chloride), Copolymere aus N-Vinylpyrrolidon und quaternisiertem N-Vinylimidazol (Polyquaterinium-16, -44, -46), Copolymere aus N-Vinylpyrrolidon/Dimethylaminoethylmethacrylat, quaternisiert mit Diethylsulfat (Polyquaternium-11) und andere.

Weiterhin können die Duschgel-/Shampoo-Formulierungen Verdicker, wie z. B. Kochsalz, PEG-55, Propyleneglykol-Oleat, PEG-120-Methylglucosedioleat und andere, sowie Konservierungsmittel, weitere Wirk- und Hilfsstoffe und Wasser enthalten.

Nach einer weiteren bevorzugten Ausführungsform handelt es sich bei den erfindungsgemäßen Mitteln um ein Haarbehandlungsmittel.

Erfindungsgemäße Haarbehandlungsmittel enthalten vorzugsweise wenigstens ein Polymer P) in einer Menge im Bereich von etwa 0,1 bis 30 Gew.-%, bevorzugt 0,5 bis 20 Gew.-%, bezogen auf das Gesamtgewicht des Mittels.

Vorzugsweise liegen die erfindungsgemäßen Haarbehandlungsmittel in Form eines Schaumfestigers, Haarmousses, Haargels, Shampoos, Haarsprays, Haarschaums, Spitzenfluids, Egalisierungsmittels für Dauerwellen, Haarfärbe- und -bleichmittels oder "Hot-Oil-Treatments" vor. Je nach Anwendungsgebiet können die haarkosmetischen Zubereitungen als (Aerosol-)Spray, (Aerosol-)Schaum, Gel, Gelspray, Creme, Lotion oder Wachs appliziert werden. Haarsprays umfassen dabei sowohl Aerosolsprays als auch Pumpsprays ohne Treibgas. Haarschäume umfassen sowohl Aerosolschäume wie auch Pumpschäume ohne Treibgas. Haarsprays und Haarschäume umfassen vorzugsweise überwiegend oder ausschließlich wasserlösliche oder wasserdispergierbare Komponenten. Sind die in den erfindungsgemäßen Haarsprays und Haarschäumen eingesetzten Verbindungen wasserdispergierbar, können sie in Form von wässrigen Mikrodispersionen mit Teilchendurchmessern von üblicherweise 1 bis 350 nm, bevorzugt 1 bis 250 nm, zur Anwendung gebracht werden. Die Feststoffgehalte dieser Präparate liegen dabei üblicherweise in einem Bereich von etwa 0,5 bis 20 Gew.-%. Diese Mikrodispersionen benötigen in der Regel keine Emulgatoren oder Tenside zu ihrer Stabilisierung.

Die erfindungsgemäßen haarkosmetischen Formulierungen enthalten in einer bevorzugten Ausführungsform

a) 0,05 bis 20 Gew.-% wenigstens eines Polymers P),

b) 20 bis 99,95 Gew.-% Wasser und/oder Alkohol,

c) 0 bis 79,5 Gew.-% weitere Bestandteile.

Unter Alkohol sind alle in der Kosmetik üblichen Alkohole zu verstehen, z. B. Ethanol, Isopropanol, n-Propanol.

Unter weiteren Bestandteilen sind die in der Kosmetik üblichen Zusätze zu verstehen, beispielsweise Treibmittel, Entschäumer, grenzflächenaktive Verbindungen, d. h. Tenside, Emulgatoren, Schaumbildner und Solubilisatoren. Die eingesetzten grenzflächenaktiven Verbindungen können anionisch, kationisch, amphoter oder neutral sein. Weitere übliche Bestandteile können ferner sein z. B. Konservierungsmittel, Parfümöle, Trübungsmittel, Wirkstoffe, UV-Filter, Pflegestoffe wie Panthenol, Collagen, Vitamine, Eiweißhydrolysate, Alpha- und Beta-Hydroxycarbonsäuren, Stabilisatoren, pH-Wert-Regulatoren, Farbstoffe, Viskositätsregulierer, Gelbildner, Salze, Feuchthaltemittel, Rückfetter, Komplexbildner und weitere übliche Additive.

Weiterhin zählen hierzu alle in der Kosmetik bekannten Styling- und Conditionerpolymere, die in Kombination mit den erfindungsgemäßen Polymerisaten eingesetzt werden können, falls ganz spezielle Eigenschaften eingestellt werden sollen.

Als herkömmliche Haarkosmetik-Polymere eignen sich beispielsweise die zuvor genannten kationischen, anionischen, neutralen, nichtionischen und amphoteren Polymere, auf die hier Bezug genommen wird.

Zur Einstellung bestimmter Eigenschaften können die Zubereitungen zusätzlich auch konditionierende Substanzen auf Basis von Silikonverbindungen enthalten. Geeignete Silikonverbindungen sind beispielsweise Polyalkylsiloxane, Polyarylsiloxane, Polyarylalkylsiloxane, Polyethersiloxane, Silikonharze oder Dimethicon Copolyole (CTFA) und aminofunktionelle Silikonverbindungen wie Amodimethicone (CTFA).

Die erfindungsgemäßen Polymerisate eignen sich insbesondere als Festigungsmittel in Haarstyling-Zubereitungen, insbesondere Haarsprays (Aerosolsprays und Pumpsprays ohne Treibgas) und Haarschäume (Aerosolschäume und Pumpschäume ohne Treibgas).

In einer bevorzugten Ausführungsform enthalten diese Zubereitungen

a) 0,1 bis 10 Gew.-% wenigstens eines Polymers P),
b) 20 bis 99,9 Gew.-% Wasser und/oder Alkohol,
c) 0 bis 70 Gew.-% wenigstens eines Treibmittel,
d) 0 bis 20 Gew.-% weitere Bestandteile.

Treibmittel sind die für Haarsprays oder Aerosolschäume üblich verwendeten Treibmittel. Bevorzugt sind Gemische aus Propan/Butan, Pentan, Dimethylether, 1,1-Difluorethan (HFC-152 a), Kohlendioxid, Stickstoff oder Druckluft.

Eine erfindungsgemäß bevorzugte Formulierung für Aerosolhaarschäume enthält

a) 0,1 bis 10 Gew:-% wenigstens eines Polymers P),
b) 55 bis 99,8 Gew.-% Wasser und/oder Alkohol,
c) 5 bis 20 Gew.-% eines Treibmittel,
d) 0,1 bis 5 Gew.-% eines Emulgators,
e) 0 bis 10 Gew.-% weitere Bestandteile.

Als Emulgatoren können alle in Haarschäumen üblicherweise eingesetzten Emulgatoren verwendet werden. Geeignete Emulgatoren können nichtionisch, kationisch bzw. anionisch oder amphoter sein.

Beispiele für nichtionische Emulgatoren (INCI-Nomenklatur) sind Laurethe, z. B. Laureth-4; Cetethe, z. B. Cetheth-1, Polyethylenglycolcetylether; Ceatearethe, z. B. Cetheareth-25, Polyglycolfettsäureglyceride, hydroxyliertes Lecithin, Lactylester von Fettsäuren, Alkylpolyglycoside.

Beispiele für kationische Emulgatoren sind Cetyldimethyl-2-hydroxyethylammoniumdihydrogenphosphat, Cetyltrimoniumchlorid, Cetyltrimmoniumbromid, Cocotrimoniummethylsulfat, Quaternium-1 bis x (INCI).

Anionische Emulgatoren können beispielsweise ausgewählt werden aus der Gruppe der Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z. B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxid-Einheiten im Molekül aufweisen.

Eine erfindungsgemäß für Styling-Gele geeignete Zubereitung kann beispielsweise wie folgt zusammengesetzt sein:

a) 0,1 bis 10 Gew.-% wenigstens eines Polymers P),
b) 60 bis 99,85 Gew.-% Wasser und/oder Alkohol,
c) 0,05 bis 10 Gew.-% eines Gelbildners,

d) 0 bis 20 Gew.-% weitere Bestandteile.

Als Gelbildner können alle in der Kosmetik üblichen Gelbildner eingesetzt werden. Hierzu zählen leicht vernetzte Polyacrylsäure, beispielsweise Carbomer (INCI), Cellulosederivate, z. B. Hydroxypropylcellulose, Hydroxyethylcellulose, kationisch modifizierte Cellulosen, Polysaccharide, z. B. Xanthangummi, Capryl/Caprin-Triglycerid, Natriumacrylat-Copolymere, Polyquaternium-32 (und) Paraffinum Liquidum (INCI), Natriumacrylat-Copolymere (und) Paraffinum Liquidum (und) PPG-1 Trideceth-6, Acrylamidopropyltrimoniumchlorid/Acrylamid-Copolymere, Steareth-1 0 Allylether Acrylat-Copolymere, Polyquaternium-37 (und) Paraffinum Liquidum (und) PPG-1 Trideceth-6, Polyquaternium 37 (und) Propylenglycoldicapratdicaprylat (und) PPG-1 Trideceth-6, Polyquaternium-7, Polyquaternium-44.

Die erfindungsgemäßen Polymere P) und Dispersionen Pd) können in kosmetischen Zubereitungen als Konditioniermittel eingesetzt werden.

Die erfindungsgemäßen Polymere P) und Dispersionen Pd) können bevorzugt in Shampooformulierungen als Festigungs- und/oder Konditioniermittel eingesetzt werden. Bevorzugte Shampooformulierungen enthalten

a) 0,05 bis 10 Gew.-% wenigstens eines Polymers P),
b) 25 bis 94,95 Gew.-% Wasser,
c) 5 bis 50 Gew.-% Tenside,
d) 0 bis 5 Gew.-% eines weiteren Konditioniermittels,
e) 0 bis 10 Gew.-% weitere kosmetische Bestandteile.

In den Shampooformulierungen können alle in Shampoos üblicherweise eingesetzte anionische, neutrale, amphotere oder kationische Tenside verwendet werden.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z. B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxid-Einheiten im Molekül aufweisen.

Geeignet sind zum Beispiel Natriumlaurylsulfat, Ammoniumlaurylsulfat, Natriumlaurylethersulfat, Ammoniumlaurylethersulfat, Natriumlauroylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind zum Beispiel Alkylbetaine, Alkylamidopropylbetaine, Alkylsulfobetaine, Alkylglycinate, Alkylcarboxyglycinate, Alkylamphoacetate oder -propionate, Alkylamphodiacetate oder -dipropionate.

Beispielsweise können Cocodimethylsulfopropylbetain, Laurylbetain, Cocamidopropylbetain oder Natriumcocamphopropionat eingesetzt werden.

Als nichtionische Tenside sind beispielsweise geeignet die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid. Die Menge Alkylenoxid beträgt ca. 6 bis 60 Mole auf ein Mol Alkohol. Ferner sind Alkylaminoxide, Mono- oder Dialkylalkanolamide, Fettsäureester von Polyethylenglykolen, Alkylpolyglykoside oder Sorbitanetherester geeignet.

Außerdem können die Shampooformulierungen übliche kationische Tenside enthalten, wie z. B. quaternäre Ammoniumverbindungen, beispielsweise Cetyltrimethylammoniumchlorid.

In den Shampooformulierungen können zur Erzielung bestimmter Effekte übliche Konditioniermittel in Kombination mit den Polymerisaten P) eingesetzt werden. Hierzu zählen beispielsweise die zuvor genannten kationischen Polymere mit der Bezeichnung Polyquaternium nach INCI, insbesondere Copolymere aus Vinylpyrrolidon/N-Vinylimidazoliumsalzen (Luviquat® FC, Luviquat® HM, Luviquat® MS, Luviquat® Care), Copolymere aus N-Vinylpyrrolidon/Dimethylaminoethylmethacrylat, quaternisiert mit Diethylsulfat (Luviquat® PQ 11), Copolymere aus N-Vinylcaprolactam/N-Vinylpyrrolidon/N-Vinylimidazoliumsalzen (Luviquat® Hold); kationische Cellulosederivate (Polyquaternium-4 und -10), Acrylamidcopolymere (Polyquaternium-7). Ferner können Eiweißhydrolysate verwendet werden, sowie konditionierende Substanzen auf Basis von Silikonverbindungen, beispielsweise Polyalkylsiloxane, Polyarylsiloxane, Polyarylalkylsiloxane, Polyethersiloxane oder Silikonharze. Weitere geeignete Silikonverbindungen sind Dimethicon Copolyole (CTFA) und aminofunktionelle Silikonverbindungen wie Amodimethicone (CTFA). Ferner können kationische Guarderivate wie Guarhydroxypropyltrimoniumchlorid (INCI) verwendet werden.

Die Erfindung wird anhand der folgenden nicht einschränkenden Beispiele näher erläutert.

Beispiele

Beispiel 1:

In einer Rührapparatur mit Ankerrührer (200 Upm), Stickstoffeinleitung und separater Zulaufvorrichtung wurden 312 g Natriumsalz (40 %ige wässrige Lösung) eines Maleinsäure-Acrylsäure-Copolymerisats (Sokalan® CP 5 der BASF AG, 7 Teile Acrylsäure und 3 Teile Maleinsäure, Mn = 70000), 160 g Vinylpyrrolidon, 88,9 g N-Vinyl-2-methylimidazoliummethylsulfat (45 %ige wässrige Lösung) und 0,3 g Triallylamin in 146,2 g Wasser vorgelegt und der pH Wert der Lösung durch Zusatz von 7,5 g Schwefelsäure (50 %ige wässrige Lösung) auf 6,8 eingestellt. Man leitete permanent Stickstoff durch die Reaktionsmischung und erhitzte das Reaktionsgemisch zur Polymerisation auf eine Temperatur von 65 °C. Innerhalb von drei Stunden ließ man 100 g einer 1,5 gew.-%igen Lösung von 2,2'-Azobis-2-(aminopropan) dihydrochlorid (WAKO® V 50) zulaufen. Nach Beendigung der Zulaufzeit ließ man noch vier Stunden weiter polymerisieren. Danach wurde die Polymerisationstemperatur auf 70 °C erhöht, und es wurden weitere 100 g einer 1,5 gew.-%igen Lösung von 2,2'-Azobis-2-(aminopropan)dihydrochlorid (WAKO® V 50) innerhalb von einer Stunde zugegeben. Danach wurde noch einmal zwei Stunden bei 70 °C polymerisiert. Die erhaltene Dispersion hat einen Wirkstoffgehalt von 20 % und einen Feststoffgehalt von 32,5 %. Der LD-Wert der Dispersion betrug < 0,05 und die Viskosität 8000 mPas.

Beispiel 2:

In einer Rührapparatur mit Ankerrührer (200 Upm), Stickstoffeinleitung und separater Zulaufvorrichtung wurden 312 g Natriumsalz (40 %ige wässrige Lösung) eines Maleinsäure-Acrylsäure-Copolymerisats (Sokalan® CP 5 der BASF AG, 7 Teile Acrylsäure und 3 Teile Maleinsäure, Mn = 70000), 42 g Vinylpyrrolidon, 31,10 g N-Vinyl-2-methylimidazoliummethylsulfat (45 %ige wässrige Lösung) und 0,7 g Triallylamin in 146,2 g Wasser vorgelegt und der pH-Wert der Lösung durch Zusatz von 7 g Schwefelsäure (50 %ige wässrige Lösung) auf 6,8 eingestellt. Man leitete permanent Stickstoff durch die Reaktionsmischung und erhitzte das Reaktionsgemisch zur Polymerisation auf eine Temperatur von 65 °C. Innerhalb von zwei Stunden ließ man 168 g Vinylpyrrolidon, 124,4 g Vinylimidazoliumdimethylsulfat und 116,8 g Wasser und innerhalb von drei Stunden 100 g einer 1,5 gew.-%igen Lösung von 2,2'-Azobis-2-(aminopropan)dihydrochlorid (WAKO® V 50) zulaufen. Die Polymerisationszeit nach Zulaufende betrug vier Stunden. Danach wurde die Polymerisationstemperatur auf 70 °C erhöht und es wurden weitere 100 g einer 1,5 gew.-%igen Lösung von 2,2'-Azobis-2-(aminopropan)dihydrochlorid (WAKO® V 50) innerhalb von einer Stunde zugegeben. Danach wurde noch zwei Stunden bei 70 °C polymerisiert. Die Dispersion hat einen Wirkstoffgehalt von 20 % und einen Feststoffgehalt von 32,5 %. Der LD-Wert der Dispersion betrug < 0,05 und die Viskosität 8500 mPas.

Beispiel 3:

In einer Rührapparatur mit Ankerrührer (200 Upm), Stickstoffeinleitung und separater Zulaufvorrichtung wurden 312 g Natriumsalz (40 %ige wässrige Lösung) eines Maleinsäure-Acrylsäure-Copolymerisats (Sokalan® CP 7 der BASF AG), 33 g Vinylpyrrolidon, 24,90 g N-Vinyl-2-methylimidazoliummethylsulfat (45 %ige wässrige Lösung) und 0,7 g Triallylamin in 146,2 g Wasser vorgelegt und der pH-Wert der Lösung durch Zusatz von 7 g Schwefelsäure (50 %ige wässrige Lösung) auf 6,8 eingestellt. Man leitete permanent Stickstoff durch die Reaktionsmischung und erhitzte das Reaktionsgemisch zur Polymerisation auf eine Temperatur von 65 °C. Innerhalb von 2,5 Stunden ließ man 134 g Vinylpyrrolidon, 44,8 g Methylacrylat, 99,1 g Vinylimidazoliumdimethylsulfat und 130,9 g Wasser und innerhalb von drei Stunden 100 g einer 1,5 gew.-%igen Lösung von 2,2'-Azobis-2-(aminopropan)dihydrochlorid (WAKO® V 50) zulaufen. Die Polymerisationszeit nach Zulaufende betrug vier Stunden. Danach wurde die Polymerisationstemperatur auf 70 °C erhöht und es wurde weiteren 100 g einer 1,5 gew.-%igen Lösung von 2,2'-Azobis-2-(aminopropan)dihydrochlorid (WAKO® V 50) innerhalb von einer Stunde zugegeben. Danach wurde noch zwei Stunden bei 80 °C polymerisiert. Die Dispersion hat einen Wirkstoffgehalt von 20 % und einen Feststoffgehalt von 32,5 %. Der LD-Wert der Dispersion betrug < 0,05 und die Viskosität 12100 mPas.

Man erhielt eine wässrige Dispersion mit einem Feststoffgehalt von 20 %, einer Viskosität von 8000 mPas und einem LD-Wert (gemessen bei 20 % Wirkstoffgehalt) von < 1 % (0,01).

Die Polymerdispersionen der Vergleichsbeispiele 1 und 2 wurden analog zu Beispiel 1 erhalten. Ihre Zusammensetzung ist der Tabelle 1 zu entnehmen.

Die Brookfield-Viskositätsmessung wurde bei 25 °C, mit Spindel 4 und 12 Umdrehungen gemessen.

Die Bestimmung der Kämmkraftabnahme wurde wie folgt durchgeführt:

Bestimmung Blindwert Nasskämmbarkeit: Die gewaschenen Haare wurden über Nacht im Klimaraum getrocknet. Vor der Messung wurden sie zweimal mit Texapon NSO insgesamt eine Minute shampooniert und eine Minute ausgespült, damit sie definiert nass, d. h. gequollen, sind. Vor Beginn der Messung wurde die Tresse so vorgekämmt, bis keine Verhakungen der Haare mehr vorhanden sind und somit bei wiederholtem Messkämmen eine konstante

Kraftaufwendung erforderlich ist. Anschließend wurde die Tresse an der Halterung fixiert und mit der feinzinkigen Seite des Kammes in die feinzinkige Seite des Prüfkammes eingekämmt. Das Einlegen der Haare in den Prüfkamm erfolgte bei jeder Messung gleichmäßig und spannungsfrei. Die Messung wurde gestartet und mittels Software (EGRANUDO-Programm, Fa. Frank) ausgewertet. Die Einzelmessung wurde 5 bis 10 Mal wiederholt. Der errechnete Mittelwert wurde notiert.

Bestimmung Messwert Nasskämmbarkeit: Nach der Bestimmung des Blindwertes wurden die Haare mit einem Shampoo, enthaltend die erfindungsgemäße Dispersion bzw. solchen enthaltend die Vergleichsdispersion gemäß Tabelle 1 behandelt. Die Messung der Kämmkraft erfolgt analog der Blindwertbestimmung.

Auswertung:

$$\text{Kämmkraftabnahme nass [\%]} = 100 - (\text{Messwert} * 100/\ \text{Blindwert})$$

Die vergebene Note wurde nach folgender Skala ermittelt: 1 sehr gut, 2 befriedigend, 3 unbefriedigend

Tabelle 1

| Beispiel Nr. | VP 1) [Gew.-%] | QVI 2) [Gew.-%] | TAA 3) [Gew.-%] | CP5 4) [Gew.-%] | Reduktion der Kämmkraft | |
|---|---|---|---|---|---|---|
| | | | | | % | Note |
| 1 | 80 | 20 | 0,15 | 12,5 | 60 | 1- |
| V1 | 80 | 20 | 0,15 | 0 | 26 | 2- |
| V2 | 80 | 20 | 0 | 12,5 | 23 | 2-3 |

1) Vinylpyrrolidon
2) N-Vinyl-2-methyl-imidazoliummethylsulfat
3) Triallylamin
4) Copolymer aus 7 Teilen Acrylsäure und 3 Teilen Maleinsäure, Natriumsalz (Sokalan® CP 5
5) bezogen auf VP und QVI

Die erfindungsgemäße Dispersion (Beispiel 1) zeigt exzellente haarkosmetische Eigenschaften. Sie ist mit vergleichsweise hohem Feststoffgehalt bei erwünschter Viskosität herstellbar. Die entsprechenden Dispersionen, hergestellt ohne Vernetzer (Vergleichsbeispiel V2) zeigen unbefriedigende haarkosmetische Eigenschaften. Die Herstellung in Gegenwart eines Vernetzer ist zwingend notwendig zum Erreichen der anwendungstechnischen Eigenschaften. Polymerisate, die ohne polymeres Dispergiermittel hergestellt werden (Vergleichsbeispiel V1), weisen unbefriedigende haarkosmetischen Eigenschaften im Vergleich zu denen der erfindungsgemäßen Dispersion auf. Für die Herstellung von anwendungstechnisch als exzellent eingestuften Polymerisaten ist daher die Gegenwart eines geeigneten polymeren Dispergiermittels erforderlich.

Beispiele für kosmetische Zubereitungen (alle Angaben in Gew.-%)

In allen Formulierungen wurden die in Beispiel 1 erhaltene Dispersion eingesetzt.

Beispiel 1: Flüssiges Makeup

| | |
|---|---|
| A | |
| 1,70 | Glycerylstearat |
| 1,70 | Cetylalkohol |
| 1,70 | Ceteareth-6 |
| 1,70 | Ceteareth-25 |
| 5,20 | Capryl/Caprin-Triglycerid |
| 5,20 | Mineralöl |

(fortgesetzt)

| | |
|---|---|
| B | |
| q.s. | Konservierungsmittel |
| 4,30 | Propylenglycol |
| 2,50 | erfindungsgemäße Dispersion |
| 59,50 | dest. Wasser |
| C | |
| q.s. | Parfumöl |
| D | |
| 2,00 | Eisenoxid |
| 12,00 | Titandioxid |

Herstellung:

Phase A und Phase B getrennt voneinander auf 80 °C erwärmen. Dann Phase B in Phase A mit einem Rührer mischen. Alles auf 40 °C abkühlen lassen und Phase C und Phase D zugeben. Wiederholt homogenisieren.

Bespiel 2: Ölfreies Makeup

| | |
|---|---|
| A | |
| 0,35 | Veegum |
| 5,00 | Butylenglycol |
| 0,15 | Xanthangummi |
| B | |
| 53,00 | dest. Wasser |
| q.s. | Konservierungsmittel |
| 0,20 | Polysorbat-20 |
| 1,60 | Tetrahydroxypropylethylendiamin |
| C | |
| 1,00 | Siliciumdioxid |
| 2,00 | Nylon-12 |
| 4,15 | Mica |
| 6,00 | Titandioxid |
| 1,85 | Eisenoxid |
| D | |
| 4,00 | Stearinsäure |
| 1,50 | Glycerylstearat |
| 7,00 | Benzyllaurat |
| 5,00 | Isoeicosan |
| q.s. | Konservierungsmittel |

(fortgesetzt)

| | |
|---|---|
| E | |
| 1,00 | dest. Wasser |
| 0,50 | Panthenol |
| 0,10 | Imidazolidinyl-Harnstoff |
| 5,00 | erfindungsgemäße Dispersion |

Herstellung:

Phase A mit Butylenglycol benetzen, in Phase B hineingeben und gut mischen. Phase AB auf 75°C erwärmen. Phase C Einsatzstoffe pulverisieren, in Phase AB hineingeben und gut homogenisieren. Einsatzstoffe von Phase D mischen, auf 80 °C erwärmen und zu Phase ABC geben. Einige Zeit mischen, bis alles homogen ist. Alles in ein Gefäß mit Propellermischer übertragen. Einsatzstoffe von Phase E mischen, in Phase ABCD hineingeben und gut vermischen.

Beispiel 3: Eyeliner

| | |
|---|---|
| A | |
| 40,60 | dest. Wasser |
| 0,20 | Dinatrium-EDTA |
| q.s. | Konservierungsmittel |
| | |
| B | |
| 0,60 | Xanthangummi |
| 0,40 | Veegum |
| 3,00 | Butyleneglycol |
| 0,20 | Polysorbat-20 |
| | |
| C | |
| 15,00 | Eisenoxid / Al-Pulver / Siliciumdioxid (z. B. Sicopearl Fantastico Gold™ von BASF) |
| | |
| D | |
| 10,00 | Dest. Wasser |
| 30,00 | erfindungsgemäße Dispersion |

Herstellung:

Phase B vormischen. Mit einem Propellermischer Phase B in Phase A hineinmischen, wobei man den Verdicker quellen lässt. Phase C mit Phase D benetzen, alles in Phasen AB zugeben und gut mischen.

Beispiel 4: Schimmerndes Gel

| | |
|---|---|
| A | |
| 32,60 | Dest. Wasser |
| 0,10 | Dinatrium-EDTA |
| 25,00 | Carbomer (2 %ige wässrige Lösung) |
| 0,30 | Konservierungsmittel |

(fortgesetzt)

| | |
|---|---|
| B | |
| 0,50 | Dest. Wasser |
| 0,50 | Triethanolamin |
| C | |
| 10,00 | Dest. Wasser |
| 9,00 | erfindungsgemäße Dispersion |
| 1,00 | Polyquaternium-46 |
| 5,00 | Eisenoxid |
| D | |
| 15,00 | Dest. Wasser |
| 1,00 | D-Panthenol 50 P (Panthenol und Propylenglycol) |

Herstellung:

Mit einem Propellermischer die Einsatzstoffe von Phase A in der angegebenen Reihenfolge gut mischen. Dann Phase B in Phase A hineingeben. Langsam rühren bis alles homogen ist. Phase C gut homogenisieren, bis die Pigmente gut verteilt sind. Phase C und Phase D zu Phase AB geben und gut mischen.

Beispiel 5: Wasserfester Mascara

| | |
|---|---|
| A | |
| 46,70 | Dest. Wasser |
| 3,00 | Lutrol E 400 (PEG-8) |
| 0,50 | Xanthangummi |
| q.s. | Konservierungsmittel |
| 0,10 | Imidazolidinyl-Harnstoff |
| 1,30 | Tetrahydroxypropylethylendiamin |
| B | |
| 8,00 | Carnaubawachs |
| 4,00 | Bienenwachs |
| 4,00 | Isoeicosan |
| 4,00 | Polyisobuten |
| 5,00 | Stearinsäure |
| 1,00 | Glycerylstearat |
| q.s. | Konservierungsmittel |
| 2,00 | Benzyllaurat |
| C | |
| 10,00 | Eisenoxid / Al-Pulver / Siliciumdioxid (z. B. Sicopearl Fantastico Gold™ von BASF) |
| E | |
| 8,00 | Polyurethan-1 |

(fortgesetzt)

| | |
|---|---|
| E | |
| 2,00 | erfindungsgemäße Dispersion |

Herstellung:

Phase A und Phase B getrennt voneinander auf 85 ºC erwärmen. Temperatur halten und Phase C zu Phase A geben und homogenisieren, bis die Pigmente gleichmäßig verteilt sind. Phase B zu Phasen AC geben und für 2 - 3 Minuten homogenisieren. Dann Phase E zugeben und langsam rühren. Alles auf Raumtemperatur abkühlen lassen.

Beispiel 6: Sonnenschutz-Gel

| | |
|---|---|
| Phase A | |
| 1,00 | PEG-40 hydriertes Rizinusöl |
| 8,00 | Octylmethoxycinnamat (Uvinul MC 80™ von BASF) |
| 5,00 | Octocrylen (Uvinul N 539™ von BASF) |
| 0,80 | Octyltriazon (Uvinul T 150™ von BASF) |
| 2,00 | Butylmethoxydibenzoylmethan (Uvinul BMBM™ von BASF) |
| 2,00 | Tocopherylacetat |
| q.s. | Parfümöl |
| Phase B | |
| 2,50 | erfindungsgemäße Dispersion |
| 0,30 | Acrylat/$C_{10-30}$Alkylacrylat-Copolymere |
| 0,20 | Carbomer |
| 5,00 | Glycerin |
| 0,20 | Dinatrium-EDTA |
| q.s. | Konservierungsmittel |
| 72,80 | dest. Wasser |
| Phase C | |
| 0,20 | Natriumhydroxid |

Herstellung:

Die Komponenten der Phase A mischen. Phase B quellen lassen und unter Homogenisieren in Phase A einrühren. Mit Phase C neutralisieren und erneut homogenisieren.

Beispiel 7: Sonnenschutzemulsion mit $TiO_2$ und $ZnO_2$

| | |
|---|---|
| Phase A | |
| 6,00 | PEG-7 hydriertes Rizinusöl |
| 2,00 | PEG-45/Dodecylglycol-Copolymer |
| 3,00 | Isopropylmyristat |
| 8,00 | Jojobaöl (Buxus Chinensis) |
| 4,00 | Octylmethoxycinnamat (Uvinul MC 80) |
| 2,00 | 4-Methylbenzyliden-Kampfer (Uvinul MBC 95) |
| 3,00 | Titandioxid, Dimethicon |
| 1,00 | Dimethicon |

(fortgesetzt)

| | |
|---|---|
| Phase A | |
| 5,00 | Zinkoxid, Dimethicon |
| Phase B | |
| 2,00 | erfindungsgemäße Dispersion |
| 0,20 | Dinatrium-EDTA |
| 5,00 | Glycerin |
| q.s. | Konservierungsmittel |
| 58,80 | dest. Wasser |
| Phase C | |
| q.s. | Parfümöl |

Herstellung:

Die Phasen A und B getrennt auf ca. 85 °C erwärmen. Phase B in Phase A einrühren und homogenisieren. Abkühlen auf ca. 40 °C, Phase C hinzugeben und nochmals kurz homogenisieren.

Beispiel 8: Sonnenschutz-Lotion

| | |
|---|---|
| Phase A | |
| 6,00 | Octylmethoxycinnamat (Uvinul MC 80™ von BASF) |
| 2,50 | 4-Methylbenzyliden-Kampfer (Uvinul MBC 95™ von BASF) |
| 1,00 | Octyltriazon (Uvinul T 150™ von BASF) |
| 2,00 | Butylmethoxydibenzoylmethan (Uvinul BMBM™ von BASF) |
| 2,00 | PVP/Hexadecen-Copolymer |
| 5,00 | PPG-3 Myristylether |
| 0,50 | Dimethicon |
| 0,10 | BHT, Ascorbylpalmitat, Citronensäure, Glycerylstearat, Propylenglycol |
| 2,00 | Cetylalkohol |
| 2,00 | Kaliumcetylphosphat |
| Phase B | |
| 2,50 | erfindungsgemäße Dispersion |
| 5,00 | Propylenglycol |
| 0,20 | Dinatrium-EDTA |
| q.s. | Konservierungsmittel |
| 63,92 | dest. Wasser |
| Phase C | |
| 5,00 | Mineralöl |
| 0,20 | Carbomer |
| Phase D | |
| 0,08 | Natriumhydroxid |

(fortgesetzt)

| | |
|---|---|
| Phase E | |
| q.s. | Parfümöl |

Herstellung:

Die Phasen A und B getrennt auf ca. 80 °C erwärmen. Phase B unter Homogenisieren in Phase A einrühren, kurz nachhomogenisieren. Phase C anschlämmen, in Phase AB einrühren, mit Phase D neutralisieren und nachhomogenisieren. Abkühlen auf ca. 40 °C, Phase E zugeben, nochmals homogenisieren.

Beispiel 9: Abziehbare Gesichtsmaske

| | |
|---|---|
| Phase A | |
| 57,10 | dest. Wasser |
| 6,00 | Polyvinylalkohol |
| 5,00 | Propylenglycol |
| Phase B | |
| 20,00 | Alkohol |
| 4,00 | PEG-32 |
| q.s | Parfümöl |
| Phase C | |
| 5,00 | Polyquaternium-44 |
| 2,70 | erfindungsgemäße Dispersion |
| 0,20 | Allantoin |

Herstellung:

Phase A auf mind. 90 °C erwärmen und rühren bis gelöst. Phase B bei 50 °C lösen und in Phase A einrühren. Bei ca. 35 °C den Ethanolverlust ausgleichen. Phase C zugeben und unterrühren.

Beispiel 10: Gesichtsmaske

| | |
|---|---|
| Phase A | |
| 3,00 | Ceteareth-6 |
| 1,50 | Ceteareth-25 |
| 5,00 | Cetearylalkohol |
| 6,00 | Cetearyloctanoat |
| 6,00 | Mineralöl |
| 0,20 | Bisabolol |
| 3,00 | Glycerylstearat |
| Phase B | |
| 2,00 | Propylenglycol |
| 5,00 | Panthenol |
| 2,80 | erfindungsgemäße Dispersion |
| q.s. | Konservierungsmittel |

(fortgesetzt)

| | |
|---|---|
| Phase B | |
| 65,00 | dest. Wasser |
| Phase C | |
| q.s. | Parfümöl |
| 0,50 | Tocopherylacetat |

Herstellung:

Phase A und B getrennt auf ca. 80 °C erwärmen. Phase B in Phase A unter Homogenisieren einrühren, kurz nachhomogenisieren. Abkühlen auf ca. 40 °C, Phase C hinzugeben, nochmals homogenisieren.

Beispiel 11: Körperlotion-Schaum

| | |
|---|---|
| Phase A | |
| 1,50 | Ceteareth-25 |
| 1,50 | Ceteareth-6 |
| 4,00 | Cetearylalkohol |
| 10,00 | Cetearyloctanoat |
| 1,00 | Dimethicon |
| Phase B | |
| 3,00 | erfindungsgemäße Dispersion |
| 2,00 | Panthenol |
| 2,50 | Propylenglycol |
| q.s. | Konservierungsmittel |
| 74,50 | dest. Wasser |
| Phase C | |
| q.s. | Parfümöl |

Herstellung:

Die Phasen A und B getrennt auf ca. 80 °C erwärmen. Phase B in Phase A einrühren und homogenisieren. Abkühlen auf ca. 40 °C, Phase C hinzugeben und nochmals kurz homogenisieren. Abfüllung: 90 % Wirkstoff und 10 % Propan/Butan bei 3,5 bar (20 °C).

Beispiel 12: Gesichtswasser für trockene und empfindliche Haut

| | |
|---|---|
| Phase A | |
| 2,50 | PEG-40 hydriertes Rizinusöl |
| q.s. | Parfümöl |
| 0,40 | Bisabolol |
| Phase B | |
| 3,00 | Glycerin |
| 1,00 | Hydroxyethylcetyldimoniumphosphat |

(fortgesetzt)

| | |
|---|---|
| Phase B | |
| 5,00 | Zaubernuss-Destillat (Hamamelis Virginiana) |
| 0,50 | Panthenol |
| 0,50 | erfindungsgemäße Dispersion |
| q.s. | Konservierungsmittel |
| 87,60 | dest. Wasser |

Herstellung:

Phase A klar lösen. Phase B in Phase A einrühren.

Beispiel 13: Gesichtswaschpaste mit Peelingeffekt

| | |
|---|---|
| Phase A | |
| 70,00 | dest. Wasser |
| 3,00 | erfindungsgemäße Dispersion |
| 1,50 | Carbomer |
| q.s. | Konservierungsmittel |
| Phase B | |
| q.s. | Parfümöl |
| 7,00 | Hydriertes Kaliumcocoyl-Protein |
| 4,00 | Cocamidpropylbetain |
| Phase C | |
| 1,50 | Triethanolamin |
| Phase D | |
| 13,00 | Polyethylen (Luwax A™ von BASF) |

Herstellung:

Phase A quellen lassen. Phase B klar lösen. Phase B in Phase A einrühren. Mit Phase C neutralisieren. Danach Phase D einrühren.

Gesichtsseife

| | |
|---|---|
| Phase A | |
| Kaliumcocoat | |
| Dinatriumcocoamphodiacetat | |
| 2,00 | Lauramid-DEA |
| Glycol Stearate | |
| 2,00 | erfindungsgemäße Dispersion |
| 50,00 | dest. Wasser |
| q.s. | Citronensäure |
| Phase B | |
| q.s. | Konservierungsmittel |

(fortgesetzt)

| | |
|---|---|
| Phase B | |
| q.s. | Parfumöl |

Herstellung:

Phase A unter Rühren auf 70 °C erwärmen, bis alles homogen ist. pH-Wert auf 7,0 - 7,5 mit Citronensäure. Alles auf 50 °C abkühlen lassen und Phase B zugeben.

Beispiel 14: Gesichtsreinigungsmilch Typ O/W

| | |
|---|---|
| Phase A | |
| 1,50 | Ceteareth-6 |
| 1,50 | Ceteareth-25 |
| 2,00 | Glycerylstearat |
| 2,00 | Cetylalkohol |
| 10,00 | Mineralöl |
| Phase B | |
| 5,00 | Propylenglycol |
| q.s. | Konservierungsmittel |
| 1,00 | erfindungsgemäße Dispersion |
| 66,30 | dest. Wasser |
| Phase C | |
| 0,20 | Carbomer |
| 10,00 | Cetearyloctanoat |
| Phase D | |
| 0,40 | Tetrahydroxypropylethylendiamin |
| Phase E | |
| q.s. | Parfümöl |
| 0,10 | Bisabolol |

Herstellung:

Die Phasen A und B getrennt auf ca. 80 °C erwärmen. Phase B unter Homogenisieren in Phase A einrühren, kurz nachhomogenisieren. Phase C anschlämmen, in Phase AB einrühren, mit Phase D neutralisieren und nachhomogenisieren. Abkühlen auf ca. 40 °C, Phase E zugeben, nochmals homogenisieren.

Beispiel 15: Transparente Seife

| | |
|---|---|
| 4,20 | Natriumhydroxid |
| 3,60 | dest. Wasser |
| 2,00 | erfindungsgemäße Dispersion |
| 22,60 | Propylenglycol |
| 18,70 | Glycerin |
| 5,20 | Cocoamid-DEA |

(fortgesetzt)

| | |
|---|---|
| 10,40 | Cocaminoxid |
| 4,20 | Natriumlaurylsulfat |
| 7,30 | Myristinsäure |
| 16,60 | Stearinsäure |
| 5,20 | Tocopherol |

Herstellung:

Alle Zutaten mischen. Die Mischung klar schmelzen bei 85 °C. Sofort in die Form ausgießen.

Beispiel 16: Peeling-Creme, Typ O/W

| | |
|---|---|
| Phase A | |
| 3,00 | Ceteareth-6 |
| 1,50 | Ceteareth-25 |
| 3,00 | Glycerylstearat |
| 5,00 | Cetearylalkohol, Natriumcetearylsulfat |
| 6,00 | Cetearyloctanoat |
| 6,00 | Mineralöl |
| 0,20 | Bisabolol |
| Phase B | |
| 2,00 | Propylenglycol |
| 0,10 | Dinatrium-EDTA |
| 3,00 | erfindungsgemäße Dispersion |
| q.s. | Konservierungsmittel |
| 59,70 | dest. Wasser |
| Phase C | |
| 0,50 | Tocopherylacetat |
| q.s. | Parfümöl |
| Phase D | |
| 10,00 | Polyethylen |

Herstellung:

Die Phasen A und B getrennt auf ca. 80 °C erwärmen. Phase B in Phase A einrühren und homogenisieren. Abkühlen auf ca. 40 °C, Phase C hinzugeben und nochmals kurz homogenisieren. Anschließend Phase D unterrühren.

Beispiel 17: Rasierschaum

| | |
|---|---|
| 6,00 | Ceteareth-25 |
| 5,00 | Poloxamer 407 |
| 52,00 | dest. Wasser |
| 1,00 | Triethanolamin |
| 5,00 | Propylenglycol |
| 1,00 | PEG-75 Lanolinöl |

(fortgesetzt)

| | |
|---|---|
| 5,00 | erfindungsgemäße Dispersion |
| q.s. | Konservierungsmittel |
| q.s. | Parfümöl |
| 25,00 | Natriumlaurethsulfat |

Herstellung:

Alles zusammen wiegen, danach rühren bis gelöst. Abfüllung: 90 Teile Wirksubstanz und 10 Teile Propan/Butan-Mischung 25:75.

Beispiel 18: After Shave-Balsam

| | |
|---|---|
| Phase A | |
| 0,25 | Acrylat/$C_{10-30}$ Alkylacrylat-Copolymere |
| 1,50 | Tocopherylacetat |
| 0,20 | Bisabolol |
| 10,00 | Capryl/Caprin-Triglycerid |
| q.s. | Parfümöl |
| 1,00 | PEG-40 hydriertes Rizinusöl |
| Phase B | |
| 1,00 | Panthenol |
| 15,00 | Alkohol |
| 5,00 | Glycerin |
| 0,05 | Hydroxyethylcellulose |
| 1,92 | erfindungsgemäße Dispersion |
| 64,00 | dest. Wasser |
| Phase C | |
| 0,08 | Natriumhydroxid |

Herstellung:

Die Komponenten der Phase A mischen. Phase B unter Homogenisieren in Phase A einrühren, kurz nachhomogenisieren. Mit Phase C neutralisieren und erneut homogenisieren.

Beispiel 19: Körperpflegecreme

| | |
|---|---|
| Phase A | |
| 2,00 | Ceteareth-6 |
| 2,00 | Ceteareth-25 |
| 2,00 | Cetearylalkohol |
| 3,00 | Glycerylstearat SE |
| 5,00 | Mineralöl |
| 4,00 | Jojobaöl (Buxus Chinensis) |
| 3,00 | Cetearyloctanoat |
| 1,00 | Dimethicon |
| 3,00 | Mineralöl, Lanolinalkohol |

(fortgesetzt)

| | |
|---|---|
| Phase A | |
| Phase B | |
| 5,00 | Propylenglycol |
| 0,50 | Veegum |
| 1,00 | Panthenol |
| 1,70 | erfindungsgemäße Dispersion |
| 6,00 | Polyquaternium-44 |
| q.s. | Konservierungsmittel |
| 60,80 | dest. Wasser |
| Phase C | |
| q.s. | Parfümöl |

Herstellung:

Die Phasen A und B getrennt auf ca. 80 °C erwärmen. Phase B homogenisieren. Phase B unter Homogenisieren in Phase A einrühren, kurz nachhomogenisieren. Abkühlen auf ca. 40 °C, Phase C hinzugeben und nochmals kurz homogenisieren.

Beispiel 20: Zahnpasta

| | |
|---|---|
| Phase A | |
| 34,79 | dest. Wasser |
| 3,00 | erfindungsgemäße Dispersion |
| 0,30 | Konservierungsmittel |
| 20,00 | Glycerin |
| 0,76 | Natriummonofluorphosphat |
| Phase B | |
| 1,20 | Natriumcarboxymethylcellulose |
| Phase C | |
| 0,80 | Aromaöl |
| 0,06 | Saccharin |
| 0,10 | Konservierungsmittel |
| 0,05 | Bisabolol |
| 1,00 | Panthenol |
| 0,50 | Tocopherylacetat |
| 2,80 | Siliciumdioxid |
| 1,00 | Natriumlaurylsulfat |
| 7,90 | Dicalciumphosphat wasserfrei |
| 25,29 | Dicalciumphosphat-Dihydrat |
| 0,45 | Titandioxid |

Herstellung:

Phase A lösen. Phase B in Phase A einstreuen und lösen. Phase C zugeben und unter Vakuum bei Raumtemperatur ca. 45 Min. rühren lassen.

Beispiel 21: Mundwasser

| | |
|---|---|
| Phase A | |
| 2,00 | Aromaöl |
| 4,00 | PEG-40 hydriertes Rizinusöl |
| 1,00 | Bisabolol |
| 30,00 | Alkohol |
| Phase B | |
| 0,20 | Saccharin |
| 5,00 | Glycerin |
| q.s. | Konservierungsmittel |
| 5,00 | Poloxamer 407 |
| 0,5 | erfindungsgemäße Dispersion |
| 52,30 | dest. Wasser |

Herstellung:

Phase A und Phase B getrennt klar lösen. Phase B in Phase A einrühren.

Beispiel 22: Prothesenhaftmittel

| | |
|---|---|
| Phase A | |
| 0,20 | Bisabolol |
| 1,00 | Beta-Carotin |
| q.s. | Aromaöl |
| 20,00 | Cetearyloctanoat |
| 5,00 | Siliciumdioxid |
| 33,80 | Mineralöl |
| Phase B | |
| 5,00 | erfindungsgemäße Dispersion |
| 35,00 | PVP (20 %ige Lösung in Wasser) |

Herstellung:

Phase A gut mischen. Phase B in Phase A einrühren.

Beispiel 23: Hautpflegecreme, Typ O/W

| | |
|---|---|
| Phase A | |
| 8,00 | Cetearylalkohol |
| 2,00 | Ceteareth-6 |
| 2,00 | Ceteareth-25 |
| 10,00 | Mineralöl |
| 5,00 | Cetearyloctanoat |
| 5,00 | Dimethicon |

(fortgesetzt)

| | |
|---|---|
| Phase B | |
| 3,00 | erfindungsgemäße Dispersion |
| 2,00 | Panthenol, Propylenglycol |
| q.s. | Konservierungsmittel |
| 63,00 | dest. Wasser |
| Phase C | |
| q.s. | Parfümöl |

Herstellung:

Phase A und B getrennt auf ca. 80 °C erwärmen. Phase B in Phase A unter Homogenisieren einrühren, kurz nachhomogenisieren. Abkühlen auf ca. 40 °C, Phase C hinzugeben, nochmals homogenisieren.

Beispiel 24: Hautpflegecreme, Typ W/O

| | |
|---|---|
| Phase A | |
| 6,00 | PEG-7 hydriertes Rizinusöl |
| 8,00 | Cetearyloctanoat |
| 5,00 | Isopropylmyristat |
| 15,00 | Mineralöl |
| 2,00 | PEG-45/Dodecylglycol-Copolymer |
| 0,50 | Magnesiumstearat |
| 0,50 | Aluminumstearat |
| Phase B | |
| 3,00 | Glycerin |
| 3,30 | erfindungsgemäße Dispersion |
| 0,70 | Magnesiumsulfat |
| 2,00 | Panthenol |
| q.s. | Konservierungsmittel |
| 48,00 | dest. Wasser |
| Phase C | |
| 1,00 | Tocopherol |
| 5,00 | Tocopherylacetat |
| q.s. | Parfümöl |

Herstellung:

Die Phasen A und B getrennt auf ca. 80 °C erwärmen. Phase B in Phase A einrühren und homogenisieren. Abkühlen auf ca. 40 °C, Phase C hinzugeben und nochmals kurz homogenisieren.

Beispiel 25: Lippenpflegecreme

| | |
|---|---|
| Phase A | |
| 10,00 | Cetearyloctanoat |
| 5,00 | Polybuten |

(fortgesetzt)

| | |
|---|---|
| Phase A | |
| | |
| Phase B | |
| 0,10 | Carbomer |
| | |
| Phase C | |
| 2,00 | Ceteareth-6 |
| 2,00 | Ceteareth-25 |
| 2,00 | Glycerylstearat |
| 2,00 | Cetylalkohol |
| 1,00 | Dimethicon |
| 1,00 | Benzophenon-3 |
| 0,20 | Bisabolol |
| 6,00 | Mineralöl |
| | |
| Phase D | |
| 8,00 | erfindungsgemäße Dispersion |
| 3,00 | Panthenol |
| 3,00 | Propylenglycol |
| q.s. | Konservierungsmittel |
| 54,00 | dest. Wasser |
| | |
| Phase E | |
| 0,10 | Triethanolamin |
| | |
| Phase F | |
| 0,50 | Tocopherylacetat |
| 0,10 | Tocopherol |
| q.s. | Parfümöl |

Herstellung:

Phase A klar lösen. Phase B dazugeben und homogenisieren. Phase C addieren und schmelzen bei 80 °C. Phase D auf 80 °C erwärmen. Phase D zu Phase ABC geben und homogenisieren. Abkühlen auf ca. 40 °C, Phase E und Phase F zugeben, nochmals homogenisieren.

Beispiel 26: Glänzender Lippenstift

| | |
|---|---|
| Phase A | |
| 5,30 | Candelillawachs (Euphorbia Cerifera) |
| 1,10 | Bienenwachs |
| 1,10 | mikrokristalliner Wachs |
| 2,00 | Cetylpalmitat |
| 3,30 | Mineralöl |
| 2,40 | Rizinusöl, Glycerylricinoleat, Octyldodecanol, Carnaubawachs, Candelillawachs |
| 0,40 | Bisabolol |
| 16,00 | Cetearyloctanoat |
| 2,00 | hydrierte Cocoglyceride |

(fortgesetzt)

| | |
|---|---|
| Phase A | |
| q.s. | Konservierungsmittel |
| 1,00 | erfindungsgemäße Dispersion |
| 60,10 | Rizinusöl (Ricinus Communis) |
| 0,50 | Tocopherylacetat |
| Phase B | |
| 0,80 | C. I. 14 720:1, Acid Red 14 Aluminum Lake |
| Phase C | |
| 4,00 | Mica, Titandioxid |

Herstellung:

Die Komponenten der Phase A einwiegen und aufschmelzen. Phase B homogen einarbeiten. Phase C zugeben und unterrühren. Unter Rühren auf Raumtemperatur abkühlen.

Beispiel 26: Duschgel

| | |
|---|---|
| 50,00 | Natriumlaurethsulfat, Magnesiumlaurethsulfat, Natriumlaureth-8-Sulfat, Magnesiumlaureth-8 |
| 1,00 | Cocoamid-DEA |
| 4,00 | erfindungsgemäße Dispersion |
| 2,00 | Natriumlaurethsulfat, Glycoldistearat, Cocamid-MEA, Laureth-10 |
| q.s. | Konservierungsmittel |
| q.s. | Parfümöl |
| 2,00 | Natriumchlorid |
| 41,00 | Aqua dem. |

Herstellung:

Alles zusammen wiegen, rühren bis gelöst.

Beispiel 27: Duschgel

| | |
|---|---|
| 30,00 | Natriumlaurethsulfat |
| 6,00 | Natriumcocoamphodiacetat |
| 6,00 | Cocamidpropylbetain |
| 3,00 | Natriumlaurethsulfat, Glycoldistearat, Cocamid-MEA, Laureth-10 |
| 7,70 | Polyquaternium-44 |
| 1,50 | erfindungsgemäße Dispersion |
| 1,00 | Panthenol |
| q.s. | Konservierungsmittel |
| q.s. | Parfümöl |
| q.s. | Citronensäure |
| 0,50 | Natriumchlorid |
| 44,30 | Aqua dem. |

Herstellung:

Die Komponenten der Phase A einwiegen und lösen. Den pH-Wert auf 6 - 7 einstellen.

Beispiel 28: Klares Duschgel

| | |
|---|---|
| 40,00 | Natriumlaurethsulfat |
| 5,00 | Decylglucosid |
| 5,00 | Cocamidpropylbetain |
| 0,50 | Polyquaternium-10 |
| 2,20 | erfindungsgemäße Dispersion |
| 1,00 | Panthenol |
| q.s. | Parfümöl |
| q.s. | Konservierungsmittel |
| q.s. | Citronensäure |
| 2,00 | Natriumchlorid |
| 44,30 | Aqua dem. |

Herstellung:

Die Komponenten der Phase A einwiegen und klar lösen.

Beispiel 29: Duschbad

| | |
|---|---|
| A | |
| 40,00 | Natriumlaurethsulfat |
| 5,00 | Natrium-$C_{12-15}$-Pareth-15-Sulfonat |
| 5,00 | Decylglucosid |
| q.s. | Parfümöl |
| 0,10 | Phytantriol |
| B | |
| 43,60 | Aqua dem. |
| 0,1 | Guarhydroxypropyltrimoniumchlorid |
| 2,20 | erfindungsgemäße Dispersion |
| 1,00 | Panthenol |
| q.s. | Konservierungsmittel |
| 1,00 | Laureth-3 |
| q.s. | Citronensäure |
| 2,00 | Natriumchlorid |

Herstellung:

Die Komponenten der Phase A mischen. Die Komponenten der Phase B nacheinander zugeben und mischen. Den pH-Wert auf 6 - 7 einstellen.

Beispiel 30: Flüssigseife

| | |
|---|---|
| A | |
| 44,06 | Aqua dem. |
| 0,34 | Aminomethylpropanol |
| 3,40 | Acrylat-Copolymer |
| B | |
| 40,00 | Natriumlaurethsulfat |
| 10,00 | Cocamidpropylbetain |
| 0,20 | erfindungsgemäße Dispersion |
| q.s. | Parfümöl |
| q.s. | Konservierungsmittel |
| 2,00 | Natriumchlorid |

Herstellung:

Die Komponenten der Phase A einwiegen und klar lösen. Die Komponenten der Phase B nacheinander zugeben und mischen.

Beispiel 31: Flüssiges Fußbad

| | |
|---|---|
| A | |
| 1,00 | Nonoxynol-14 |
| 0,10 | Bisabolol |
| 1,00 | Pinienöl (Pinus Sylvestris) |
| B | |
| 5,00 | PEG-8 |
| 1,20 | erfindungsgemäße Dispersion |
| 0,50 | Triclosan |
| 30,00 | Natriumlaurethsulfat |
| 3,00 | Polyquaternium-16 |
| 58,20 | Aqua dem. |
| q.s. | C.I. 19 140 + C.I. 42 051 |

Herstellung:

Phase A solubilisieren. Phase B mischen.

Beispiel 32: Erfrischungsgel

| | |
|---|---|
| A | |
| 0,60 | Carbomer |
| 45,40 | Aqua dem. |
| B | |
| 0,50 | Bisabolol |
| 0,50 | Farnesol |

(fortgesetzt)

| | |
|---|---|
| B | |
| q.s. | Parfümöl |
| 5,00 | PEG-40 hydriertes Rizinusöl |
| 0,50 | erfindungsgemäße Dispersion |
| 1,00 | Tetrahydroxypropylethylendiamin |
| 1,50 | Menthol |
| 45,00 | Alkohol |
| q.s. | C. I. 74 180, Direct Blue 86 |

Herstellung:

Phase A quellen lassen. Phase B lösen. Phase B in Phase A einrühren.

Beispiel 33: Roll-on Antiperspirant

| | |
|---|---|
| A | |
| 0,40 | Hydroxyethylcellulose |
| 50,00 | Aqua dem. |
| B | |
| 25,00 | Alkohol |
| 0,10 | Bisabolol |
| 0,30 | Farnesol |
| 2,00 | PEG-40 hydriertes Rizinusöl |
| q.s. | Parfümöl |
| C | |
| 5,00 | Aluminumchlorhydrat |
| 3,00 | Propylenglycol |
| 3,00 | Dimethiconcopolyol |
| 3,00 | Polyquaternium-16 |
| 1,20 | erfindungsgemäße Dispersion |
| 7,00 | Aqua dem. |

Herstellung:

Phase A quellen lassen. Phase B und C getrennt lösen. Phase A und B in Phase C einrühren.

Beispiel 34: Transparenter Deostift

| | |
|---|---|
| 5,00 | Natriumstearat |
| 0,50 | Triclosan |
| 3,00 | Ceteareth-25 |
| 20,00 | Glycerin |
| 0,50 | erfindungsgemäße Dispersion |
| q.s. | Parfümöl |
| 60,00 | Propylenglycol |
| 0,20 | Bisabolol |

(fortgesetzt)

| | |
|---|---|
| 10,80 | Aqua dem. |

Herstellung:

Phase A zusammen wiegen, schmelzen und homogenisieren. Anschließend in die Form gießen.

Beispiel 35: Wasserlösliches Badeöl

| | |
|---|---|
| 15,00 | Cetearyloctanoat |
| 15,00 | Capryl/Caprin-Triglycerid |
| 1,00 | Panthenol, Propylenglycol |
| 0,10 | Bisabolol |
| 2,00 | Tocopherylacetat |
| 2,00 | Retinylpalmitat |
| 0,10 | Tocopherol |
| 37,00 | PEG-7-Glyceryl-Cocoat |
| 0,40 | erfindungsgemäße Dispersion |
| 3,80 | Aqua dem. |
| q.s. | Parfümöl |
| 23,60 | PEG-40 hydriertes Rizinusöl |

Herstellung:

Mischen und rühren bis alles klar gelöst ist.

Beispiel 36: Tagespflege-Aerosol

| | |
|---|---|
| A | |
| 4,00 | Ethylhexylmethoxycinnamat |
| 1,50 | Octocrylen |
| 9,00 | Capryl/Caprin-Triglycerid |
| 5,00 | Simmondsia Chinensis (Jojoba) Samenöl |
| 1,50 | Cyclomethicon |
| 3,00 | hydrierte Coco-Glyceride |
| 1,00 | PVP/Hexadecen-Copolymer |
| 1,00 | Ceteareth-6, Stearylalkohol |
| B | |
| 5,00 | Zinkoxid |
| C | |
| 2,00 | Ceteareth-25 |
| 1,20 | Panthenol |
| 0,20 | Natriumascorbylphosphat |
| 0,30 | Imidazolidinyl-Harnstoff |

(fortgesetzt)

| | |
|---|---|
| C | |
| 0,10 | Dinatrium-EDTA |
| 1,50 | erfindungsgemäße Dispersion |
| 62,67 | Aqua dem. |
| D | |
| 0,50 | Tocopherylacetat |
| 0,20 | Bisabolol |
| 0,33 | Capryl/Caprin-Triglycerid, Retinol |
| q.s. | Parfümöl |

Herstellung:

Phase A auf 80 °C erwärmen. Phase A klar lösen. Phase B einarbeiten und homogenisieren. Phase C zugeben, erhitzen auf 80 °C, aufschmelzen und homogenisieren. Unter Rühren auf ca. 40 °C abkühlen, Phase D hinzugeben und kurz homogenisieren. 90 % Wirkstofflösung: 10 % Propan/Butan mit 3,5 bar (20 °C) abfüllen.

Beispiel 37: Feuchtigkeitscreme

| | |
|---|---|
| A | |
| 3,00 | Vitis Vinifera (Grape) Samenöl |
| 1,00 | Cyclopentasiloxan, Cyclohexasiloxan |
| 1,50 | Cyclomethicon |
| 2,00 | Sojaöl (Glycinsoja) |
| 2,00 | Ethylhexylmethoxycinnamat |
| 1,00 | Uvinul A Plus (BASF) |
| 1,00 | hydriertes Lecithin |
| 1,00 | Cholesterol |
| 2,00 | PEG-40 hydriertes Rizinusöl |
| 5,00 | Cetearyloctanoat |
| 5,00 | Capryl/Caprin-Triglycerid |
| B | |
| 3,00 | Capryl/Caprin-Triglycerid, Acrylat-Copolymer |
| C | |
| 3,00 | erfindungsgemäße Dispersion |
| 0,50 | Cocotrimoniummethsulfat |
| 2,00 | Panthenol, Propylenglycol |
| 3,00 | Glycerin |
| 0,10 | Dinatrium-EDTA |
| 60,30 | Aqua dem. |
| D | |
| 0,30 | Parfum |
| 0,30 | DMDM Hydantoin |

(fortgesetzt)

| | |
|---|---|
| D | |
| 1,00 | Tocopherylacetat |
| 2,00 | Tocopherol |

Herstellung:

Phase A auf 80 °C erwärmen. Phase B in Phase A einrühren. Phase C auf ca. 80 °C erwärmen und unter Homogenisieren in Phase A+B einrühren. Unter Rühren auf ca. 40 °C abkühlen, Phase D hinzugeben und kurz homogenisieren.

Beispiel 38: Aerosolhaarschaum

| | |
|---|---|
| A | |
| 2,00 | Cocotrimoniummethsulfat |
| 0,20 | Parfümöl |
| B | |
| 63,90 | Aqua dem. |
| 6,70 | erfindungsgemäße Dispersion |
| 0,50 | Acrylat-Copolymer |
| 0,10 | Aminomethyl Propanol |
| 0,20 | Ceteareth-25 |
| 0,20 | Trimethylsilylamodimethicon, Trideceth-10, Cetrimoniumchlorid |
| 0,10 | PEG-25 PABA |
| 0,20 | Hydroxyethylcellulose |
| 0,20 | PEG-8 |
| 0,20 | Panthenol |
| 15,00 | Alkohol |
| C | |
| 10,00 | Propan/Butan 3,5 bar (20 °C) |

Herstellung:

Phasen A und B mischen und mit Treibgas abfüllen.

Beispiel 39: Pumpmousse

| | |
|---|---|
| A | |
| 2,00 | Cocotrimoniummethsulfat |
| q.s. | Parfümöl |
| C | |
| 86,30 | Aqua dem. |
| 7,00 | Polyquaternium-46 |
| 3,00 | erfindungsgemäße Dispersion |
| 0,50 | PEG-8 |

(fortgesetzt)

| | |
|---|---|
| C | |
| 1,00 | Panthenol |
| q.s. | Konservierungsmittel |
| 0,20 | PEG-25 PABA |

Herstellung:

Die Komponenten der Phase A mischen. Die Komponenten der Phase B nacheinander zugeben und klar lösen.

Beispiel 40: Aerosolschaum

| | |
|---|---|
| 15,00 | erfindungsgemäße Dispersion |
| 5,00 | PVP/VA Copolymer |
| 0,50 | Hydroxyethylcetyldimoniumphosphat |
| 0,20 | Ceteareth-25 |
| 0,40 | Parfümöl PC 910.781/Cremophor |
| 68,90 | Aqua dem. |
| q.s. | Konservierungsmittel |
| 10,00 | Propan/Butan 3,5 bar (20 °C) |

Herstellung:

Alles zusammen wiegen, rühren bis gelöst, dann abfüllen.

Beispiel 41: Farbstyling-Mousse

| | |
|---|---|
| A | |
| 2,00 | Cocotrimoniummethsulfat |
| q.s. | Parfümöl |
| B | |
| 6,70 | erfindungsgemäße Dispersion |
| 0,50 | Acrylat-Copolymer |
| 0,10 | Aminomethylpropanol |
| 0,20 | Ceteareth-25 |
| 0,20 | Panthenol |
| 0,20 | Hydroxyethylcellulose |
| 10,00 | Alkohol |
| 69,97 | Aqua dem. |
| 0,08 | C.I. 12245, Basic Red 76 |
| 0,05 | C.I. 42510, Basic Violet 14 |
| C | |
| 10,00 | Propan/Butan 3,5 bar (20 °C) |

Herstellung:

Alles zusammen wiegen, rühren bis gelöst, dann abfüllen.

Nur für dunkelblondes und braunes Haar geeignet!

Beispiel 42: Aerosolhaarschaum

| | |
|---|---|
| A | |
| 0,20 | Parfümöl |
| 2,00 | Cocotrimoniummethsulfat |
| B | |
| 69,90 | Aqua dem. |
| 14,70 | Polyurethan-1 |
| 2,00 | erfindungsgemäße Dispersion |
| 0,50 | PEG-25 PABA |
| 0,20 | Amodimethicon, Tallowtrimoniumchlorid, Nonoxynol-10 |
| q.s. | Konservierungsmittel |
| 0,50 | Ceteareth-25 |
| C | |
| 10,00 | Propan/Butan 3,5 bar (20 °C) |

Herstellung:

Phase A mischen. Die Komponenten der Phase B nacheinander zugeben und lösen. Mit Phase C abfüllen.

Beispiel 43: Pumphaarschaum

| | |
|---|---|
| A | |
| 1,50 | Cocotrimoniummethsulfat |
| q.s. | Parfümöl |
| B | |
| 2,00 | erfindungsgemäße Dispersion |
| 94,04 | Aqua dem. |
| C | |
| 0,46 | Aminomethylpropanol |
| 4,00 | PEG/PPG-25/25 Dimethicon/Acrylat-Copolymer |
| q.s. | Konservierungsmittel |

Herstellung:

Phase A mischen. Phase B in Phase A einrühren. Phase C zugeben und rühren bis gelöst.

Beispiel 44: Haarstylinggel

| | |
|---|---|
| A | |
| 0,50 | Carbomer |
| 87,60 | Aqua dem. |

(fortgesetzt)

| | |
|---|---|
| A | |
| | |
| B | |
| 0,70 | Triethanolamin |
| | |
| C | |
| 6,00 | erfindungsgemäße Dispersion |
| 5,00 | PVP (Luviskol K30 oder Luviskol K90) |
| q.s. | Parfümöl |
| q.s. | PEG-40 hydriertes Rizinusöl |
| q.s. | Konservierungsmittel |
| 0,10 | Tocopherylacetat |

Herstellung:

Phase A quellen lassen und mit Phase B neutralisieren. Phase C lösen und in Phase A+B einrühren.

Beispiel 45: Haarstylinggel

| | |
|---|---|
| A | |
| 0,50 | Carbomer |
| 87,60 | Aqua dem. |
| | |
| B | |
| 0,90 | Tetrahydroxypropylethylendiamin |
| | |
| C | |
| 2,00 | erfindungsgemäße Dispersion |
| 9,00 | VPNA Copolymer (Luviskol VA64W; BASF) |
| q.s. | Parfümöl |
| q.s. | PEG-40 hydriertes Rizinusöl |
| q.s. | Konservierungsmittel |
| 0,10 | Propylenglycol |

Herstellung:

Phase A quellen lassen und mit Phase B neutralisieren. Phase C lösen und in Phase A+B einrühren.

Beispiel 46: Haarstylinggel

| | |
|---|---|
| 2,00 | erfindungsgemäße Dispersion |
| 6,00 | Corn Starch Modified (Amaze, National Starch) |
| 0,50 | Chitosan |
| q.s. | Parfümöl |
| q.s. | PEG-40 hydriertes Rizinusöl |
| 0,10 | PEG-14 Dimethicon |
| 0,10 | Konservierungsmittel |
| 91,40 | Aqua dem. |

Herstellung:

Alle Komponenten mischen bis sie homogen sind.

Beispiel 47: Haarstylinggel

| | |
|---|---|
| 8,00 | erfindungsgemäße Dispersion |
| 5,00 | VP/DMAPA Acrylat-Copolymere (ISP: Styleze CC-10) |
| 0,05 | Aminomethylpropanol |
| 84,85 | Aqua dem. |
| q.s. | Parfümöl |
| q.s. | PEG-40 hydriertes Rizinusöl |
| 0,10 | Dimethiconcopolyol |
| 0,10 | Konservierungsmittel |
| 2,00 | Hydroxypropylcellulose |

Herstellung:

Alle Komponenten mischen bis sie homogen sind.

Beispiel 48: Haarstylinggel

| | |
|---|---|
| 6,00 | erfindungsgemäße Dispersion |
| 1,00 | VP/Acrylat/Laurylmethacrylat-Copolymere (ISP: Styleze 2000) |
| 0,26 | Aminomethylpropanol |
| 90,64 | Aqua dem. |
| q.s. | Parfümöl |
| q.s. | PEG-40 hydriertes Rizinusöl |
| 0,10 | Sorbitol |
| 0,10 | Konservierungsmittel |
| 2,00 | Hydroxypropylguar (Rhodia Inc., N-Hance Hydroxypropylguar) |

Herstellung:

Alle Komponenten mischen bis sie homogen sind.

Beispiel 49: Haargel

| | |
|---|---|
| A | |
| 0,50 | Carbomer |
| 90,01 | Aqua dem. |
| B | |
| 0,70 | Triethanolamin |

(fortgesetzt)

| | |
|---|---|
| C | |
| 6,00 | erfindungsgemäße Dispersion |
| 2,00 | Acrylat/$C_1$-$_2$ Succinat/Hydroxyacrylat-Copolymere (Rohm&Haas, Allianz LT-120) |
| 0,19 | Aminomethylpropanol |
| q.s. | Parfümöl |
| q.s. | PEG-40 hydriertes Rizinusöl |
| 0,10 | PEG-8 |
| 0,10 | Konservierungsmittel |
| 0,50 | Hydroxyethylcellulose |

Herstellung:

Phase A quellen lassen und mit Phase B neutralisieren. Phase C lösen und in Phase A+B einrühren.

Beispiel 50: Haargel

| | |
|---|---|
| 7,00 | erfindungsgemäße Dispersion |
| 7,00 | Methacrylsäure/Natriumacrylamidmethylpropansulfonat-Copolymer (Ondeo Nalco, Fixomer A30) |
| 0,70 | Triethanolamin |
| q.s. | Parfümöl |
| q.s. | PEG-40 hydriertes Rizinusöl |
| 0,10 | Panthenol |
| 0,10 | Konservierungsmittel |
| 84,90 | Aqua dem. |
| 1,00 | Polyacrylamid/$C_{13}$-$_{14}$-Isoparaffin/Laureth-7 (Seppic, Sepigel 305) |

Herstellung:

Alle Komponenten mischen bis sie homogen sind.

Beispiel 51: Haargel

| | |
|---|---|
| A | |
| 0,50 | Carbomer |
| 90,50 | Aqua dem. |
| B | |
| 0,70 | Triethanolamin |
| C | |
| 7,00 | erfindungsgemäße Dispersion |
| 1,00 | Polyvinylformamid |
| q.s. | Parfümöl |
| q.s. | PEG-40 hydriertes Rizinusöl |

(fortgesetzt)

| | |
|---|---|
| C | |
| 0,10 | Konservierungsmittel |
| 0,10 | Ethylhexylmethoxycinnamat |
| 0,10 | PEG-14 Dimethicon |

Herstellung:

Phase A quellen lassen und mit Phase B neutralisieren. Phase C lösen und in Phase A+B einrühren.

Beispiel 52: Aquawachs

| | |
|---|---|
| 10,00 | erfindungsgemäßes Copolymer |
| q.s. | Parfümöl |
| q.s. | PEG-40 hydriertes Rizinusöl |
| 0,10 | Diethylphthalat |
| 0,10 | Cetearylethylhexanoat |
| 0,10 | PEG-7 Glycerylcocoat |
| 0,10 | Konservierungsmittel |
| 87,70 | Aqua dem. |
| 2,00 | Capryl/Caprin-Triglycerid, Acrylat-Copolymere |

Herstellung:

Alles mischen und homogenisieren. 15 Minuten nachrühren.

Beispiel 53: Rinse-off Conditioner and Repair Treatment

| | |
|---|---|
| A | |
| 0,20 | Cetearyloctanoat |
| 0,10 | Phytantriol |
| 2,00 | PEG-40 hydriertes Rizinusöl |
| B | |
| q.s. | Parfümöl |
| 2,00 | Cocotrimoniummethsulfat |
| C | |
| 77,70 | Aqua dem. |
| D | |
| 2,00 | Polyquaternium-16 |
| 5,00 | erfindungsgemäße Dispersion |
| 1,00 | Dimethiconcopolyol |
| q.s. | Konservierungsmittel |

(fortgesetzt)

| | |
|---|---|
| D | |
| 10,00 | Alkohol |
| q.s. | Citronensäure |

Herstellung:

Die Phasen A und B getrennt mischen. Phase C in Phase B einrühren.

Beispiel 54: Haarkur

| | |
|---|---|
| A | |
| 2,00 | Ceteareth-6, Stearylalkohol |
| 1,00 | Ceteareth-25 |
| 6,00 | Cetearylalkohol |
| 6,00 | Cetearyloctanoat |
| 0,30 | Phytantriol |
| B | |
| 5,00 | erfindungsgemäße Dispersion |
| 0,70 | Guarhydroxypropyltrimoniumchlorid |
| 5,00 | Propylenglycol |
| 2,00 | Panthenol |
| 0,30 | Imidazolidinyl-Harnstoff |
| 69,00 | Aqua dem. |
| C | |
| 2,00 | Cosi Silk Soluble |
| 0,20 | Parfüm |
| 0,50 | Phenoxyethanol |

Herstellung:

Die Phasen A und B getrennt auf ca. 80 °C erwärmen. Phase B homogenisieren.

Beispiel 55: Haar-Cocktail

| | |
|---|---|
| A | |
| 0,40 | Acrylat/$C_{10}$-$_{30}$-Alkylacrylat-Copolymere |
| 2,00 | Dimethicon |
| 3,00 | Cyclomethicon, Dimethiconol |
| 2,00 | Phenyltrimethicon |
| 2,00 | Amodimethicon, Cetrimoniumchlorid, Trideceth-10 |
| 0,50 | Dimethiconcopolyol |
| 1,00 | Macadamianussöl (Ternifolia) |
| 0,50 | Tocopherylacetat |
| 1,00 | PEG-40 hydriertes Rizinusöl |
| q.s. | Parfümöl |

(fortgesetzt)

| | |
|---|---|
| B | |
| 82,84 | Aqua dem. |
| 0,30 | erfindungsgemäße Dispersion |
| 0,46 | Aminomethylpropanol |
| 4,00 | PEG/PPG-25/25 Dimethicon/Acrylat-Copolymer |

Herstellung:

Die Komponenten der Phase A mischen. Phase B lösen. Phase B unter Homogenisieren in Phase A einrühren.

Beispiel 56: Dauerwelle

Well-Lösung

| | |
|---|---|
| A | |
| 73,95 | Aqua dem. |
| 0,20 | Cocamidpropylbetain |
| 0,20 | Polysorbat 20 |
| 1,25 | erfindungsgemäße Dispersion |
| 0,20 | Dinatrium-EDTA |
| 0,20 | Hydroxyethylcellulose |
| B | |
| 8,00 | Thioglycolsäure |
| C | |
| 11,00 | Ammoniumhydroxid |
| D | |
| 5,00 | Ammoniumcarbonat |

Herstellung:

Die Komponenten der Phase A einwiegen und klar lösen. Phase B in Phase A einrühren.

Fixierung:

| | |
|---|---|
| A | |
| 1,00 | PEG-40 hydriertes Rizinusöl |
| 0,20 | Parfümöl |
| 93,60 | Aqua dem. |
| B | |
| 0,20 | Cocamidopropylbetain |
| 0,20 | Ceteareth-25 |
| 2,50 | erfindungsgemäße Dispersion |
| q.s. | Konservierungsmittel |

(fortgesetzt)

| | |
|---|---|
| C | |
| 2,30 | Wasserstoffperoxid |
| D | |
| q.s. | Phosphorsäure |

Herstellung:

Phase A solubilisieren. Die Komponenten der Phase B nacheinander zugeben und klar lösen.

Beispiel 57: dunkelbraune Permanenthaarfarbe (Oxidationshaarfarbe)

| | |
|---|---|
| A | |
| 50,90 | Aqua dem. |
| 0,20 | Natriumsulfit |
| 0,05 | Dinatrium-EDTA |
| 0,20 | p-Phenylendiamin |
| 0,30 | Resorcinol |
| 0,20 | 4-Amino-2-hydroxytoluol |
| 0,10 | m-Aminophenol |
| 1,50 | Oleylalkohol |
| 4,50 | Propylenglycol |
| 2,30 | Natrium-$C_{12-15}$-Pareth-15-Sulfonat |
| 20,00 | Ölsäure |
| B | |
| 1,00 | erfindungsgemäße Dispersion |
| 13,70 | Ammoniumhydroxid |
| 6,00 | i-Propanol |
| q.s. | Parfüm |

Herstellung:

Phase A solubilisieren. Die Komponenten der Phase B nacheinander zugeben und mischen.

Entwickleremulsion (pH: 3-4)

| | |
|---|---|
| 3,00 | Hexadecylalkohol |
| 2,00 | erfindungsgemäße Dispersion |
| 1,00 | Ceteareth-20 |
| 1,00 | Natrium-$C_{12-15}$-Pareth-15-Sulfonat |
| 6,00 | Wasserstoffperoxid |
| 0,50 | Phosphorsäure |
| 0,01 | Acetanilid |
| 86,49 | Aqua dem. |

Herstellung:

Die Komponenten nacheinander zugeben und mischen.

Beispiel 58: hellbraune Semi-Permanethaarfarbe

| | |
|---|---|
| 10,00 | Cocodiethanolamid |
| 4,00 | Natriumdodecyl-benzylsulfonat, 50 %ig |
| 1,00 | erfindungsgemäße Dispersion |
| 6,00 | $C_9$-$_{11}$-Pareth-3 |
| 2,50 | Natriumlaurylsulfat |
| 0,40 | 2-Nitro-p-Phenylendiamin |
| 0,20 | HC Red No.3 |
| 0,20 | HC Yellow No.2 |
| 75,70 | Aqua dem. |

Herstellung:

Die Komponenten nacheinander zugeben und mischen.

Beispiel 59: Klares Conditioning Shampoo

| | |
|---|---|
| A | |
| 15,00 | Cocamidpropylbetain |
| 10,00 | Dinatriumcocoamphodiacetat |
| 5,00 | Polysorbat 20 |
| 5,00 | Decylglucosid |
| q.s. | Parfüm |
| q.s. | Konservierungsmittel |
| 0,10-1,00 | erfindungsgemäße Dispersion |
| 2,00 | Laureth-3 |
| add 100 | Aqua dem. |
| q.s. | Citronensäure |
| | |
| B | |
| 3,00 | PEG-150 Distearat |

Herstellung:

Komponenten der Phase A einwiegen und lösen. pH-Wert auf 6 - 7 einstellen. Phase B zugeben und auf 50 °C erwärmen. Auf Raumtemperatur abkühlen lassen unter Rühren.

Beispiel 60: Shampoo

| | |
|---|---|
| 30,00 | Natriumlaurethsulfat |
| 6,00 | Natriumcocoamphoacetat |
| 6,00 | Cocamidpropylbetain |
| 3,00 | Natriumlaurethsulfat, Glycol Distearat, Cocamid-MEA, Laureth-10 |

(fortgesetzt)

| | |
|---|---|
| 0,10-1,00 | erfindungsgemäße Dispersion |
| 2,00 | Dimethicon |
| q.s. | Parfüm |
| q.s. | Konservierungsmittel |
| q.s. | Citronensäure |
| 1,00 | Natriumchlorid |
| add 100 | Aqua dem. |

Herstellung:

Komponenten einwiegen und lösen. pH-Wert auf 6 - 7 einstellen.

Beispiel 61: Shampoo

| | |
|---|---|
| 30,00 | Natriumlaurethsulfat |
| 6,00 | Natriumcocoamphoacetat |
| 6,00 | Cocamidpropylbetain |
| 3,00 | Natriumlaurethsulfat, Glycol Distearat, Cocamid-MEA, Laureth-10 |
| 0,10-1,00 | erfindungsgemäße Dispersion |
| 2,00 | Amodimethicon |
| q.s. | Parfüm |
| q.s. | Konservierungsmittel |
| q.s. | Citronensäure |
| 1,00 | Natriumchlorid |
| add 100 | Aqua dem. |

Herstellung:

Komponenten einwiegen und lösen. pH-Wert auf 6 - 7 einstellen.

Beispiel 62: Shampoo

| | |
|---|---|
| 40,00 | Natriumlaurethsulfat |
| 10,00 | Cocamidpropylbetain |
| 3,00 | Natriumlaurethsulfat, Glycol Distearat, Cocamid-MEA, Laureth-10 |
| 0,10-1,00 | erfindungsgemäße Dispersion |
| 2,00 | Dow Corning 3052 |
| q.s. | Parfüm |
| q.s. | Konservierungsmittel |
| q.s. | Citronensäure |
| 2,00 | Cocamid-DEA |
| add 100 | Aqua dem. |

Herstellung:

Komponenten einwiegen und lösen. pH-Wert auf 6 - 7 einstellen.

Beispiel 63: Antischuppen-Shampoo

| | |
|---|---|
| 40,00 | Natriumlaurethsulfat |
| 10,00 | Cocamidpropylbetain |
| 10,00 | Dinatriumlaurethsulfosuccinat |
| 2,50 | Natriumlaurethsulfat, Glycoldistearat, Cocamid-MEA, Laureth-10 |
| 0,10-1,00 | erfindungsgemäße Dispersion |
| 0,50 | Climbazol |
| q.s. | Parfüm |
| q.s. | Konservierungsmittel |
| 0,50 | Natriumchlorid |
| add 100 | Aqua dem. |

Herstellung:

Komponenten einwiegen und lösen. pH-Wert auf 6 - 7 einstellen.

Beispiel 64: Shampoo

| | |
|---|---|
| 25,00 | Natriumlaurethsulfat |
| 5,00 | Cocamidpropylbetain |
| 2,50 | Natriumlaurethsulfat, Glycoldistearat, Cocamid-MEA, Laureth-10 |
| 0,10-1,00 | erfindungsgemäße Dispersion |
| q.s. | Parfüm |
| q.s. | Konservierungsmittel |
| 2,00 | Cocamid-DEA |
| add 100 | Aqua dem. |

Herstellung:

Komponenten einwiegen und lösen. pH-Wert auf 6 - 7 einstellen.

Beispiel 65: Shampoo

| | |
|---|---|
| 20,00 | Ammoniumlaurethsulfat |
| 15,00 | Ammoniumlaurylsulfat |
| 5,00 | Cocamidpropylbetain |
| 2,50 | Natriumlaurethsulfat, Glycoldistearat, Cocamid-MEA, Laureth-10 |
| 0,10-1,00 | erfindungsgemäße Dispersion |
| q.s. | Parfüm |
| q.s. | Konservierungsmittel |
| 0,50 | Natriumchlorid |
| add 100 | Aqua dem. |

Herstellung:

Komponenten einwiegen und lösen. pH-Wert auf 6 - 7 einstellen.

Beispiel 66: Klares Duschgel

| | |
|---|---|
| 40,00 | Natriumlaurethsulfat |
| 5,00 | Decylglucosid |
| 5,00 | Cocamidpropylbetain |
| 0,10-1,00 | erfindungsgemäße Dispersion |
| 1,00 | Panthenol |
| q.s. | Parfüm |
| q.s. | Konservierungsmittel |
| q.s. | Citronensäure |
| 2,00 | Natriumchlorid |
| add 100 | Aqua dem. |

Herstellung:

Komponenten einwiegen und lösen. pH-Wert auf 6 - 7 einstellen.

Beispiel 67: Shampoo

| | |
|---|---|
| 12,00 | Natriumlaurethsulfat |
| 1,50 | Decylglucosid |
| 2,50 | Cocamidpropylbetain |
| 5,00 | Coco-Glucosidglyceryloleat |
| 2,00 | Natriumlaurethsulfat, Glycoldistearat, Cocamid-MEA,Laureth-10 |
| 0,10-1,00 | erfindungsgemäße Dispersion |
| q.s. | Konservierungsmittel |
| q.s. | Sunset Yellow C.I. 15 985 |
| q.s. | Parfüm |
| 1,00 | Natriumchlorid |
| add 100 | Aqua dem. |

Herstellung:

Komponenten einwiegen und lösen. pH-Wert auf 6 - 7 einstellen.

Beispiel 68: Shampoo

| | |
|---|---|
| A | |
| 40,00 | Natriumlaurethsulfat |
| 5,00 | Natrium-$C_{12-15}$-Pareth-15-Sulfonat |
| 5,00 | Decylglucosid |
| q.s. | Parfüm |
| 0,10 | Phytantriol |
| | |
| B | |
| add 100 | Aqua dem. |

(fortgesetzt)

| | |
|---|---|
| B | |
| 0,10-1,00 | erfindungsgemäße Dispersion |
| 1,00 | Panthenol |
| q.s. | Konservierungsmittel |
| 1,00 | Laureth-3 |
| q.s. | Citronensäure |
| 2,00 | Natriumchlorid |

Herstellung:

Komponenten der Phase A einwiegen und lösen. pH-Wert auf 6 - 7 einstellen. Phase B zugeben und mischen.

**Patentansprüche**

1. Wässrige Polymerdispersion Pd), erhältlich durch radikalische Polymerisation eines Monomergemischs M), enthaltend

   a) wenigstens eine α,β-ethylenisch ungesättigte amidgruppenhaltige Verbindung der allgemeinen Formel I

   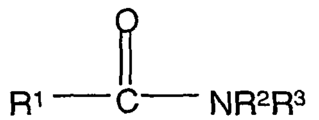

   (I)

   wobei
   $R^2$ für eine Gruppe der Formel $CH_2{=}CR^4$- steht und $R^1$ und $R^3$ unabhängig voneinander für H, Alkyl, Cycloalkyl, Heterocycloalkyl, Aryl oder Hetaryl stehen, oder $R^1$ und $R^3$ gemeinsam mit der Amidgruppe, an die sie gebunden sind, für ein Lactam mit 5 bis 8 Ringatomen stehen,
   b) wenigstens eine radikalisch polymerisierbare vernetzende Verbindung mit wenigstens zwei α,β-ethylenisch ungesättigten Doppelbindungen pro Molekül,
   c) wenigstens eine Verbindung mit einer radikalisch polymerisierbaren α,β-ethylenisch ungesättigten Doppelbindung und mindestens einer kationogenen und/oder kationischen Gruppe pro Molekül,

   in einem wässrigen Medium in Gegenwart wenigstens eines polymeren anionischen Dispergiermittels D).

2. Polymerdispersion nach Anspruch 1, wobei das Monomergemisch M) zusätzlich wenigstens ein weiteres Monomer d) enthält, das ausgewählt ist unter Estern α,β-ethylenisch ungesättigter Mono- und Dicarbonsäuren mit $C_1$-$C_{30}$-Alkanolen und $C_1$-$C_{30}$-Alkandiolen, Amiden α,β-ethylenisch ungesättigter Mono- und Dicarbonsäuren mit $C_2$-$C_{30}$-Aminoalkoholen, die eine primäre oder sekundäre Aminogruppe aufweisen, primären Amiden α,β-ethylenisch ungesättigter Monocarbonsäuren und deren N-Alkyl- und N,N-Dialkylderivaten, Estern von Vinylalkohol und Allylalkohol mit $C_1$-$C_{30}$-Monocarbonsäuren, Vinylethern, Vinylaromaten, Vinylhalogeniden, Vinylidenhalogeniden, $C_1$-$C_8$-Monoolefinen, nicht aromatischen Kohlenwasserstoffen mit mindestens zwei konjugierten Doppelbindungen und Mischungen davon.

3. Polymerdispersion nach einem der vorhergehenden Ansprüche, wobei das Monomergemisch M) zusätzlich wenigstens eine Verbindung e) mit einer radikalisch polymerisierbaren α,β-ethylenisch ungesättigten Doppelbindung und einer anionogenen und/oder anionischen Gruppe pro Molekül enthält, mit der Maßgabe, dass der Molanteil an anionogenen und anionischen Gruppen der Komponente e) geringer ist als der Molanteil an kationogenen und kationischen Gruppen der Komponente c).

4. Polymerdispersion nach einem der vorhergehenden Ansprüche, wobei die Komponente a) ausgewählt ist unter N-Vinylamiden gesättigter Monocarbonsäuren, N-Vinyllactamen und Mischungen davon.

5. Polymerdispersion nach einem der vorhergehenden Ansprüche, wobei die Komponente c) ausgewählt ist unter Estern α,β-ethylenisch ungesättigter Mono- und Dicarbonsäuren mit Aminoalkoholen, welche am Aminstickstoff mono- oder dialkyliert sein können, Amiden α,β-ethylenisch ungesättigter Mono- und Dicarbonsäuren mit Diaminen, welche mindestens eine primäre oder sekundäre Aminogruppe aufweisen, N,N-Diallylamin, N,N-Diallyl-N-alkylaminen und deren Derivaten, vinyl- und allylsubstituierten Stickstoffheterocyclen, vinyl- und allylsubstituierten heteroaromatischen Verbindungen und Mischungen davon.

6. Polymerdispersion nach Anspruch 5, wobei die Komponente c) Vinylimidazol oder ein Säuresalz oder ein Quaternisierungsprodukt davon umfasst.

7. Polymerdispersion nach Anspruch 3, wobei die Komponente e) ausgewählt ist unter monoethylenisch ungesättigten Carbonsäuren, Sulfonsäuren, Phosphonsäuren und Mischungen davon.

8. Polymerdispersion nach einem der vorhergehenden Ansprüche, wobei das polymere anionische Dispergiermittel D) ausgewählt ist unter Polymeren, die wenigstens ein Monomer, das ausgewählt ist unter Acrylsäure, Methacrylsäure, Maleinsäure und Mischungen davon, einpolymerisiert enthalten.

9. Polymerdispersion nach einem der vorhergehenden Ansprüche, wobei die Komponente a) in einer Menge von 10 bis 90 Gew.-%, bevorzugt 20 bis 70 Gew.-%, insbesondere 30 bis 60 Gew.-%, bezogen auf das Gesamtgewicht der Komponente a) und des Dispergiermittels D), eingesetzt wird.

10. Polymerdispersion nach einem der vorhergehenden Ansprüche, wobei das Dispergiermittel D) in einer Menge von 10 bis 90 Gew.-%, bevorzugt 20 bis 70 Gew.-%, insbesondere 30 bis 60 Gew.-%, bezogen auf das Gesamtgewicht der Komponente a) und des Dispergiermittels D), eingesetzt wird.

11. Polymerdispersion nach einem der vorhergehenden Ansprüche, wobei die Komponente b) ein einer Menge von 0,0005 bis 5 Gew.-%, bevorzugt 0,001 bis 2,5 Gew.-%, insbesondere 0,01 bis 1,5 Gew.-%, bezogen auf das Gewicht der Komponente a), eingesetzt wird.

12. Polymerdispersion nach einem der vorhergehenden Ansprüche, wobei die Komponente c) in einer Menge von 1 bis 40 Gew.-%, bevorzugt 5 bis 30 Gew.-%, bezogen auf das Gesamtgewicht der Komponente a) und des Dispergiermittels D), eingesetzt wird.

13. Polymerdispersion nach einem der vorhergehenden Ansprüche, wobei die Polymerisation zusätzlich in Gegenwart mindestens eines Reglers erfolgt.

14. Polymerdispersion nach einem der vorhergehenden Ansprüche, wobei der pH-Wert des wässrigen Mediums zur Polymerisation auf 6 bis 8, bevorzugt 6,5 bis 7,5, besonders bevorzugt 6,8 bis 7 eingestellt wird.

15. Polymerdispersion nach einem der vorhergehenden Ansprüche, die einen LD-Wert von höchstens 30 %, bevorzugt höchstens 20 %, insbesondere höchstens 10 % aufweist.

16. Polymer P), erhältlich durch Trocknen einer Polymerdispersion Pd), wie in einem der Ansprüche 1 bis 15 definiert.

17. Kosmetisches oder pharmazeutisches Mittel, enthaltend

    A) wenigstens eine Polymerdispersion Pd), wie in einem der Ansprüche 1 bis 15 definiert, oder ein Polymer P), wie in Anspruch 16 definiert, und
    B) wenigstens einen kosmetisch akzeptablen Träger.

18. Mittel nach Anspruch 17, wobei die Komponente B) ausgewählt ist unter

    i) Wasser,
    ii) wassermischbaren organischen Lösungsmitteln, vorzugsweise $C_1$-$C_4$-Alkanolen,
    iii) Ölen, Fetten, Wachsen,
    iv) von iii) verschiedenen Estern von $C_6$-$C_{30}$-Monocarbonsäuren mit ein-, zwei- oder dreiwertigen Alkoholen,
    v) gesättigten acyclischen und cyclischen Kohlenwasserstoffen,
    vi) Fettsäuren,

vii) Fettalkoholen

und Mischungen davon.

**19.** Mittel nach einem der Ansprüche 17 oder 18, enthaltend außerdem wenigstens einen von der Komponente A) verschiedenen Bestandteil, der ausgewählt ist unter kosmetisch aktiven Wirkstoffen, Emulgatoren, Tensiden, Konservierungsmitteln, Parfümölen, Verdickern, Haarpolymeren, Haar- und Hautconditionern, Pfropfpolymeren, wasserlöslichen oder dispergierbaren silikonhaltigen Polymeren, Lichtschutzmitteln, Bleichmitteln, Gelbildnern, Pflegemitteln, Färbemitteln, Tönungsmitteln, Bräunungsmitteln, Farbstoffen, Pigmenten, Konsistenzgebern, Feuchthaltemitteln, Rückfettern, Collagen, Eiweisshydrolysaten, Lipiden, Antioxidantien, Entschäumern, Antistatika, Emollienzien und Weichmachern.

**20.** Mittel nach einem der Ansprüche 17 bis 19 in Form eines Gels, Schaums, Sprays, einer Salbe, Creme, Emulsion, Suspension, Lotion, Milch oder Paste.

**21.** Verwendung einer Polymerdispersion, wie in einem der Ansprüche 1 bis 15 definiert oder eines Polymers, wie in Anspruch 16 definiert, in Hautreinigungsmitteln, Mitteln zur Pflege und zum Schutz der Haut, Nagelpflegemitteln; Zubereitungen für die dekorative Kosmetik und Haarbehandlungsmitteln.

**22.** Verwendung nach Anspruch 21 in Haarbehandlungsmitteln als Festiger und/oder als Conditioner.

**23.** Verwendung nach Anspruch 22, wobei das Mittel in Form eines Haargels, Shampoos, Schaumfestigers, Haarwassers, Haarsprays oder Haarschaums vorliegt.

**24.** Verwendung einer Polymerdispersion, wie in einem der Ansprüche 1 bis 15 definiert oder eines Polymers, wie in Anspruch 16 definiert, als Hilfsmittel in der Pharmazie, bevorzugt als oder in Beschichtungsmittel(n) für feste Arzneiformen, zur Modifizierung rheologischer Eigenschaften, als oberflächenaktive Verbindung sowie als oder in Beschichtungsmittel(n) für die Textil-, Papier-, Druck- und Lederindustrie.

## Claims

**1.** An aqueous polymer dispersion Pd) obtainable by free-radical polymerization of a monomer mixture M) comprising

a) at least one α,β-ethylenically unsaturated amide-group-containing compound of the formula I

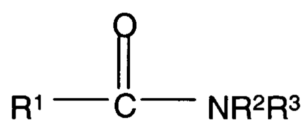

(I)

where

$R^2$ is a group of the formula $CH_2=CR^4$- and $R^1$ and $R^3$, independently of one another, are H, alkyl, cycloalkyl, heterocycloalkyl, aryl or hetaryl, or $R^1$ and $R^3$, together with the amide group to which they are bonded, are a lactam with 5 to 8 ring atoms,

b) at least one free-radically polymerizable crosslinking compound with at least two α,β-ethylenically unsaturated double bonds per molecule,
c) at least one compound with a free-radically polymerizable α,β-ethylenically unsaturated double bond and at least one cationogenic and/or cationic group per molecule,

in an aqueous medium in the presence of at least one polymeric anionic dispersant D).

**2.** The polymer dispersion according to claim 1, where the monomer mixture M) additionally comprises at least one

further monomer d) which is chosen from esters of α,β-ethylenically unsaturated mono- and dicarboxylic acids with $C_1$-$C_{30}$-alkanols and $C_1$-$C_{30}$-alkanediols, amides of α,β-ethylenically unsaturated mono- and dicarboxylic acids with $C_2$-$C_{30}$-amino alcohols which have a primary or secondary amino group, primary amides of α,β-ethylenically unsaturated monocarboxylic acids and N-alkyl and N,N-dialkyl derivatives thereof, esters of vinyl alcohol and allyl alcohol with $C_1$-$C_{30}$-monocarboxylic acids, vinyl ethers, vinyl aromatics, vinyl halides, vinylidene halides, $C_1$-$C_8$-monoolefins, nonaromatic hydrocarbons with at least two conjugated double bonds and mixtures thereof.

**3.** The polymer dispersion according to any of the preceding claims, where the monomer mixture M) additionally comprises at least one compound e) with a free-radically polymerizable α,β-ethylenically unsaturated double bond and an anionogenic and/or anionic group per molecule, with the proviso that the molar proportion of anionogenic and anionic groups in component e) is lower than the molar proportion of cationogenic and cationic groups in component c).

**4.** The polymer dispersion according to any of the preceding claims, where component a) is chosen from N-vinylamides of saturated monocarboxylic acids, N-vinyllactams and mixtures thereof.

**5.** The polymer dispersion according to any of the preceding claims, where component c) is chosen from esters of α, β-ethylenically unsaturated mono- and dicarboxylic acids with amino alcohols, which may be mono- or dialkylated on the amine nitrogen, amides of α,β-ethylenically unsaturated mono- and dicarboxylic acids with diamines which have at least one primary or secondary amino group, N,N-diallylamine, N,N-diallyl-N-alkylamines and derivatives thereof, vinyl- and allyl-substituted nitrogen heterocycles, vinyl- and allyl-substituted heteroaromatic compounds and mixtures thereof.

**6.** The polymer dispersion according to claim 5, where component c) comprises vinylimidazole or an acid salt or a quaternization product thereof.

**7.** The polymer dispersion according to claim 3, where component e) is chosen from monoethylenically unsaturated carboxylic acids, sulfonic acids, phosphonic acids and mixtures thereof.

**8.** The polymer dispersion according to any of the preceding claims, where the polymeric anionic dispersant D) is chosen from polymers which comprise, in copolymerized form, at least one monomer chosen from acrylic acid, methacrylic acid, maleic acid and mixtures thereof.

**9.** The polymer dispersion according to any of the preceding claims, where component a) is used in an amount of from 10 to 90% by weight, preferably 20 to 70% by weight, in particular 30 to 60% by weight, based on the total weight of component a) and the dispersant D).

**10.** The polymer dispersion according to any of the preceding claims, where the dispersant D) is used in an amount of from 10 to 90% by weight, preferably 20 to 70% by weight, in particular 30 to 60% by weight, based on the total weight of component a) and the dispersant D).

**11.** The polymer dispersion according to any of the preceding claims, where component b) is used in an amount of from 0.0005 to 5% by weight, preferably 0.001 to 2.5% by weight, in particular 0.01 to 1.5% by weight, based on the weight of component a).

**12.** The polymer dispersion according to any of the preceding claims, where component c) is used in an amount of from 1 to 40% by weight, preferably 5 to 30% by weight, based on the total weight of component a) and the dispersant D).

**13.** The polymer dispersion according to any of the preceding claims, where the polymerization additionally takes place in the presence of at least one regulator.

**14.** The polymer dispersion according to any of the preceding claims, where the pH of the aqueous medium for the polymerization is adjusted to 6 to 8, preferably 6.5 to 7.5, particularly preferably 6.8 to 7.

**15.** The polymer dispersion according to any of the preceding claims, which has an LT value of at most 30%, preferably at most 20%, in particular at most 10%.

**16.** A polymer P) obtainable by drying a polymer dispersion Pd), as defined in any of claims 1 to 15.

**17.** A cosmetic or pharmaceutical composition comprising

A) at least one polymer dispersion Pd), as defined in any of claims 1 to 15, or a polymer P), as defined in claim 16, and
B) at least one cosmetically acceptable carrier.

**18.** The composition according to claim 17, where component B) is chosen from

i) water,
ii) water-miscible organic solvents, preferably $C_1$-$C_4$-alkanols,
iii) oils, fats, waxes,
iv) esters of $C_6$-$C_{30}$-monocarboxylic acids with mono-, di- or trihydric alcohols which are different from iii),
v) saturated acyclic and cyclic hydrocarbons,
vi) fatty acids,
vii) fatty alcohols

and mixtures thereof.

**19.** The composition according to either claim 17 or 18, further comprising at least one constituent different from component A) which is chosen from cosmetically active ingredients, emulsifiers, surfactants, preservatives, perfume oils, thickeners, hair polymers, hair and skin conditioners, graft polymers, water-soluble or dispersible silicone-containing polymers, light protection agents, bleaches, gel formers, care agents, colorants, tinting agents, tanning agents, dyes, pigments, consistency-imparting agents, humectants, refatting agents, collagen, protein hydrolyzates, lipids, antioxidants, antifoams, antistats, emollients and softeners.

**20.** The composition according to any of claims 17 to 19 in the form of a gel, foam, spray, ointment, cream, emulsion, suspension, lotion, milk or paste.

**21.** The use of a polymer dispersion as defined in any of claims 1 to 15, or of a polymer as defined in claim 16 in skin-cleansing compositions, compositions for the care and protection of the skin, nailcare compositions, preparations for decorative cosmetics and hair-treatment compositions.

**22.** The use according to claim 21 in hair-treatment compositions as setting agents and/or as conditioners.

**23.** The use according to claim 22, where the composition is in the form of a hair gel, shampoo, setting foam, hair tonic, hairspray or hair mousse.

**24.** The use of a polymer dispersion as defined in any of claims 1 to 15 or of a polymer as defined in claim 16 as auxiliary in pharmacy, preferably as or in (a) coating(s) for solid drug forms, for modifying rheological properties, as surface-active compound, and as or in (a) coating(s) for the textile, paper, printing and leather industries.

## Revendications

**1.** Dispersion aqueuse de polymère Pd), que l'on peut obtenir par polymérisation radicalaire d'un mélange de monomères M), contenant

a) au moins un composé α, β-éthyléniquement insaturé, contenant des groupes amide, de la formule générale 1 :

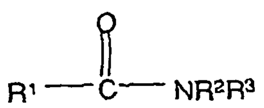

(I)

dans laquelle $R^2$ représente un groupe de la formule $CH_2$=$CR^4$ et $R^1$ et $R^3$ représentent, indépendamment l'un de l'autre, H, un groupe alkyle, cycloalkyle, hétérocycloalkyle, aryle ou hétaryle, $R^1$ et $R^3$ pouvant former conjointement avec le groupe amide, sur lequel ils sont fixés, un lactame comportant 5 à 8 atomes cycliques,

b) au moins un composé réticulant, polymérisable par voie radicalaire, comportant au moins deux doubles liaisons α,β-éthyléniquement insaturées par molécule,

c) au moins un composé comportant une double liaison α,β-éthyléniquement insaturée, polymérisable par voie radicalaire, et au moins un groupe cationogène et/ou cationique par molécule,

dans un milieu aqueux, en présence d'au moins un agent dispersant anionique polymère D).

**2.** Dispersion de polymère suivant la revendication 1, dans laquelle le mélange de monomères M) contient en supplément au moins un autre monomère d) qui est choisi parmi des esters d'acides monocarboxyliques et dicarboxyliques (α, β-éthyléniquement insaturés avec des alcanols en $C_1$-$C_{30}$ et des alcanediols en $C_1$-$C_{30}$, des amides d'acides monocarboxyliques et dicarboxyliques α, β-éthyléniquement insaturés avec des aminoalcools en $C_2$-$C_{30}$, qui présentent un groupe amino primaire ou secondaire, des amides primaires d'acides monocarboxyliques α,β-éthyléniquement insaturés et leurs dérivés N-alkyliques et N,N-dialkyliques, des esters d'alcool vinylique et d'alcool allylique avec des acides monocarboxyliques en $C_1$-$C_{30}$, des éthers vinyliques, des substances vinyl-aromatiques, des halogénures de vinyle, des halogénures de vinylidène, des monooléfines en $C_1$-$C_8$, des hydrocarbures non aromatiques comportant au moins deux doubles liaisons conjuguées et leurs mélanges.

**3.** Dispersion de polymère suivant l'une des revendications précédentes, dans laquelle le mélange de monomères M) contient en supplément au moins un composé e) comportant une double liaison α,β-éthyléniquement insaturée, polymérisable par voie radicalaire, et un groupe anionogène et/ou anionique par molécule, à la condition que la fraction molaire de groupes anionogènes et anioniques du composant e) soit plus petite que la fraction molaire des groupes cationogènes et cationiques du composant c).

**4.** Dispersion de polymère suivant l'une des revendications précédentes, dans laquelle le composant a) est choisi parmi des N-vinylamides d'acides monocarboxyliques saturés, des N-vinyl-lactames et leurs mélanges.

**5.** Dispersion de polymère suivant l'une des revendications précédentes, dans laquelle le composant c) est choisi parmi des esters d'acides monocarboxyliques et dicarboxyliques α, β-éthyléniquement insaturés avec des aminoalcools qui peuvent être monoalkylés ou dialkylés sur l'azote d'amine, des amides d'acides monocarboxyliques et dicarboxyliques α, β-éthyléniquement insaturés avec des diamines qui présentent au moins un groupe amino primaire ou secondaire, de la N,N-diallylamine, des N,N-diallyl-N-alkylamines et leurs dérivés, des hétérocycles azotés substitués par du vinyle et de l'allyle, des composés hétéroaromatiques substitués par du vinyle et de l'allyle, et leurs mélanges.

**6.** Dispersion de polymère suivant la revendication 5, dans laquelle le composant c) comporte du vinylimidazole ou un sel d'acide ou un produit de quaternisation de celui-ci.

**7.** Dispersion de polymère suivant la revendication 3, dans laquelle le composant e) est choisi parmi des acides phosphoniques, des acides sulfoniques, des acides carboxyliques monoéthyléniquement insaturés et leurs mélanges.

**8.** Dispersion de polymère suivant l'une des revendications précédentes, dans laquelle l'agent dispersant anionique polymère D) est choisi parmi des polymères qui contiennent, à l'état copolymérisé, au moins un monomère qui est choisi parmi de l'acide acrylique, de l'acide méthacrylique, de l'acide maléique et leurs mélanges.

**9.** Dispersion de polymère suivant l'une des revendications précédentes, dans laquelle le composant a) est mis en oeuvre en une quantité de 10 à 90 % en poids, avantageusement de 20 à 70 % en poids, en particulier de 30 à 60 % en poids, par rapport au poids total du composant a) et de l'agent dispersant D).

**10.** Dispersion de polymère suivant l'une des revendications précédentes, dans laquelle l'agent dispersant D) est mis en oeuvre en une quantité de 10 à 90 % en poids, avantageusement de 20 à 70 % en poids, en particulier de 30 à 60 % en poids, par rapport au poids total du composant a) et de l'agent dispersant D).

**11.** Dispersion de polymère suivant l'une des revendications précédentes, dans laquelle le composant b) est mis en oeuvre en une quantité de 0,0005 à 5 % en poids, avantageusement de 0,001 à 2,5 % en poids, en particulier de 0,01 à 1,5 % en poids, par rapport au poids du composant a).

**12.** Dispersion de polymère suivant l'une des revendications précédentes, dans laquelle le composant c) est mis en

oeuvre en une quantité de 1 à 40 % en poids, avantageusement de 5 à 30 % en poids, par rapport au poids total du composant a) et de l'agent dispersant D).

**13.** Dispersion de polymère suivant l'une des revendications précédentes, dans laquelle la polymérisation a lieu en supplément en présence d'au moins un régulateur.

**14.** Dispersion de polymère suivant l'une des revendications précédentes, dans laquelle la valeur de pH du milieu aqueux destiné à la polymérisation est ajustée à 6 jusqu'à 8, avantageusement à 6,5 jusqu'à 7,5, particulièrement avantageusement à 6,8 jusqu'à 7.

**15.** Dispersion de polymère suivant l'une des revendications précédentes, qui présente une valeur LD d'au maximum 30 %, avantageusement d'au maximum 20 %, en particulier au maximum de 10 %.

**16.** Polymère P), qui peut être obtenu par séchage d'une dispersion de polymère Pd), telle que définie dans l'une des revendications 1 à 15.

**17.** Produit cosmétique ou pharmaceutique, contenant

A) au moins une dispersion de polymère Pd), telle que définie dans une des revendications 1 à 15, ou un polymère P), tel que défini dans la revendication 16, et
B) au moins un support cosmétiquement acceptable.

**18.** Produit suivant la revendication 17, dans lequel le composant B) est choisi parmi

i) de l'eau,
ii)des solvants organiques miscibles à l'eau, de préférence des alcanols en $C_1$-$C_4$,
iii) des huiles, des graisses, des cires,
iv)des esters, différents de iii), d'acides monocarboxyliques en $C_6$-$C_{30}$ avec des alcools monofonctionnels, bifonctionnels ou trifonctionnels,
v) des hydrocarbures cycliques et acycliques saturés,
vi)des acides gras,
vii) des alcools gras

et leurs mélanges.

**19.** Produit suivant l'une des revendications 17 ou 18, contenant en outre au moins un élément différent du composant A) qui est choisi parmi des substances cosmétiquement actives, des agents émulsionnants, des agents tensioactifs, des agents de conservation, des huiles de parfum, des épaississants, des polymères pour cheveux, des après-shampooings et des crèmes traitantes, des polymères greffés, des polymères contenant du silicone dispersibles ou solubles dans l'eau, des agents de protection contre la lumière, des agents de blanchiment, des agents gélifiants, des agents de soin, des colorants, des agents de nuancement, des agents de brunissement, des matières colorantes, des pigments, des agents donnant de la consistance, des agents humidifiants, des agents regraissants, du collagène, des produits d'hydrolyse de protéine, des lipides, des antioxydants, des démoussants, des antistatiques, des émollients et des agents assouplissants.

**20.** Produit suivant l'une des revendications 17 à 19, sous la forme d'un gel, d'une mousse, d'un produit à pulvériser, d'une pommade, d'une crème, d'une émulsion, d'une suspension, d'une lotion, d'un lait ou d'une pâte.

**21.** Utilisation d'une dispersion de polymère, telle que définie dans une des revendications 1 à 15, ou d'un polymère, tel que défini dans la revendication 16, dans des produits de nettoyage de la peau, des produits de soin et de protection de la peau, des produits de soin pour les ongles, des compositions pour la cosmétique décorative et des produits de traitement des cheveux.

**22.** Utilisation suivant la revendication 21, dans des produits de traitement des cheveux, comme fixateurs et/ou comme après-shampooings.

**23.** Utilisation suivant la revendication 22, dans laquelle le produit se présente sous la forme d'un gel pour les cheveux, d'un shampooing, d'une mousse fixante, d'une eau, d'un spray ou d'une mousse pour les cheveux.

**24.** Utilisation d'une dispersion de polymère, telle que définie dans l'une des revendications 1 à 15, ou d'un polymère, tel que défini dans la revendication 16, comme adjuvant dans la pharmacie, de préférence sous la forme ou dans un (des) produit(s) de revêtement pour des formes médicamenteuses solides, pour la modification des propriétés rhéologiques, comme composé tensioactif, ainsi que sous la forme ou dans un (des) produit(s) de revêtement de l'industrie textile, du papier, de l'impression et du cuir.